# EUROPEAN PATENT SPECIFICATION

(11) **EP 2 262 764 B1**
(45) Date of publication and mention of the grant of the patent: **12.11.2014**
(21) Application number: 09755179.0
(22) Date of filing: 23.03.2009
(51) Int. Cl.: C07C 331/28, C07C 247/16, C07C 235/34, A61K 31/26, A61K 31/165, A61P 25/08

(54) **NOVEL N-BENZYLAMIDE SUBSTITUTED DERIVATIVES OF 2-(ACYLAMIDO)ACETIC ACID AND 2-(ACYLAMIDO)PROPIONIC ACIDS: POTENT NEUROLOGICAL AGENTS**
NEUARTIGE N-BENZYLAMID-SUBSTITUIERTE DERIVATE VON 2-(ACYLAMID-)ESSIGSÄURE UND 2-(ACYLAMID-)PROPIONSÄUREN: STARKE NEUROLOGISCHE WIRKSTOFFE
NOUVEAUX DÉRIVÉS SUBSTITUÉS PAR N-BENZYLAMIDE D'ACIDE 2-(ACYLAMIDO)ACÉTIQUES ET ACIDES 2-(ACYLAMIDO)PROPIONIQUES : AGENTS NEUROLOGIQUES PUISSANTS

(30) Priority: 01.04.2008 US 41311 P; 05.12.2008 US 120199 P
(43) Date of publication of application: 22.12.2010
(73) Proprietor: The University of North Carolina At Chapel Hill, Chapel Hill, NC 27599-4105 (US)
(72) Inventor: KOHN, Harold, Chapel Hill NC 27514 (US); SALOME, Christophe, Chapel Hill NC 27514 (US); PARK, Ki Duk, Chapel Hill NC 27517 (US)
(74) Representative: Horner, Martin Grenville
(86) International application number: PCT/US2009/001802
(87) International publication number: WO 2009/145816

(56) References cited:
- EP-A2- 0 169 033
- WO-A1-92/14706
- US-A- 4 696 946
- US-A- 5 378 729
- US-A- 5 654 301
- US-A- 5 654 301
- US-A- 5 773 475
- JONES, J. B. ET AL.: 'Alkylations of the side-chain nucleophiles ofcysteine, methionine, histidine, and lysine derivatives with allyl bromide, 1-bromo-2-butyne, and 2-bromoaceto- phenone' CANADIAN JOURNAL OF CHEMISTRY vol. 49, no. 18, 1971, pages 3012 - 19, XP008142176
- BEGUIN, C. ET AL.: 'Functionalized Amido Ketones: New Anticonvulsant Agents' BIOORGANIC & MEDICINAL CHEMISTRY vol. 11, no. 19, 2003, pages 4275 - 4285, XP008142126

## Description

### Field of the Invention

The present inventions concern compounds, pharmaceutical compositions, and 1. their use in methods for treating neurological disorders such as epilepsy and neuropathic pain, and as neuroprotective agents.

### Background of the Invention

Epilepsy is a major neurological disorder that affects all populations (W. Hauser et al., The prevalence of epilepsy in Rochester, Minnesota, 1940-80. Epilepsia 1991 32, 429-445). Epilepsy describes the types of recurrent seizures produced by paroxysmal excessive neuronal discharges in the brain (Evans, J.H. Post-traumatic epilepsy. Neurology 1962, 12, 665-674; Lindsay, J.M. Genetics and epilepsy. Epilepsia 1971, 12, 47-54). In the United States alone, some 2 million people suffer from epilepsy and its sequelae; 340,000 are children. For many individuals afflicted with epilepsy the disabilities and associated neuropsychological and behavioral factors limit the quality of life. The restrictions on patients with epilepsy plus the expense for treatment and rehabilitation result in a large cost to society (See, *e.g.,* Begley, C.E.; Lairson, D.R.; Reynolds, T.F.; Coan, S. Early treatment cost in epilepsy and how it varies with seizure type and frequency. Epilepsia Res. 2001, 47, 205-215).

The mainstay of treatment for epileptic disorders has been the long-term and consistent administration of anticonvulsant drugs (*See, e.g.,* McNamara, J.O. In: Goodman & Gilman's The Pharmacological Basis of Therapeutics, 10th Ed.; Hardman, J.G.; Limbird, L.E., Ed.; McGraw-Hill: New York, 2001; Ch. 21, pp. 521-547; Aiken, S.P.; Brown, W.M. Treatment of epilepsy: Existing therapies and future developments. Frontiers in Bioscience 2000, 5, 124-15). Unfortunately, current medications are ineffective for approximately one-third of patients with epilepsy (See, e.g., Bauer, J.; Reuber, M. Medical treatment of epilepsy. Expert Opinion on Emerging Drugs 2003, 8, 457-467). Many continue to have seizures, while others experience disturbing side effects (e.g., drowsiness, dizziness, nausea, liver damage) (Pellock, J.M.; Willmore, L.J. A rational guide to monitoring in patients receiving anticonvulsants. Neurology 1991, 41, 961-964). The shortcomings of current regimens highlight the need for new agents with novel mechanisms of action.

### Summary of the Invention

A first aspect of the invention is a compound (sometimes also referred to herein as an "active agent" or "active compound") of Formula I: 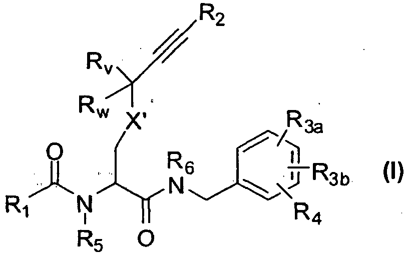 wherein:
R₁ is alkyl or cycloalkyl, each of which can be unsubstituted or substituted with from one to four independently selected electron-donating or electron-withdrawing groups;
R₂, Rᵥ and R_{w} are each independently hydrogen, alkyl, cycloalkyl, heterocyclo, aryl, or heteroaryl, which alkyl, cyloalkyl, heteroacylclo, aryl, or heteroaryl can be unsubstituted or substituted with from one to four independently selected electron-donating or electron-withdrawing groups, or R₂, Rᵥ and R_{w} are each independently an electron-donating or electron-withdrawing group;
R₃ₐ, R_{3b} and R₄ are each independently hydrogen, an electron donating or electron-withdrawing group, or alkyl, cycloalkyl, alkenyl, alkynyl, aryl, heterocyclo, heteroaryl, arylalkyl, heteroarylalkyl, heterocycloalkyl, each of which can be unsubstituted or substituted with from one to four independently selected electron-donating or electron-withdrawing groups;
R₅ and R₆ are each independently H or alkyl;
X' is S or O;
or a pharmaceutically acceptable salt thereof.

The present specification also mentions a compound (sometimes also referred to herein as an "active agent" or "active compound") of Formula **Ia:** 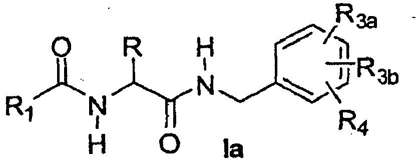 wherein:
R is R₂ or CH₂X'R₅, where X' is O, S, or N-R₆ₐ and R₆ₐ is hydrogen, alkyl, or cycloalkyl;
R₁ is alkyl or cycloalkyl, each of which can be unsubstituted or substituted with one or more (e.g., one, two, three, four) independently selected electron-donating or electron-withdrawing groups;
R₂ is alkyl, cycloalkyl, aryl, five- or six-membered cyclic heterocyclic or heteroaromatic groups, or -X-Y-Z, where X and Y = O, S, N-R₆, and Z = R₆, and where R₆ is hydrogen, alkyl, or cycloalkyl, each of which is unsubstituted or substituted with one or more (e.g., one, two, three, four) electron withdrawing or electron donating groups;
R₆ is alkyl, alkenyl (e.g., -CH₂CH = CH₂), alkynyl (e.g., -CH₂C ≡CH), or arylalkyl, each of which is unsubstituted or substituted with one or more (e.g., one, two, three, four) electron donating or electron withdrawing groups;
R_{3b}, and R₄ are each independently hydrogen, an electron donating or electron-withdrawing group, or alkyl, cycloalkyl, alkenyl, alkynyl, aryl, heterocyclo, heteroaryl, arylalkyl, heteroarylalkyl, heterocycloalkyl, arylalkyloxy, arylalkylamino, arylalkylthio, heteroarylalkyloxy, heteroarylalkylamino, heteroarylalkylthio, heterocycloalkyloxy, heterocycloalkylamino, heterocycloalkylthio, arylaminooxy, heteroarylaminooxy, heterocycloaminooxy, aryloxyamino, heteroaryloxyamino, heterocyclooxyamino, arylalkyl, heteroarylalkyl, heterocycloalkyl, arylalkenyl, heteroarylalkenyl, heterocycloalkenyl, arylalkynyl, heteroarylalkynyl, heterocycloalkynyl, arylhydrazino, heteroarylhydrazino, heterocyclohydrazino, arylazo, heteroarylazo, heterocycloazo, arylalkylaminoalkyl, heteroarylalkylaminoalkyl, heterocycloalkylaminoalkyl, arylalkyloxyalkyl, heteroarylalkyloxyalkyl, heterocycloalkyloxyalkyl, each of which can be unsubstituted or substituted with one or more (e.g., one, two, three, four) independently selected electron-donating or electron-withdrawing groups;
or a pharmaceutically acceptable salt thereof.

A further aspect of the present invention is a pharmaceutical composition comprising, consisting of or consisting essentially of an active compound as described herein (i.e., compounds of Formula I) in a pharmaceutically acceptable carrier. The compositions may optionally further comprise or include at least one additional neurological or neuroprotective active agent.

A further aspect of the invention is the use of an active agent of Formula I as described herein for the preparation of a medicament for neuroprotection or treating a neurological disorder as described here.

A further aspect of the invention is an active agent of Formula I as described herein for use in neuroprotection or treating a neurological disorder as described herein.

### Detailed Description of Embodiments of the Invention

### A. Definitions.

"*Neurological disorder*" as used herein may be any central or peripheral nervous system disorder, including but not limited to acute and chronic pain, migraine, neuropathic pain, fibromyalgia, bipolar disorders, convulsions, mania, epilepsy, epileptogenesis, convulsions and other seizure disorders, dyskinesia, anxiety, depression, etc.

"Neuroprotection" as used herein refers to any type of neuroprotection, such as against a neurotoxic agent, either prophylactically or after exposure to a neurotoxic agent.

"Alkyl" as used herein alone or as part of another group, refers to a straight or branched chain hydrocarbon containing from 1 to 10 carbon atoms. Representative examples of alkyl include, but are not limited to, methyl, ethyl, n-propyl, iso-propyl, n-butyl, sec-butyl, iso-butyl, tert-butyl, n-pentyl, isopentyl, neopentyl, n-hexyl, 3-methylhexyl, 2,2-dimethylpentyl, 2,3-dimethylpentyl, n-heptyl, n-octyl, n-nonyl, n-decyl, and the like. "Loweralkyl" as used herein, is a subset of alkyl, in some embodiments preferred, and refers to a straight or branched chain hydrocarbon group containing from 1 to 4 carbon atoms. Representative examples of lower alkyl include, but are not limited to, methyl, ethyl, n-propyl, iso-propyl, n-butyl, iso-butyl, tert-butyl, and the like. Such groups can be unsubstituted or substituted with one or more (*e.g.**,*** one, two, three four, etc.) independently selected electron-donating or electron-withdrawing groups

"Cycloalkyl" as used herein alone or as part of another group, refers to a saturated or partially unsaturated cyclic hydrocarbon group containing from 3, 4 or 5 to 6, 7 or 8 carbons (which carbons may be replaced in a heterocyclic group as discussed below). Representative examples of cycloalkyl include, cyclopropyl, cyclobutyl, cyclopentyl, cyclohexyl, cycloheptyl, and cyclooctyl. These rings may be optionally substituted with additional substituents as described herein such as halo or loweralkyl. The term "cycloalkyl" is generic and intended to include heterocyclic groups as discussed below unless specified otherwise. Such groups can be unsubstituted or substituted with one or more (*e.g*., one, two, three four, etc.) independently selected electron-donating or electron-withdrawing groups.

"Alkenyl" as used herein alone or as part of another group, refers to a straight or branched chain hydrocarbon containing from 1 to 10 carbon atoms (or in loweralkenyl 1 to 4 carbon atoms) which include 1 to 4 double bonds in the normal chain. Representative examples of alkenyl include, but are not limited to, vinyl, 2-propenyl, 3-butenyl, 2-butenyl, 4-pentenyl, 3-pentenyl, 2-hexenyl, 3-hexenyl, 2,4-heptadiene, and the like. Such groups can be unsubstituted or substituted with one or more (e.g., one, two, three four, etc.) independently selected electron-donating or electron-withdrawing groups.

"Alkynyl" as used herein alone or as part of another group, refers to a straight or branched chain hydrocarbon containing from 1 to 10 carbon atoms (or in loweralkynyl 1 to 4 carbon atoms) which include 1 triple bond in the normal chain. Representative examples of alkynyl include, but are not limited to, 2-propynyl, 3-butynyl, 2- butynyl, 4-pentynyl, 3-pentynyl, and the like. The term "alkynyl" or "loweralkynyl" is intended to include both substituted and unsubstituted alkynyl or loweralkynyl unless otherwise indicated and these groups may be substituted with the same groups as set forth in connection with alkyl and loweralkyl above. Such groups can be unsubstituted or substituted with one or more (*e.g*., one, two, three four, etc.) independently selected electron-donating or electron-withdrawing groups.

"Heterocyclo" as used herein alone or as part of another group, refers to an aliphatic (e.g., fully or partially saturated heterocyclo) or aromatic (*e.g*., heteroaryl) monocyclic- or a bicyclic-ring system. Monocyclic ring systems are exemplified by any 3 to 8 membered ring containing 1, 2, 3, or 4 heteroatoms independently selected from oxygen, nitrogen and sulfur. The 5 membered ring has from 0-2 double bonds and the 6 membered ring has from 0-3 double bonds. Representative examples of monocyclic ring systems include, but are not limited to, azetidine, azepine, aziridine, diazepine, 1,3-dioxolane, dioxane, dithiane, furan, imidazole, imidazoline, imidazolidine, isothiazole, isothiazoline, isothiazolidine, isoxazole, isoxazoline, isoxazolidine, morpholine, oxadiazole, oxadiazoline, oxadiazolidine, oxazole, oxazoline, oxazolidine, piperazine, piperidine, pyran, pyrazine, pyrazole, pyrazoline, pyrazolidine, pyridine, pyrimidine, pyridazine, pyrrole, pyrroline, pyrrolidine, tetrahydrofuran, tetrahydrothiophene, tetrazine, tetrazole, thiadiazole, thiadiazoline, thiadiazolidine, thiazole, thiazoline, thiazolidine, thiophene, thiomorpholine, thiomorpholine sulfone, thiopyran, triazine, triazole, trithiane, and the like. Bicyclic ring systems are exemplified by any of the above monocyclic and heterocyclic ring systems fused to an aryl group as defined herein, a cycloalkyl group as defined herein, or another monocyclic or heterocyclic ring system as defined herein. Representative examples of bicyclic ring systems include but are not limited to, for example, benzimidazole, benzothiazole, benzothiadiazole, benzothiophene, benzoxadiazole, benzoxazole, benzofuran, benzopyran, benzothiopyran, benzodioxine, 1,3-benzodioxole, cinnoline, indazole, indole, indoline, indolizine, naphthyridine, isobenzofuran, isobenzothiophene, isoindole, isoindoline, isoquinoline, phthalazine, purine, pyranopyridine, quinoline, quinolizine, quinoxaline, quinazoline, tetrahydroisoquinoline, tetrahydroquinoline, thiopyranopyridine, and the like. Such groups can be unsubstituted or substituted with one or more (*e.g*., one, two, three four, etc.) independently selected electron-donating or electron-withdrawing groups.

"Aryl" as used herein alone or as part of another group, refers to a monocyclic carbocyclic ring system or a bicyclic carbocyclic fused ring system having one or more aromatic rings. Representative examples of aryl include, azulenyl, indanyl, indenyl, naphthyl, phenyl, tetrahydronaphthyl, and the like. The term "aryl" is intended to include both substituted and unsubstituted aryl unless otherwise indicated and these groups may be substituted with the same groups as set forth in connection with alkyl and loweralkyl above. Such groups can be unsubstituted or substituted with one or more (*e.g.,* one, two, three four, etc.) independently selected electron-donating or electron-withdrawing groups.

"Heteroaryl" as used herein is as described in connection with heterocyclo above. Such groups can be unsubstituted or substituted with one or more (*e.g.*, one, two, three four, etc.) independently selected electron-donating or electron-withdrawing groups.

"Arylalkyl" as used herein alone or as part of another group, refers to an aryl group, as defined herein, appended to the parent molecular moiety through an alkyl group, as defined herein. Representative examples of arylalkyl include, but are not limited to, benzyl, 2-phenylethyl, 3-phenylpropyl, 2-naphth-2-ylethyl, and the like.

"Heteroarylalkyl" as used herein alone or as part of another group, refers to a heteroaryl group, as defined herein, appended to the parent molecular moiety through an alkyl group, as defined herein.

"Heterocycloalkyl" as used herein alone or as part of another group, refers to a heterocyclo group, as defined herein, appended to the parent molecular moiety through an alkyl group, as defined herein.

"Electron-withdrawing" and "electron donating" refer to the ability of a substituent to withdraw or donate electrons relative to that of hydrogen if the hydrogen atom occupied the same position in the molecule. These terms are well understood by one skilled in the art and are discussed in Advanced Organic Chemistry, by J. March, John Wiley and Sons, New York, N.Y., pp. 16-18 (1985), incorporated herein by reference. Examples of such electron withdrawing and electron donating groups or substituents include, but are not limited to halo, nitro, cyano, carboxy, loweralkenyl, loweralkynyl, loweralkanoyl (*e.g.*, formyl), carboxyamido, aryl, quaternary ammonium, aryl (loweralkanoyl), carbalkoxy and the like; acyl, carboxy, alkanoyloxy, aryloxy, alkoxysulfonyl, aryloxysulfonyl, and the like; hydroxy, alkoxy or loweralkoxy (including methoxy, ethoxy and the like); loweralkyl; amino, lower alkylamino, di(loweralkyl) amino, aryloxy (such as phenoxy), mercapto, loweralkylthio, lower alkylmercapto, disulfide (loweralkyldithio) and the like; 1-piperidino, 1-piperazino, 1-pyrrolidino, acylamino, hydroxyl, thiolo, alkylthio, arylthio, aryloxy, alkyl, ester groups (e.g., alkylcarboxy, arylcarboxy, heterocyclocarboxy), azido, isothiocyanato, isocyanato, thiocyanato, cyanato, and the like. One skilled in the art will appreciate that the aforesaid substituents may have electron donating or electron withdrawing properties under different chemical conditions. Moreover, the present invention contemplates any combination of substituents selected from the above-identified groups. See US Patent No. 6,133,261 and 5,654,301; see *also* US Patent No. 4,711,532.

"Acyl" as used herein alone or as part of another group refers to a -C(O)R radical, where R is any suitable substituent such as aryl, alkyl, alkenyl, alkynyl, cycloalkyl or other suitable substituent as described herein.

"Alkanoyl" refers to the group -C(O)R', wherein R' is lower alkyl. Hence "alkanoyl" groups are particular examples of "acyl" groups, as described above.

"Halo" or "halogen," as used herein refers to -Cl, -Br, -I or -F.

"Oxy" as used herein refers to an -O- group.

"Sulfonyl," as used herein, refers to an -SO₂ - group.

"Thio" as used herein refers to an -S- group.

"Peptide coupling reagent" as used herein may be any suitable peptide coupling reagent, including but not limited to: N,N'-dicyclohexylcarbodiimide, N,N'-dicyclohexylcarbodiimide plus hydroxysuccinimide, N,N'-dicyclohexylcarbodimide plus 1-hydroxy-benzotriazole, 1-benzotriazolyl diethyl phosphate, 1-chloro-N,N,2-trimethyl-1-propen-1-amine, diphenyl phosphoryl azide, diethylphosphorochloridate, di-loweralkyl (C1 -C8) phosphorochloridates, diphenyl phosphorochloridate, phenyl phosphorodichloridate benzotriazol-1-yloxytris(dimethylamino)-phosphonium hexafluorophosphate, N,N'-bis[2-oxo-3-oxazolidinyl]-phosphorodiamidic chloride, diphenylphosphinyl chloride, diethoxyphosphoryl cyanide, bis(pentafluorophenyl)sulfite, N,N'-carbonyldiimidazole, or isobutyl chloroformate plus N-methylmorpholine, 2-chloro-1-methylpyridinium iodide, N,N'-disuccinimidyl carbonate, 1-bromo-N,N,2-trimethyl-1-propen-1-amine, 1-benzotriazolyl diethyl phosphate (see US Patent No. 5,189,023); dicyclohexylcarbodiimide (DCC), diisopropylcarbodiimide (DIPC), 1-ethyl-3-(3-dimethylaminopropyl)carbodiimide (EDC), benzotriazole-1-yloxy-tris(dimethylamino)phosphonium hexafluorophosphate (BOP) and diphenylphosphorylazide (DPPA) (see US Patent No. 6,172,067); DIC, TBTU, WSCDI, EEDQ, HBTU, TNTU, TSTU, HATU, BOP, BOP-CI, PyBOP, PyBroP, IPCF, IBCF and propane phosphonic acid anhydride (see US Patent No. 5,977,302), and 4-(4,6-dimethoxy-1,3,5-triazin-2-yl)-4-methylmorpholinium chloride) (DMTMM), tetramethylfluoroformamidiniun hexafluorophosphate, etc.

"Subjects" as used herein are typically human subjects, but may also be other mammalian subjects such as dogs, cats, horses, etc. treated for veterinary purposes. Subjects may be male or female and may be neonate, infant, juvenile, adolescent, adult or geriatric subjects.

"Treat" as used herein refers to any type of treatment that imparts a benefit to a subject, including delaying the onset or progression of one or more symptom, reducing the severity of one or more symptom, etc..

"Pharmaceutically acceptable" as used herein means that the compound or composition is suitable for administration to a subject to achieve the treatments described herein, without unduly deleterious side effects in light of the severity of the disease and necessity of the treatment.

"Concurrently" as used herein means sufficiently close in time to produce a combined effect (that is, concurrently may be simultaneously, or it may be two or more events occurring within a short time period before or after each other).

### B. Active compounds of Formula I.

As noted above, the present invention provides compounds of Formula **I:** 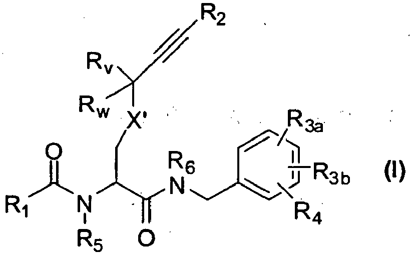 wherein:
R₁ is alkyl or cycloalkyl, each of which can be unsubstituted or substituted with from one to four independently selected electron-donating or electron-withdrawing groups;
R₂, Rᵥ and R_{w} are each independently hydrogen, alkyl, cycloalkyl, heterocyclo, aryl, or heteroaryl, which alkyl, cyloalkyl, heteroacylclo, aryl, or heteroaryl can be unsubstituted or substituted with from one to four independently selected electron-donating or electron-withdrawing groups, or R₂, Rᵥ and R_{w} are each independently an electron-donating or electron-withdrawing group;
R₃ₐ, R_{3b} and R₄ are each independently hydrogen, an electron donating or electron-withdrawing group, or alkyl, cycloalkyl, alkenyl, alkynyl, aryl, heterocyclo, heteroaryl, arylalkyl, heteroarylalkyl, heterocycloalkyl, each of which can be unsubstituted or substituted with from one to four independently selected electron-donating or electron-withdrawing groups;
R₅ and R₆ are each independently H or alkyl;
X' is S or 0;
or a pharmaceutically acceptable salt thereof.

In some embodiments of the foregoing, R⁴ is H; R⁵ is H; R⁶ is H; and X' is O.

In some embodiments of the foregoing, R₁ is C1-C4 alkyl; R₂ is hydrogen or C1-C4 alkyl; R₃ₐ is H; R_{3b} is H, N₃, or NCS; R₄ is H; R₅ and R₆ are both H; and X' is O.
or a pharmaceutically acceptable salt thereof.

### Exemplary embodiments of the foregoing include, but are not limited to:

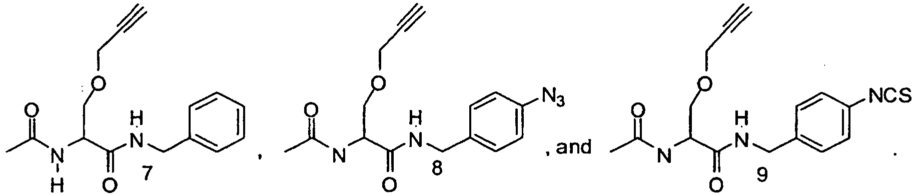

In some embodiments of the foregoing, the active compounds are provided in the (R)-configuration.

In some embodiments of the foregoing, the active compounds are provided in isolated and/or purified form, or substantially pure form.

Compounds of Formula **I** can be prepared by reacting a compound of Formula **II**: 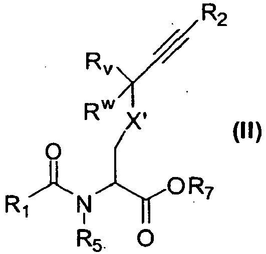 (where R₇ is preferably H) with a peptide coupling reagent to form an activated intermediate and reacting (separately or in the same vessel or "single pot") said activated intermediate in a polar aprotic solvent with a compound of **Formula III:** 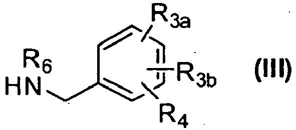 to produce said compound of **Formula I.** Any suitable solvent can be used, including tetrahydrofuran, dimethylformamide, diethyl ether, dichloromethane, mixtures thereof, etc. In some embodiments the polar aprotic solvent is anhydrous. In some embodiments, the reaction temperature is from -80 to 25 °C.

The compounds of **Formula II** are useful as intermediates for making compounds of **Formula I** as described herein. Compounds of Formula **II** (wherein R₇ is H or alkyl) are in turn made by reacting a compound of Formula IV: 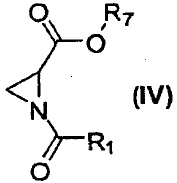 with a ring opening reagent (e.g., a Lewis acid) in an aprotic solvent (e.g., dichloromethylene) with a compound of **Formula V:** 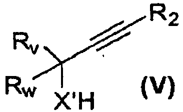 to produce the compound of **Formula II**. When R₇ is alkyl in compounds of Formula II, the alkyl can be converted to H in accordance with known techniques.

### C. Active compounds of Formula Ia.

As noted above, the present specification inventions compounds of Formula **Ia** (which fall outside the appended claims): 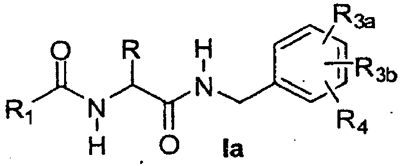 wherein:
R is R₂ or CH₂X'R₅, where X' is O, S, or N-R₆ₐ and R₆ₐ is hydrogen, alkyl, or cycloalkyl;
R, is alkyl or cycloalkyl, each of which can be unsubstituted or substituted with one or more (e.g., one, two, three, four) independently selected electron-donating or electron-withdrawing groups;
R₂ is alkyl, cycloalkyl, aryl, five- or six-membered cyclic heterocyclic or heteroaromatic groups, or -X-Y-Z, where X and Y = O, S, N-R₆, and Z = R₆, and where R₆ is hydrogen, alkyl, or cycloalkyl, each of which is unsubstituted or substituted with one or more (e.g., one, two, three, four) electron withdrawing or electron donating groups;
R₅ is alkyl, alkenyl, alkynyl, or arylalkyl, each of which is unsubstituted or substituted with one or more (e.g., one, two, three, four) electron donating or electron withdrawing groups;
R₃ₐ R_{3b}, and R₄ are each independently hydrogen, an electron donating or electron-withdrawing group, or alkyl, cycloalkyl, alkenyl, alkynyl, aryl, heterocyclo, heteroaryl, arylalkyl, heteroarylalkyl, heterocycloalkyl, arylalkyloxy, arylalkylamino, arylalkylthio, heteroarylalkyloxy, heteroarylalkylamino, heteroarylalkylthio, heterocycloalkyloxy, heterocycloalkylamino, heterocycloalkylthio, arylaminooxy, heteroarylaminooxy, heterocycloaminooxy, aryloxyamino, heteroaryloxyamino, heterocyclooxyamino, arylalkyl, heteroarylalkyl, heterocycloalkyl, arylalkenyl, heteroarylalkenyl, heterocycloalkenyl, arylalkynyl, heteroarylalkynyl, heterocycloalkynyl, arylhydrazino, heteroarylhydrazino, heterocyclohydrazino, arylazo, heteroarylazo, heterocycloazo, arylalkylaminoalkyl, heteroarylalkylaminoalkyl, heterocycloalkylaminoalkyl, arylalkyloxyalkyl, heteroarylalkyloxyalkyl, heterocycloalkyloxyalkyl, each of which can be unsubstituted or substituted with one or more (e.g., one, two, three, four) independently selected electron-donating or electron-withdrawing groups;
optionally but preferably wherein at least one of R₃ₐ and R₄ is not H;
and optionally but preferably wherein at least one, or both, of R₃ₐ and R₄ is a primary substituent selected from the group consisting of electron donating groups, electron-withdrawing groups, alkyl, cycloalkyl, alkenyl, alkynyl, aryl, heterocyclo, heteroaryl, arylalkyl, heteroarylalkyl, heterocycloalkyl, arylalkyloxy, arylalkylamino, arylalkylthio, heteroarylalkyloxy, heteroaryalkylamino, heterocycloalkyloxy, and heterocycloalkylamino, heterocycloalkylthio, heteroarylalkylthio, arylaminooxy, heteroarylaminooxy, heterocycloaminooxy, aryloxyamino, heteroaryloxyamino, heterocyclooxyamino, arylalkyl, heteroarylalkyl, heterocycloalkyl, arylalkenyl, heteroarylalkenyl, heterocycloalkenyl, arylalkynyl, heteroarylalkynyl, heterocycloalkynyl, arylhydrazino, heteroarylhydrazino, heterocyclohydrazino, arylazo, heteroarylazo, heterocycloazo, arylalkylaminoalkyl, heteroarylalkylaminoalkyl, heterocycloalkylaminoalkyl, arylalkyloxyalkyl, heteroarylalkyloxyalkyl, heterocycloalkyloxyalkyl;
and which primary substituent is in turn optionally but preferably substituted with one or more (e.g., one, two, three, four) secondary substituents independently selected from the group consisting of electron-donating groups, and electron-withdrawing groups (*e.g.,* aryl such as phenyl); which secondary substituent can in turn be unsubstituted or substituted with one or more (e.g., one, two, three, four) additional independently selected electron-donating or electron-withdrawing groups (e.g., halo such as fluoro), *etc.*
or a pharmaceutically acceptable salt thereof.

In some embodiments of the foregoing, R is R₂. In other embodiments of the foregoing, R is CH₂X'R₅.

In some embodiments of the foregoing, R_{3b} is H.

In some embodiments of the foregoing, R₁ is CH₃.

In some embodiments of the foregoing, R₂ is 2-furan, 2-thiazole, 2-oxazole, 2-pyridine, 2-pyrimidine, 2-pyridazine, N(H)OCH₃, and N(CH₃)OCH₃, or N(H)N(H)CO₂CH₃.

In some embodiments of the foregoing, wherein X is O, and R₅ is CH₃, CH₂CH₃, CH(CH₃)₂, CH₂CH₂CHCH₂, CH₂CCH.

In some embodiments of the foregoing, either or both of R₃ₐ and R₄ are OCH₂C₆H₄(*m*-F), OCF₃, N₃, CCCH₂OCH₃, CH₂OCH₃, (CH₂)₃OCH₃, C(N2)CF₃, C(O)C₆H₅ or CF₃.

Additional representative examples of R₃ₐ R_{3b} and/or R₄ include OC(H)ₘ(W)ₙ where W is a typical electron withdrawing or electron donating group and m can vary from 0-2 and n can vary from 1-3, CC-(CH₂)ₒOR₆ and o can vary from 1-6, (CH₂)oOR₆, 1-trifluorodiazinryl, azide, XCH₂C₆H₅, and where each of these atypical groups can be unsubstituted or substituted with one or more typical electron withdrawing and/or electron donating groups, or C(H)ₘ₍W)ₙ where W is a typical electron withdrawing or electron donating group and m can vary from 0-2 and n can vary from 1-3.

Specific examples of the foregoing include, but are not limited to: 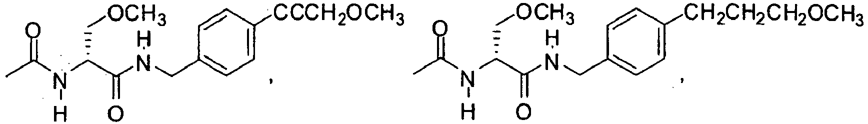 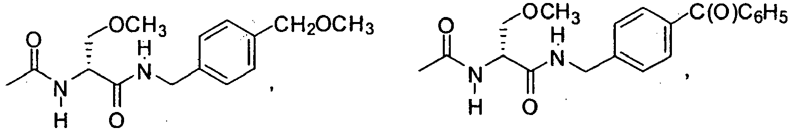 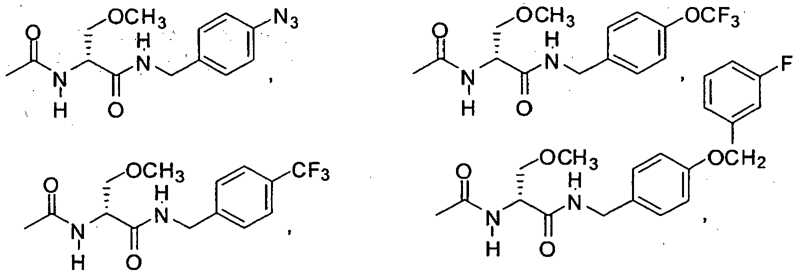 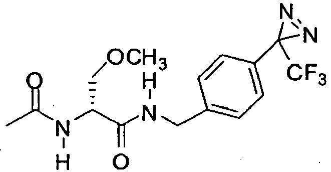 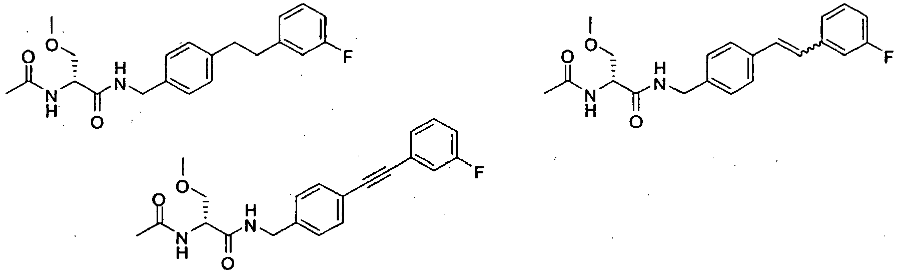 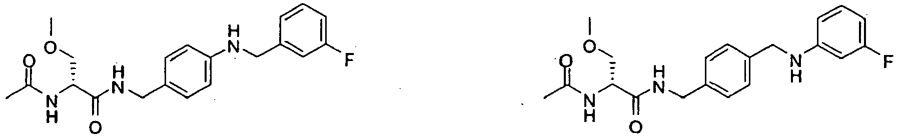 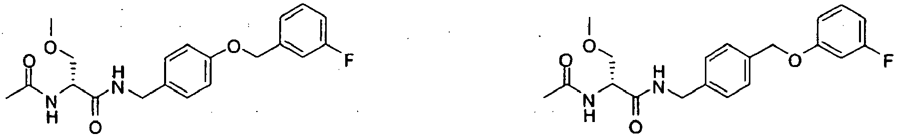 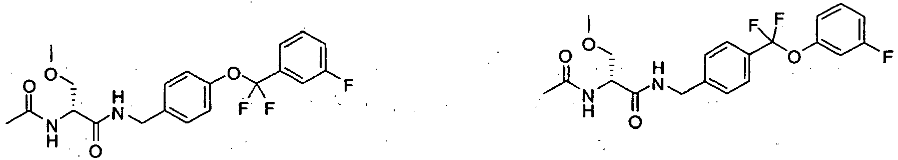 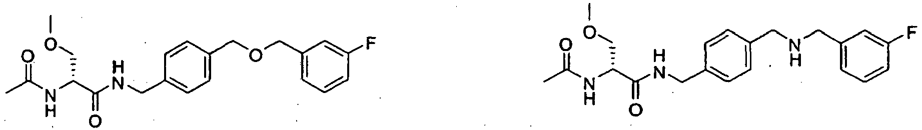 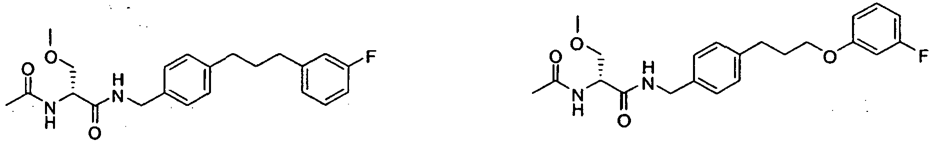 and pharmaceutically acceptable salts thereof.

In some embodiments of the foregoing, the active compounds are provided in the (R)-configuration.

In some embodiments of the foregoing, the active compounds are provided in isolated and/or purified form, or substantially pure form.

Compounds of the present invention can be prepared in accordance with known techniques or variations thereof that will be apparent to those skilled in the art based on the present disclosure.

### D. Pharmaceutical formulations.

The active compounds disclosed herein can, as noted above, be prepared in the form of their pharmaceutically acceptable salts. Pharmaceutically acceptable salts are salts that retain the desired biological activity of the parent compound and do not impart undesired toxicological effects. Examples of such salts are (a) acid addition salts formed with inorganic acids, for example hydrochloric acid, hydrobromic acid, sulfuric acid, phosphoric acid, nitric acid and the like; and salts formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, palmitic acid, alginic acid, polyglutamic acid, naphthalenesulfonic acid, methanesulfonic acid, p-toluenesulfonic acid, naphthalenedisulfonic acid, polygalacturonic acid, and the like; (b) salts formed from elemental anions such as chlorine, bromine, and iodine, and (c) salts derived from bases, such as ammonium salts, alkali metal salts such as those of sodium and potassium, alkaline earth metal salts such as those of calcium and magnesium, and salts with organic bases such as dicyclohexylamine and N-methyl-D-glucamine.

As also noted above, the compounds of the invention can be prepared as pharmaceutically acceptable prodrugs. Pharmaceutically acceptable prodrugs are those prodrugs of the compounds of the present invention which are, within the scope of sound medical judgment, suitable for use in contact with the tissues of humans and lower animals without undue toxicity, irritation, allergic response and the like, commensurate with a reasonable risk/benefit ratio, and effective for their intended use, as well as the zwitterionic forms, where possible, of the compounds of the invention. The term "prodrug" refers to compounds that are rapidly transformed in vivo to yield the parent compound of the above formulae, for example, by hydrolysis in blood. A thorough discussion is provided in T. Higuchi and V. Stella, Prodrugs as Novel delivery Systems, Vol. 14 of the A.C.S. Symposium Series and in Edward B. Roche, ed., Bioreversible Carriers in Drug Design, American Pharmaceutical Association and Pergamon Press, 1987, both of which are incorporated by reference herein. *See also* US Patent No. 6,680,299 Examples include a prodrug that is metabolized in vivo by a subject to an active drug having an activity of active compounds as described herein, wherein the prodrug is an ester of an alcohol or carboxylic acid group, if such a group is present in the compound; an acetal or ketal of an alcohol group, if such a group is present in the compound; an N-Mannich base or an imine of an amine group, if such a group is present in the compound; or a Schiff base, oxime, acetal, enol ester, oxazolidine, or thiazolidine of a carbonyl group, if such a group is present in the compound, such as described in US Patent No. 6,680,324 and US Patent No. 6,680,322.

The active compounds described above may be formulated for administration in a pharmaceutical carrier in accordance with known techniques. See, *e.g.,* Remington, The Science And Practice of Pharmacy (9th Ed. 1995). In the manufacture of a pharmaceutical formulation according to the invention, the active compound (including the physiologically acceptable salts thereof) is typically admixed with, *inter alia,* an acceptable carrier. The carrier must, of course, be acceptable in the sense of being compatible with any other ingredients in the formulation and must not be deleterious to the patient. The carrier may be a solid or a liquid, or both, and is preferably formulated with the compound as a unit-dose formulation, for example, a tablet, which may contain from 0.01 or 0.5% to 95% or 99% by weight of the active compound. One or more active compounds may be incorporated in the formulations of the invention, which may be prepared by any of the well known techniques of pharmacy comprising admixing the components, optionally including one or more accessory ingredients.

The formulations of the invention include those suitable for oral, rectal, topical, buccal (e.g., sub-lingual), vaginal, parenteral (e.g., subcutaneous, intramuscular, intradermal, or intravenous), topical (*i.e.,* both skin and mucosal surfaces, including airway surfaces such as by nasal administration or inhalation administration to nasal passages and/or to the lungs) and transdermal administration, although the most suitable route in any given case will depend on the nature and severity of the condition being treated and on the nature of the particular active compound which is being used.

Formulations suitable for oral administration may be presented in discrete units, such as capsules, cachets, lozenges, or tablets, each containing a predetermined amount of the active compound; as a powder or granules; as a solution or a suspension in an aqueous or non-aqueous liquid; or as an oil-in-water or water-in-oil emulsion. Such formulations may be prepared by any suitable method of pharmacy which includes the step of bringing into association the active compound and a suitable carrier (which may contain one or more accessory ingredients as noted above). In general, the formulations of the invention are prepared by uniformly and intimately admixing the active compound with a liquid or finely divided solid carrier, or both, and then, if necessary, shaping the resulting mixture. For example, a tablet may be prepared by compressing or molding a powder or granules containing the active compound, optionally with one or more accessory ingredients. Compressed tablets may be prepared by compressing, in a suitable machine, the compound in a free-flowing form, such as a powder or granules optionally mixed with a binder, lubricant, inert diluent, and/or surface active/dispersing agent(s). Molded tablets may be made by molding, in a suitable machine, the powdered compound moistened with an inert liquid binder.

Formulations suitable for buccal (sub-lingual) administration include lozenges comprising the active compound in a flavoured base, usually sucrose and acacia or tragacanth; and pastilles comprising the compound in an inert base such as gelatin and glycerin or sucrose and acacia.

Formulations of the present invention suitable for parenteral administration comprise sterile aqueous and non-aqueous injection solutions of the active compound(s), which preparations are preferably isotonic with the blood of the intended recipient. These preparations may contain anti-oxidants, buffers, bacteriostats and solutes which render the formulation isotonic with the blood of the intended recipient. Aqueous and non-aqueous sterile suspensions may include suspending agents and thickening agents. The formulations may be presented in unit\dose or multi-dose containers, for example sealed ampoules and vials, and may be stored in a freeze-dried (lyophilized) condition requiring only the addition of the sterile liquid carrier, for example, saline or water-for-injection immediately prior to use. Extemporaneous injection solutions and suspensions may be prepared from sterile powders, granules and tablets of the kind previously described. For example, in one aspect of the present invention, there is provided an injectable, stable, sterile composition comprising an active compound(s), or a salt thereof, in a unit dosage form in a sealed container. The compound or salt is provided in the form of a lyophilizate which is capable of being reconstituted with a suitable pharmaceutically acceptable carrier to form a liquid composition suitable for injection thereof into a subject. The unit dosage form typically comprises from about 10 mg to about 10 grams of the compound or salt. When the compound or salt is substantially water-insoluble, a sufficient amount of emulsifying agent which is physiologically acceptable may be employed in sufficient quantity to emulsify the compound or salt in an aqueous carrier. One such useful emulsifying agent is phosphatidyl choline.

Formulations suitable for rectal administration are preferably presented as unit dose suppositories. These may be prepared by admixing the active compound with one or more conventional solid carriers, for example, cocoa butter, and then shaping the resulting mixture.

Formulations suitable for topical application preferably take the form of an ointment, cream, lotion, paste, gel, spray, aerosol, or oil. Carriers which may be used include petroleum jelly, lanoline, polyethylene glycols, alcohols, transdermal enhancers, and combinations of two or more thereof. Formulations for inhalation administration may be administered as aqueous or dry powder particles by any suitable means, such as by a nebulizer or dry powder inhaler).

Formulations suitable for transdermal administration may be presented as discrete patches adapted to remain in intimate contact with the epidermis of the recipient for a prolonged period of time. Formulations suitable for transdermal administration may also be delivered by iontophoresis (see, for example, Pharmaceutical Research 3 (6):318 (1986)) and typically take the form of an optionally buffered aqueous solution of the active compound. Suitable formulations comprise citrate or bis\tris buffer (pH 6) or ethanol/water and contain from 0.1 to 0.2M active ingredient.

Further, the present invention provides liposomal formulations of the compounds disclosed herein and salts thereof. The technology for forming liposomal suspensions is well known in the art. When the compound or salt thereof is an aqueous-soluble salt, using conventional liposome technology, the same may be incorporated into lipid vesicles. In such an instance, due to the water solubility of the compound or salt, the compound or salt will be substantially entrained within the hydrophilic center or core of the liposomes. The lipid layer employed may be of any conventional composition and may either contain cholesterol or may be cholesterol-free. When the compound or salt of interest is water-insoluble, again employing conventional liposome formation technology, the salt may be substantially entrained within the hydrophobic lipid bilayer which forms the structure of the liposome. In either instance, the liposomes which are produced may be reduced in size, as through the use of standard sonication and homogenization techniques.

Of course, the liposomal formulations containing the compounds disclosed herein or salts thereof, may be lyophilized to produce a lyophilizate which may be reconstituted with a pharmaceutically acceptable carrier, such as water, to regenerate a liposomal suspension.

Other pharmaceutical compositions may be prepared from the water-insoluble compounds disclosed herein, or salts thereof, such as aqueous base emulsions. In such an instance, the composition will contain a sufficient amount of pharmaceutically acceptable emulsifying agent to emulsify the desired amount of the compound or salt thereof. Particularly useful emulsifying agents include phosphatidyl cholines, and lecithin.

The formulations may be prepared as "timed release" formulations, e.g., by combining or mixing the active compound with a biodegradable or bioerodable polymer, by encapsulating the active compounds in a biodegradable capsule or with an enteric coating, or any other suitable technique

In addition to active compound(s), the pharmaceutical compositions may contain other additives, such as pH-adjusting additives. In particular, useful pH-adjusting agents include acids, such as hydrochloric acid, bases or buffers, such as sodium lactate, sodium acetate, sodium phosphate, sodium citrate, sodium borate, or sodium gluconate. Further, the compositions may contain microbial preservatives. Useful microbial preservatives include methylparaben, propylparaben, and benzyl alcohol. The microbial preservative is typically employed when the formulation is placed in a vial designed for multidose use. Of course, as indicated, the pharmaceutical compositions of the present invention may be lyophilized using techniques well known in the art.

### E. Dosage and routes of administration.

As noted above, the present invention provides pharmaceutical formulations comprising the active compounds (including the pharmaceutically acceptable salts thereof), in pharmaceutically acceptable carriers for oral, inhalation, rectal, topical, buccal, parenteral, intramuscular, intradermal, intravenous, and transdermal administration.

The therapeutically effective dosage of any specific compound, the use of which is in the scope of present invention, will vary somewhat from compound to compound, and patient to patient, and will depend upon the condition of the patient and the route of delivery. In some embodiments, a dosage from about 0.1 or 1 up to about 1 or 2 g/patient will have therapeutic efficacy, with all weights being calculated based upon the weight of the active compound, including the cases where a salt is employed. Toxicity concerns at the higher level may restrict intravenous dosages to a lower level such as up to about 1 or 2 g/patient, with all weights being calculated based upon the weight of the active base, including the cases where a salt is employed.

For an oral administration form, the dosage may be from 50, 100 or 200 mg per subject up to 1 or 2 grams per subject, once or twice a day.

### F. Combination compositions and treatment.

The compounds above can be administered alone or in combination (concurrently) with the administration of other neurological active agents. The other neurological active agent can be administered separately, or included in the compositions described above that also contain the active agents described above.

Examples of other neurological active agents for the treatment of epilepsy, seizure disorders and the like, and which can be used to carry out the present invention, include but are not limited to: felbamate, gabapentin, lamotrigine, topiramate, tiagabine, levetiracetam, zonisamide, fosphenytoin, carbamazepine, oxcarbazepine, valproate sodium, diazepam, midazolam, propofol, ethosuximide, phensuximide, methsuximide, clonazepam, clorazepate dipotassium, phenytoin, mephenytoin, ethotoin, phenobarbital, mephobarbital, pyrimidone, pregabilin, etc. Such compounds may be administered to the subjects, or included in the compositions described above, in amounts known in the art.

Examples of other neurological active agents for the treatment of pain such as neuropathic pain, and which can be used to carry out the present invention, include but are not limited to: tricyclic antidepressants, serotonin reuptake inhibitors, anticonvulsants, nonopioid analgesics, etc. Particular examples include but are not limited to amitriptyline, desipramine, nortriptyline, fluoxetine, citalopram, venlafaxine, carbamazepine, phenytoin, gabapentin, lamotrigine, pregabilin, etc. Such compounds may be administered to the subjects, or included in the compositions described above, in amounts known in the art.

The present invention is explained in greater detail in the following non-limiting Examples.

### Examples for Active Compounds of Formula I

**The structure-activity relationship of functionalized amino acids (1).** In 1985, we reported that functionalized amino acids (FAA; 1) were potent anticonvulsant agents that provided significant protection against maximal electroshock (MES)-induced seizures in mice. This finding led us to conduct a tightly focused structure-activity relationship (SAR) study (*See*, *e.g*., Choi, D.; Stables, J.P.; Kohn, H. Synthesis and anticonvulsant activities of N-benzyl-2-acetamidopropionamide derivatives. J. Med. Chem. 1996, 39, 1907-1916).

More than 250 FAAs **(1)** have been synthesized in our laboratory and screened in animal model systems. The majority of our compounds were evaluated at the NINDS under an Epilepsy Branch initiative (Anticonvulsant Screening Project (ASP)). The Eli Lilly Laboratories (Indianapolis, IN) played a role in an early phase of this program. The results of our studies have led others to report the pharmacological activities of additional FAAs. Most sites (*e.g*., R¹, R², R³) in 1 have been modified. We learned that the highest activity for R¹ was methyl (CH₃), but we and others have shown that significant activity is obtained with the parent amine (See*, e.g.,* Paruszewski, R.; Rostafinska-Suchar, G.; Strupinska, M.; Jaworski, P.; Stables, J.P. Synthesis and anticonvulsant activity of some amino acid derivative. Pharmazie 1996, 3, 145-148. (b) Paruszewski, R.; Rostafinska-Suchar, G.; Strupinska, M.; Jaworski, P,; Winiecka, I.; Stables, J.P. Synthesis and anticonvulsant activity of some amino acid derivatives. Pharmazie 1996, 51, 212-215; Andurkar, S.V.; Stables, J.P.; Kohn, H. Synthesis and anticonvulsant activities of (R)-(O)-methylserine derivatives. Tetrahedron: Asymmetry 1998, 9, 3841-3854; Béguin, C.; LeTiran, A.; Stables, J.P.; Voyksner, R.D.; Kohn, H. N-Substituted amino acid N-benzylamides: synthesis, anticonvulsant, and metabolic activities. Biorg. Med. Chem. 2004, 12, 3079-3096).

Our initial studies indicated that the optimal R³ substituent is benzyl (PhCH₂). Finally, isoelectronic substitution of the amide carbonyl (site a) with a thiocarbonyl group, insertion of an alkyl unit or deletion of the chiral center in 1 (site b), and replacement of the amide hydrogen with an alkyl group (site c) reduced anticonvulsant activity.

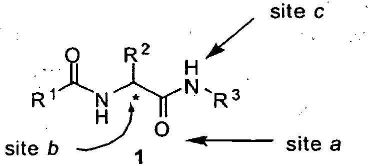

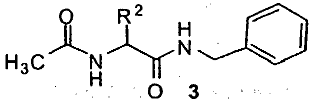

The stringent structural requirements for 1 (R¹, R³, sites *a*-*c*) led us to focus on molecular template 3. More than 150 compounds conforming to this structure, in which only R² was modified, have been examined. We have observed impressive anticonvulsant activities in mice and rats for *small* R² groups (e.g., 2-furanyl, 2-pyrrolyl, 1-pyrazolyl, 2-oxazolyl, 2-thiazolyl, 2-pyridyl, 2-pyrimidyl, 2-pyrazinyl, *O-*methylhydroxylamino, *N*,*O*-dimethylhydroxylamino) that contained a substituted heteroatom, one atom removed from the chiral carbon. We found that *all* of these compounds exhibited activity that was either comparable with or exceeded that of phenytoin (MES ED₅₀ = 9.5 mg/kg) in the maximal electroshock (MES)-induced seizure test. This test is the established method used to identify new drug candidates for the treatment of generalized and partial seizures that are secondarily generalized, and compounds that prevent seizure spread; phenytoin is considered the prototype.

One structural feature of the SAR dominated all others. For compounds **4, 5, 6,** and **2,** we independently evaluated the two stereoisomers. We showed that the anticonvulsant activity resided in the (R)-enantiomer. The ratio of the potency of the more active to the less active isomer (Eudismic ratio = ratio of activities of the two enantiomers. Lehmann, P.A. Trends Pharmacol. Sci. 1982, 3, 103-106) ranged from 10 to >22 **(Table** 1). These differences are among the highest, if not the highest, reported for MES-selective anticonvulsants. These findings coupled with the demonstration that comparable concentration levels of (*R*)- and (S)-**5** were found in the brains of mice (Leander, J.D.; Robertson, D.W. (Eli Lilly Laboratory) unpublished results) suggested that the antiseizure properties of 1 resulted from interaction at a specific protein target site(s).

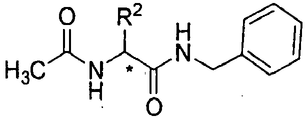

4 R² = CH₃

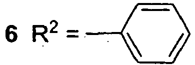

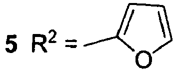

2 R² = CH₂OCH₃

**Table 1. Pharmacological data for FAA (1) stereoisomers**

| **No.** | **R²** | **MES,^{a} ED₅₀^{b}** | **No.** | **R²** | **MES,^{a} ED₅₀^{b}** |
|---|---|---|---|---|---|
| (*R*,*S*)-4 | CH₃ | 77 (67-89) | (R,S)**-6** | phenyl | 32 |
| *(R)-***4** | CH₃ | 55 (50-60) | (*R*)-**6** | phenyl | 26 (21-32) |
| (*S*)-**4** | CH₃ | 550 (460-740) | (*S*)-**6** | phenyl | > 300 |
| (*R*,S)-**5** | 2-furan | 10 (9.1-12) | (*R,S*)-**2** | CH₂OCH₃ | 8.3 (7.9-9.8) |
| (R)-**5** | 2-furan | 3.3 (2.8-3.9) | *(R)-2* | CH₂OCH₃ | 4.5 (3.7-5.5) |
| (*S*)-**5** | 2-furan | > 25 | (*S*)**-2** | CH₂OCH₃ | > 100, < 300 |

| | | | | | |
|---|---|---|---|---|---|
| ^{a}MES = maximal electroshock seizure test. The compounds were administered intraperitoneally (ip) to mice. ED₅₀ values are in mg/kg. Numbers in parentheses are 95% confidence intervals. ^{b}The ED₅₀ values for **2, 4,** and **6** were determined at the NIH Epilepsy Branch and for 5 at the Lilly Research Laboratories. | | | | | |

**Results.** Recent studies in our laboratory have led to an unexpected discovery. We prepared the (R)- and (S)- stereoisomers corresponding to **7, 8** and **9 (Scheme 1)** and determined their pharmacological activity at the NIH ASP. We observed that **7** and **8** exhibited excellent seizure activity in the MES and 6 Hz seizure model assays that was comparable to or exceeded that observed for phenytoin and phenobarbital in mice (ip) and rats (po) **(Table 2).** 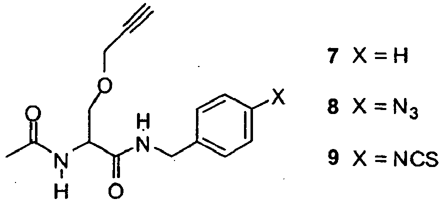 The finding was surprising since earlier studies showed a significant decrease in seizure protection as we increased the size of the oxygen substituent located one atom removed from the C(2) site. For example, the MES ED₅₀ values (mice, ip) for (*R*,*S*)-**2** (*O*-methyl),

(*R*,*S*)-**10** (*O*-ethyl) and (*R,S*)-11 (*O*-allyl) were 8.3, 17, and >30, <100 mg/kg, respectively. Moreover, we found that high pharmacological activity was retained in this propargyl series even upon substitution of the *N*-benzyl amide group in 7 with an azido moiety to give **8** or an isothiocyanate group to give 9. Similar to results obtained with other FAAs, the pharmacological activity for **7-9** principally resided in the stereoisomer corresponding to the D-stereoisomer **(Table 2)**.

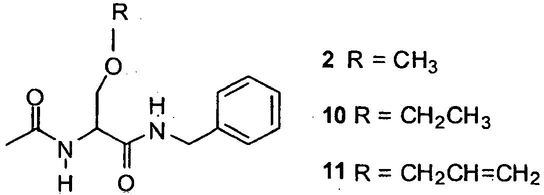

The anticonvulsant activities of the *O*-propargyl FAAs 12 was not anticipated and is not embraced in previous patent applications where the *O*-substituent is restricted to lower alkyl and aryl with said lower alkyl or aryl being substituted or unsubstituted (U.S. Patents 5,378,729, 5,654,301; RE38,551E; 6,048,899).

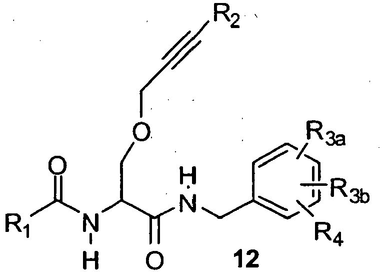

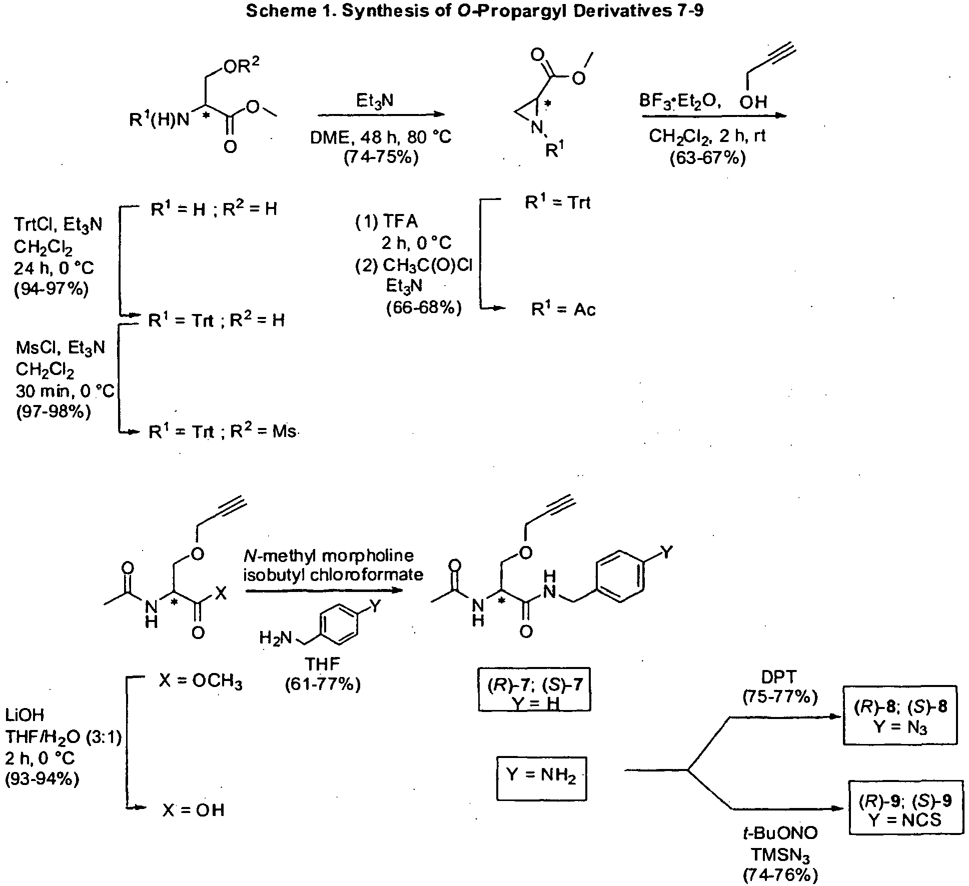

**Table 2. Selected Physical and Pharmacological Data for O-Propargyl Derivatives 7-9 and their Reference Compounds**

| | | Mice (ip)*^{a}* | | | Rat (po)*^{b}* | | |
|---|---|---|---|---|---|---|---|
| compd | mp*^{c}* | MES,*^{d}* ED₅₀ | Tox,*^{e}* TD₅₀ | PI*^{f}* | MES,*^{d}* ED₅₀ | Tox,*^{e}* TD₅₀ | PI*^{f}* |
| (*R*)-**7** | 149 | 16 [0.25] | 59 [0.25] | 3.7 | 8 [0.5] | > 500 | > 63 |
| | | (13 - 19) | (55 - 66) | | (5 - 11) | | |
| (*S*)-**7** | 148 - 149 | > 300 | > 300 | | > 30 | > 30 | |
| (*R*)-**8** | 135 - 136 | 20 [0.25] | 82 [0.25] | 4.0 | 12 [1] | > 500 | > 41 |
| | | (18 - 23) | (63 - 108) | | (10 - 16) | | |
| (*S*)-**8** | 135-136 | 163 [0.25] | > 300 | | > 30 | > 30 | |
| | | (105 - 225) | | | | | |
| *(R)-***9** | 157 - 159 | 44.6 [1] | 109.5 [0.25] | 2.5 | > 30 | > 30 | |
| | | (41 - 48) | (76 - 155) | | | | |
| (*S*)-**9** | 158-159 | > 300 | > 300 | | | | |
| phenytoin *^{g}* | | 9.5 [2] | 66 [2] | 6.9 | 30 [4] | *h* | > 100 |
| | | (8 - 10) | (53 - 72) | | (22 - 39) | | |
| Phenobarbital *^{g}* | | 22 [1] | 69 [0.5] | 3.2 | 9 [5] | 61 [0.5] | 6.7 |
| | | (15 - 23) | (63 - 73) | | (7.6 - 12) | (44 - 96) | |
| valproate *^{g}* | | 270 [0.25] | 430 [0.25] | 1.6 | 490 [0.5] | 280 [0.5] | 0.6 |
| | | (250 - 340) | (370 - 450) | | (350 - 730) | (190 - 350) | |

| | | | | | | | |
|---|---|---|---|---|---|---|---|
| *^{a}* The compounds were administered intraperitoneally. ED₅₀ and TD₅₀ values are in mg/kg. Numbers in parentheses are 95% confidence intervals. The dose effect data was obtained at the "time of peak effect" (indicated in hours in the brackets). *^{b}* The compounds were administered orally. *^{c}* Melting points (°C). *^{d}* MES = maximal electroshock seizure test. *^{e}* Tox = neurologic toxicity determined from rotorod test. *^{f}*PI = protective index (TD₅₀/ED₅₀). *^{g}* Reference compounds. *^{h}*No ataxia observed up to 3000 mg/kg. | | | | | | | |

### EXPERIMENTAL SECTION

**(*R*)-Methyl 3-Hydroxy-2-(*N*-tritylamino)propanoate** (S. Bhatia and J. Hajdu, Tetrahedron Lett. 1998, 29, 31-34). To a solution of D-serine methyl ester hydrochloride (20.00 g, 0.13 mol) and Et₃N (35.82 mL, 0.26 mol) in CH₂Cl₂ (80 mL) at 0 °C, was added in one portion a solution of TrCl (36.53 g, 0.13 mol) in CH₂Cl₂ (80 mL). The mixture was allowed to stir at 0 °C (24 h) under Ar and then successively washed with 10% aqueous citric acid (120 mL) and saturated aqueous brine (120 mL). The organic layer was dried (Na₂SO₄) and evaporated in vacuo to yield 45.20 g (97%) of crude desired product as a pale yellow crystalline solid. The product was used for next step without further purification: *R_{f}*=0.50 (1/1 EtOAc/hexanes); ¹H NMR (CDCl₃) *δ* 2.29 (br s, 1 H), 2.98 (br s, 1 H), 3.29 (s, OCH₃), 3.51-3.60 (m, CHH'OH, CH), 3.64-3.74 (m, CHH'OH), 7.16-7.30 (m, 9 PhH), 7.47-7.50 (m, 6 PhH).

**(*S*)-Methyl 3-Hydroxy-2-(*N*-tritylamino)propanoate** (U. Larsson and R. Carlson, Acta Chem. Scand. 1994, 48, 511-516; M. Meyer et al., J. Am. Chem. Soc. 1997, 119, 10093-10103). Using the preceding procedure and using L-serine methyl ester hydrochloride (20.00 g, 0.13 mol), Et₃N (35.82 mL, 0.26 mol) and TrCl (36.53 g, 0.13 mol) gave 43.80 g (94%) of crude desired product as a pale yellow crystalline solid: *R_{f}* = 0.50 (1/1 EtOAc/hexanes); ¹H NMR (CDCl₃) *δ* 2.31 (br s, 1 H), 2.84-3.02 (br m, 1 H), 3.29 (s, OC**H**₃), 3.50-3.59 (m, CHH'OH, CH), 3.63-3.74 (m, CHH'OH), 7.18-7.29 (m, 9 PhH), 7.48-7.50 (m, 6 PhH).

**(R)-Methyl 1-Tritylaziridine-2-carboxylate** (S. Kato et al., J. Chem. Soc., Perkin Trans. 1997, 1, 3219-3225; J. Smrt et al., Experientia 1957, 15, 291). Crude (R)-methyl 3-hydroxy-2-(N-tritylamino)propanoate (45.20 g, 0.13 mol) was dissolved in CH₂Cl₂ (300 mL) and cooled to 0 °C under Ar. Methanesulfonyl chloride (10.64 mL, 0.14 mol) was added to the cooled solution, followed by the dropwise addition of Et₃N (26.14 mL, 0.19 mol). The resulting solution was allowed to stir at 0 °C (30 min) and then successively washed with 10% aqueous citric acid (200 mL) and saturated aqueous brine (200 mL). After drying (Na₂SO₄) and evaporation of the solvent, the crude mesylate (53.30 g, 0.12 mol) was dissolved in DME (300 mL) and Et₃N (33.73 mL, 0.24 mol) was added. The reaction mixture was stirred at 80 °C (48 h) and then evaporated in vacuo. The residue was dissolved in EtOAc (300 mL) and washed with 10% aqueous citric acid (200 mL) and saturated aqueous brine (200 mL). The organic layer was dried (Na₂SO₄) and evaporated in vacuo. The crude product was recrystallized (EtOH) to yield 30.81 g (74%) of desired product as a transparent crystal: mp 127-128 °C (Kato, S. et al., J. Chem. Soc., Perkin Trans. 1997, 1, 3219-3225; mp 129-131 °C); [α]²⁰_{D} +94.7° (c 1.5, CHCl₃) (Smrt, J. et al., Experientia 1957, 15, 291; [α]²⁰_{D} +95 (c 1, CHCl₃)); *R_{f}* = 0.45 (1/5 EtOAc/hexanes); ¹H NMR (CDCl₃) *δ* 1.41 (dd, *J* = 1.7, 6.4 Hz, NCHH'CH), 1.89 (dd, *J* = 2.8, 6.4 Hz, CHH'CHN), 2.26 (dd, *J* = 1.7, 2.8 Hz, NCH**H'**CH), 3.76 (s, OC**H**₃), 7.20-7.31 (m, 9 PhH), 7.48-7.52 (m, 6 PhH); ¹³C NMR (CDCl₃) *δ* 28.8 (NCH₂CH), 31.8 (CH₂CHN), 52.2 (OCH₃), 74.5 (N**C**Ph₃), 127.1, 127.8, 129.4, 143.7 (3 **C**₆H₅), 172.0 (C(O)).

**(*S*)-Methyl 1-Tritylaziridine-2-carboxylate** (U. Larsson et al., *supra*; S. Kato et al., *supra*; J. Smrt et al., *supra*). Using the preceding procedure and using crude (S)-methyl 3-hydroxy-2-(*N*-tritylamino)propanoate (43.80 g, 0.12 mol), methanesulfonyl chloride (10.30 mL, 0.13 mol) and Et₃N (25.30 mL, 0.18 mol) gave crude mesylate ((S)-2) (53.90 g, 0.12 mol), which was treated with Et₃N (34.29 mL, 0.25 mol) to yield 31.58 g (75%) of desired product as a transparent crystal: mp 127-128 °C (Kato, S. et al., J. Chem. Soc., Perkin Trans. 1997, 1, 3219-3225.; mp 130-131 °C); [α]²⁰_{D} -95.1 (c 1.5, CHCl₃) (Smrt, J. et al., Experientia 1957, 15, 291; [α]²⁰_{D} -94.2° (c 1, CHCl₃)); *R_{f}* = 0.45 (1/5 EtOAc/hexanes); ¹H NMR (CDCl₃) *δ* 1.41 (dd, *J*= 1.7, 6.4 Hz, NC**H**H'CH), 1.89 (dd, *J* = 2.8, 6.4 Hz, CHH'CHN), 2.26 (dd, *J* = 1.7, 2.8 Hz, NCHH'CH), 3.75 (s, OC**H**₃), 7.18-7.30 (m, 9 PhH), 7.48-7.52 (m, 6 PhH); ¹³C NMR (CDCl₃) *δ* 28.9 (N**C**H₂CH), 31.9 (CH₂**C**HN), 52.3 (O**C**H₃), 74.6 (N**C**Ph₃), 127.1, 127.8, 129.5, 143.8 (3 **C**₆H₅), 172.1 (C(O)).

**(*R*)-Methyl 1-Acetylaziridine-2-carboxylate** (P. Davoli et al., Tetrahedron: Asym. 1995, 6, 2011-2016). (R)-Methyl 1-tritylaziridine-2-carboxylate (9.00 g, 26.24 mmol) was dissolved in MeOH/CHCl₃ (1:1, 90 mL) and cooled to 0 °C under Ar and TFA (15.15 mL, 196.80 mmol) was added dropwise. After the reaction mixture was allowed to stir at 0 °C (2 h), the solvent was evaporated in vacuo. The product was dissolved in CH₂Cl₂ (120 mL), cooled to 0 °C, and Et₃N (18.29 mL, 131.20 mmol) and AcCl (2.04 mL, 28.86 mmol) were added in two portions over 5 min. The solution was stirred at 0 °C (1 h) and then washed with saturated aqueous NaHCO₃ (100 mL) and saturated aqueous brine (100 mL). The organic layer was dried (Na₂SO₄) and evaporated in vacuo, and then the crude product was purified by column chromatography (SiO₂; 1/1 EtOAc/hexanes) and crystallized (cold hexanes) to yield 2.46 g (66%) of desired product as a clear crystal: mp 39-40 °C; [α]²⁵_{D} +84.3° (c 1.2, CHCl₃); *R_{f}* = 0.30 (1/2 EtOAc/hexanes); ¹H NMR (CDCl₃) *δ* 2.16 (s, C**H**₃C(O)), 2.51 (dd, *J* = 1.8, 5.5 Hz, NCHH'CH), 2.58 (dd, *J* = 1.8, 3.0 Hz, NCH**H'**CH), 3.16 (dd, *J* = 3.0, 5.5 Hz, CHH'CHN), 3.80 (s, OC**H**₃); ¹³C NMR (CDCl₃) *δ* 23.7 (**C**H₃C(O)), 30.9 (N**C**H₂CH), 34.4 (CH₂**C**HN), 52.9 (O**C**H₃), 168.9 (C(O)O), 180.6 (**C**(O)N); HRMS (ESI) 166.0474 [M + Na⁺] (calcd. for C₆H₉NO₃Na 166.0480).

**(S)- Methyl 1-Acetylaziridine-2-carboxylate** (P. Davoli et al., *supra*)*.* Using (S)-methyl 1-tritylaziridine-2-carboxylate (9.00 g, 26.24 mmol), TFA (15.15 mL, 196.80 mmol), Et₃N (18.29 mL, 131.20 mmol) and AcCl (2.04 mL, 28.86 mmol), and the preceding procedure gave 2.56 g (68%) of desired product as a clear crystal: mp 39-40 °C; [α]²⁵_{D} -83.9° (c 1.2, CHCl₃); *R_{f}* = 0.30 (1/2 EtOAc/hexanes); ¹H NMR (CDCl₃) *δ* 2.17 (s, C**H**₃C(O)), 2.51 (dd, J = 1.8, 5.4 Hz, NC**H**H'CH), 2.58 (dd, J = 1.8, 2.9 Hz, NCH**H'**CH), 3.16 (dd, J = 2.9, 5.4 Hz, CHH'C**H**N), 3.80 (s, OC**H**₃); ¹³C NMR (CDCl₃) *δ* 23.7 (**C**H₃C(O)), 30.8 (N**C**H₂CH), 34.4 (CH₂**C**HN), 52.8 (O**C**H₃), 168.8 (**C**(O)O), 180.5 (**C**(O)N); HRMS (ESI) 166.0474 [M + Na⁺] (calcd. for C₆H₉NO₃Na 166.0480).

**(*R*)-Methyl 2-Acetamido-3-(prop-2-ynyloxy)propanoate.** (R)-Methyl 1-acetylaziridine-2-carboxylate (1.50 g, 10.49 mmol) was dissolved in CH₂Cl₂ (40 mL) and cooled to -20 °C under Ar, and then propargyl alcohol (0.92 mL, 15.74 mmol) was added. After the solution was stirred (2 min), BF₃·Et₂O (1.39 mL, 11.01 mmol) was added dropwise. The reaction mixture was allowed to stir at room temperature (2-3 h) and then washed with saturated aqueous NaHCO₃ (30 mL) and saturated aqueous brine (30 mL). The organic layer was dried (Na₂SO₄) and evaporated in vacuo, and then the crude product was purified by column chromatography (SiO₂; 3/1 EtOAc/hexanes) and crystallized (cold hexanes) to yield 1.43 g (68%) of desired product as a white solid: mp 58.5-59.5 °C; [α]²⁵_{D} -64.6° (c 1, CHCl₃); *R_{f}* = 0.35 (3/1 EtOAc/hexanes); IR (nujol mull) 3301, 2919, 2114, 1750, 1639, 1542, 1457 cm⁻¹; ¹H NMR (CDCl₃) δ 2.06 (s, C**H**₃C(O)), 2.46 (t, *J* = 2.3 Hz, CH₂C**CH**), 3.78 (s, OC**H**₃), 3.79 (dd, *J* = 3.3, 9.6 Hz, C**H**H'OCH₂), 3.98 (dd, *J* = 2.9, 9.6 Hz, CH**H**'OCH₂), 4.09-4.21 (m, OC**H**₂C), 4.77-4.82 (m, C**H**), 6.33-6.35 (br d, *J* = 7.2 Hz, NHCH); ¹³C NMR (CDCl₃) δ 23.3 (**C**H₃C(O)), 52.6 (CH), 52.8 (O**C**H₃), 58.7 (**C**H₂CCH), 69.6 (**C**H₂OCH₂), 75.3 (CH₂C**C**H), 79.0 (CH₂**C**CH), 170.1, 170.8 (2 **C**(O)); HRMS (ESI) 200.0916 [M + H⁺] (calcd. for C₉H₁₄NO₄ 200.0923); Anal. (C₉H₁₃NO₄) Calcd.: C, 54.26%; H, 6.58%; N, 7.03%. Found: C, 54.30%; H, 6.66%; N, 6.89%.

**(*S*)-Methyl 2-Acetamido-3-(prop-2-ynyloxy)propanoate.** Using (*S*)-methyl 1-acetylaziridine-2-carboxylate (1.50 g, 10.49 mmol), propargyl alcohol (0.92 mL, 15.74 mmol) and BF₃·Et₂O (1.39 mL, 11.01 mmol), and the preceding procedure gave 1.32 g (63%) of desired product as a white solid: mp 59-59.5 °C; [α]²⁵_{D} +65.3° (c 1, CHCl₃); *R_{f}* = 0.35 (3/1 EtOAc/hexanes); IR (nujol mull) 3296, 2924, 2116, 1740, 1641, 1543, 1457 cm⁻¹; ¹H NMR (CDCl₃) δ 2.06 (s, C**H**₃C(O)), 2.46 (t, *J* = 2.4 Hz, CH₂C**CH**), 3.78 (s, OC**H**₃), 3.79 (dd, *J* = 3.2, 9.4 Hz, C**H**H'OCH₂), 3.98 (dd, *J* = 3.0, 9.4 Hz, CH**H**'OCH₂), 4.12 (1/2**H**H'_{q}, *J* = 2.4, 16.2 Hz, OCHH'C), 4.18 (1/2H**H**'_{q}, *J* = 2.4, 16.2 Hz, OCHH'C), 4.77-4.82 (m, CH), 6.32-6.34 (br d, *J* = 7.2, NHCH); ¹³C NMR (CDCl₃) δ 23.3 (**C**H₃C(O)), 52.6 (**C**H), 52.8 (O**C**H₃), 58.7 (**C**H₂CCH), 69.6 (**C**H₂OCH₂), 75.3 (CH₂C**C**H), 79.0 (CH₂**C**CH), 170.1, 170.7 (2 **C**(O)); HRMS (ESI) 200.0917 [M + H⁺] (calcd. for C₉H₁₄NO₄ 200.0923); Anal. (C₉H₁₃NO₄) Calcd.: C, 54.26%; H, 6.58%; N, 7.03%. Found: C, 54.05%; H, 6.55%; N, 6.95%.

**(*R*)-2-Acetamido-3-(prop-2-ynyloxy)propanoic Acid.** (*R*)-Methyl 2-acetamido-3-(prop-2-ynyloxy)propanoate (1.00 g, 5.03 mmol) was dissolved in THF (24 mL) and cooled to 0 °C, and LiOH (aqueous 0.6 M sol., 8.4 mL, 5.03 mmol) was added. The reaction solution was stirred at 0 °C (2 h) and then concentrated in vacuo: The resulting aqueous phase was diluted with H₂O (12 mL) and washed with Et₂O (2 x 20 mL). The aqueous layer was acidified to pH 1-2 with aqueous 1 M HCl, and extracted with EtOAc (6 x 20 mL). The combined organic phases were washed with saturated aqueous brine (80 mL), dried (Na₂SO₄) and evaporated in vacuo to yield 0.87 g of desired product (94%) as a yellow oil: *R_{f}* = 0.35 (15/5/1 EtOAc/hexanes/AcOH); IR (nujol mull) 2924, 2120, 1726; 1642, 1548, 1459 cm⁻¹; ¹H NMR (CD₃OD) δ 2.01 (s, C**H**₃C(O)), 2.88 (t, *J* = 2.6 Hz, CH₂C**CH**), 3,77 (dd, *J =* 3.6, 97 Hz, C**H**H'OCH2), 3.92 (dd, *J* = 5.0, 9.7 Hz, CH**H**'OCH₂), 4.18 (d, *J* = 2.6 Hz, OC**H**₂C), 4.61-4.64 (m, CH); ¹³C NMR (CD₃OD) *δ* 22.5 (**C**H₃C(O)), 54.1 (CH), 59.3 (**C**H₂CCH), 70.4 (**C**H₂OCH₂), 76.5 (CH₂C**C**H), 80.2 (CH₂**C**CH), 173.1, 173.5 (2 **C**(O)); HRMS (ESI) 186.0761 [M + H⁺] (calcd. for C₈H₁₂NO₄ 186.0766); Anal. (C₈H₁₁NO_{4,} 0.14 H₂O) Calcd.: C, 51.18%; H, 6.06%; N, 7.46%. Found: C, 51.15%; H, 6.13%; N, 7.27%.

**(*S*)-2-Acetamido-3-(prop-2-ynyloxy)propanoic Acid.** Using (S)-methyl 2-acetamido-3-(prop-2-ynyloxy)propanoate (1.00 g, 5.03 mmol) and LiOH (aqueous 0.6 M sol., 8.4 mL, 5.03 mmol), and the preceding procedure gave 0.86 g (93%) of desired product as a yellow oil: *R_{f} =* 0.35 (15/5/1 EtOAc/hexanes/AcOH); IR (nujol mull) 3270, 2924, 2120, 1729, 1641, 1547, 1459 cm⁻¹; ¹H NMM (DMSO-*d*₆) *δ* 1.87 (s, C**H**₃C(O)), 3.48 (t, *J* = 2.4 Hz, CH₂C**CH**), 3.62 (dd, *J* = 4.1, 9.6 Hz, C**H**H'OCH₂), 3.74 (dd, *J* = 2.4, 9.6 Hz, CH**H**'OCH₂), 4.16 (d, *J* = 2.4 Hz, OC**H**₂C), 4.41-4.47 (m, CH), 8.21-8.23 (d, *J* = 8.4 Hz, NHCH); ¹³C NMR (DMSO-*d*₆) δ 22.4 (**C**H₃C(O)), 52.1 (CH), 57.8 (**C**H₂CCH), 69.2 (**C**H₂OCH₂), 77.6 (CH₂CCH), 79.9 (CH₂**C**CH), 169.6, 171.5 (2 **C**(O)); HRMS (ESI) 186.0760 [M + H⁺] (calcd. for C₈H₁₂NO₄ 186.0766); Anal. (C₈H₁₁NO₄, 0.25 H₂O) Calcd.: C, 50.66%; H, 6.11%; N, 7.38%. Found: C, 50.76%; H, 6.29%; N, 7.10%.

**General Procedure for the Preparation *N*-Benzylamide Amino Acids Derivatives Using Mixed-Anhydride Coupling (MAC) (Method A)** (G. Anderson et al., J. Am. Chem. Soc. 1967, 87, 5012-5017; D. Choi et al., J. Med. Chem. 1996, 39, 1907-1916). A dry THF solution of the carboxylic acid (∼0.2-1.0 M) was cooled to -78 °C under Ar, and 4-methylmorpholine (NMM) (1.3-1.5 equiv) was added. After stirring (2 min), isobutyl chloroformate (IBCF) (1.05-1.25 equiv) was added leading to the precipitation of a white solid. The reaction was allowed to proceed for an additional 5 min, and then benzylamine (1.05-1.2 equiv) was added at -78 °C. The reaction mixture was allowed to stir at room temperature (1-2 h), and then the insoluble salts were filtered. The organic layer was concentrated in vacuo, and the product was purified by column chromatography on SiO₂ gel.

**General procedure for the Preparation *N*-Benzylamide Amino Acids Derivatives Using DMTMM (Method B)** (M. Kunishima et al., Tetrahedron 1999, 55, 13159-13170) To a THF solution of acid ([C] ∼ 0.1M) at room temperature was added benzylamine (1.2 equiv). The solution was stirred for 5-10 min until the benzylammonium carboxylate precipitated. While stirring, DMTMM (1.2 equiv) is added at once, and the resulting suspension is stirred at room temperature (3-12h). In those cases where the salt does not precipitate, the DMTMM was added after 15 min to the solution. The salts were removed by filtration, washed with THF, and the solvent was removed in vacuo. The obtained residue was purified by flash column chromatography to afford the benzylamide, and then recrystallized with ethyl acetate and hexanes.

**(*R*)-*N*-Benzyl-2-acetamido-3-(prop-2-ynyloxy)propanamide ((*R*)-7).** Utilizing Method A, (*R*)-2-acetamido-3-(prop-2-ynyloxy)propanoic acid (1.28 g, 6.92 mmol), NMM (1.14 mL, 10.38 mmol), IBCF (1.14 mL, 8.72 mmol), and benzylamine (0.90 mL, 8.30 mmol) gave crude (*R*)-7. The product was recrystallized (EtOAc) to yield 1.15 g (61%) of (R)-7 as white solid: mp 149 °C; [α]²⁵_{D} -26.8° (c 1.5, CHCl₃); *R_{f}* = 0.45 (1/9 MeOH/CHCl₃); IR (nujol mull) 3263, 2925, 2111, 1639, 1553, 1459 cm⁻¹; ¹H NMR (CDCl₃) δ 2.04 (s, C**H**₃C(O)), 2.45 (t, *J* = 2.4 Hz, CH₂C**CH**), 3.64 (dd, *J* = 7.4, 9.3 Hz, C**H**H'OCH₂), 3.94 (dd, *J* = 3.9, 9.3 Hz, CH**H**'OCH₂), 4.15 (1/2**H**H'_{q}, *J* = 2.4, 15.9 Hz, OC**H**H'C), 4.23 (1/2HH'q, *J* = 2.4, 15.9 Hz, OCH**H**'C), 4.45 (1/2**H**H'_{q}, *J* = 6.0, 15.0 Hz, C**H**H'Ph), 4.51 (1/2H**H**'_{q}, *J* = 6.0, 15.0 Hz, CHH'Ph), 4.57-4.63 (m, CH), 6.43-6.45 (br d, *J* = 6.6 Hz, N**H**CH), 6.65-6.73 (br m, N**H**CH₂), 7.25-7.37 (m, 5 PhH), addition of excess (*R*)-(-)-mandelic acid to a CDCl₃ solution of (*R*)-7 gave only a single signal for the acetyl methyl protons and the alkyne proton, addition of excess (R)-(-)-mandelic acid to a CDCl₃ solution of (*R*)-7 and (*S*)-7 (3:5 ratio) gave two signals for the acetyl methyl protons (*δ* 2.005 (*R*) and 2.020 (S) (Δppm = 0.015)), and two signals for the alkyne proton (*δ* 2.391 (*S*) and 2.432 *(R)* (Δppm = 0.041)); ¹³C NMR (CDCl₃) δ 23.3 (**C**H₃C(O)), 43.8 (**C**H₂Ph), 52.7 (CH), 58.8 (**C**H₂CCH), 69.3 (**C**H₂OCH₂), 75.5 (CH₂C**C**H), 79.0 (CH₂**C**CH), 127.7, 128.9, 138.0 (**C**₆H₅), 169.8, 170.5 (2 **C**(O)), the remaining aromatic signal was not detected; HRMS (ESI) 275.1389 [M + H⁺] (calcd. for C₁₅H₁₉N₂O₃ 275.1396); Anal. (C₁₅H₁₈N₂O₃) Calcd.: C, 65.68%; H, 6.61%; N, 10.21%. Found: C, 65.72%; H, 6.62%; N, 10.06%.

**(*S*)-*N-*Benzyl-2-acetamido-3-(prop-2-ynyloxy)propanamide ((*S*)-7).** Utilizing Method A, (*S*)-2-acetamido-3-(prop-2-ynyloxy)propanoic acid (1.30 g, 7.03 mmol), NMM (1.16 mL, 10.55 mmol), IBCF (1.16 mL, 8.86 mmol), and benzylamine (0.92 mL, 8.44 mmol) gave crude (S)-7. The product was recrystallized (EtOAc) to yield 1.21 g (63%) of (S)-7 as a white solid: mp 148.5-149 °C; [α]²⁵_{D} +27.3° (c 1.7, CHCl₃); *R_{f}* = 0.45 (1/9 MeOH/CHCl₃); IR (nujol mull) 3132, 2924, 2111, 1640, 1552, 1459 cm⁻¹; ¹H NMR (CDCl₃) δ 2.04 (s, C**H**₃C(O)), 2.45 (t, *J* = 2.4 Hz, CH₂C**CH**), 3.64 (dd, *J* = 7.5, 9.1 Hz, C**H**H'OCH₂), 3.94 (dd, *J* = 4.1, 9.1 Hz, CH**H**'OCH₂), 4.15 (1/2**H**H'q, *J* = 2.4, 15.9 Hz, OC**H**H'C), 4.23 (1/2H**H**'_{q}, *J* = 2.4, 15.9 Hz, OCH**H**'C), 4.45 (1/2**H**H'q, *J* = 6.0, 15.0 Hz, C**H**H'Ph), 4.51 (1/2H**H**'q, *J* = 6.0, 15.0 Hz, CH**H**'Ph), 4.57-4.63 (m, CH), 6.43-6.45 (br d, *J* = 6.3 Hz, N**H**CH), 6.65-6.74 (br m, N**H**CH₂), 7.25-7.36 (m, 5 Ph**H**), addition of excess (*R*)-(-)-mandelic acid to a CDCl₃ solution of (*S*)-**7** gave only a single signal for the acetyl methyl protons and the alkyne proton, addition of excess (*R*)-(-)-mandelic acid to a CDCl₃ solution of (*R*)-**7** and (*S*)-**7** (3:5 ratio) gave two signals for the acetyl methyl protons (δ 2.005 (*R)* and 2.020 (*S*) (Δppm = 0.015)), and two signals for the alkyne proton (*δ* 2.391 (S) and 2.432 (R) (Δppm = 0.041)); ¹³C NMR (CDCl₃) *δ* 23.3 (**C**H₃C(O)), 43.7 (**C**H₂Ph), 52.7 (CH), 58.8 (**C**H₂CCH), 69.4 (**C**H₂OCH₂), 75.4 (CH₂C**C**H), 79.1 (CH₂**C**CH), 127.6, 127.7, 128.8, 138.0 (**C**₆H₅), 169.9, 170.6 (2 **C**(O)); HRMS (ESI) 275.1390 [M + H⁺] (calcd. for C₁₅H₁₉N₂O₃ 275.1396); Anal. (C₁₅H₁₈N₂O₃) Calcd.: C, 65.68%; H, 6.61%; N, 10.21%. Found: C, 65.55%; H, 6.61%; N, 10.07%.

**(*R*)-*N*-(4-Aminobenzyl)-2-acetamido-3-(prop-2-ynyloxy)propanamide.** Utilizing Method A, (*R*)-2-acetamido-3-(prop-2-ynyloxy)propanoic acid (0.25 g, 1.37 mmol), NMM (0.23 mL, 2.06 mmol), IBCF (0.23 mL, 1.73 mmol), and 4-aminobenzylamine (0.21 mL, 1.85 mmol) gave crude desired product. The product was purified by column chromatography (SiO₂; 1/9 MeOH/CHCl₃) and then recrystallized (EtOAc) to yield 0.24 g (61%) of desired product as a light yellow solid: mp 97.5-98.5°C; [α]²⁷_{D} +4.2° (*c* 1, MeOH); *R_{f}* = 0.40 (1/9 MeOH/CHCl₃); IR (nujol mull) 3279, 2930, 2111, 1628, 1536, 1459 cm⁻¹; ¹H NMR (CDCl₃) *δ* 2.00 (s, C**H**₃C(O)), 2.44 (t, *J* = 2.4 Hz, CH₂C**CH**), 3.62 (dd, *J* = 6.9, 9.3 Hz, C**H**H'OCH₂), 3.56-3.72 (br m, N**H**₂), 3.88 (dd, *J* = 4.1, 9.3 Hz, CH**H**'OCH₂), 4.12 (1/2**H**H'_{q}, *J =* 2.4, 15.9 Hz, OC**H**H'C), 4.19 (1/2HH'q, *J* = 2.4, 15.9 Hz, OCH**H**'C), 4.32 (d, *J* = 5.7 Hz, C**H**₂Ar), 4.54-4.60 (m, C**H**), 6.55-6.58 (br d, *J =* 7.2 Hz, N**H**CH), 6.60-6.65 (m, 2 ArH), 6.65-6.71 (br m, N**H**CH₂), 7.03-7.06 (m, 2 Ar**H**); ¹³C NMR (CDCl₃) *δ* 23.4 (**C**H₃C(O)), 43.5 (**C**H₂Ph), 52.7 (CH), 58.8 (**C**H₂CCH), 69.4 (**C**H₂OCH₂), 75.5 (CH₂C**C**H), 79.1 (CH₂**C**CH), 115.4 (2**C**_{3'}), 127.7 (C_{1'}), 129.1 (2**C**_{2'}), 146.1 (**C**_{4'}), 169.6, 170.5 (2 **C**(O)); HRMS (ESI) 290.1500 [M + H⁺] (calcd. for C₁₅H₂₀N₃O₃ 290.1505); Anal. (C₁₅H₁₉N₃O₃) Calcd.: C, 62.27%; H, 6.62%; N, 14.52%. Found: C, 62.07%; H, 6.62%; N, 14.30%.

**(*S*)-*N*-(4-Aminobenzyl)-2-acetamido-3-(prop-2-ynyloxy)propanamide.** Utilizing Method A, (*S*)-2-acetamido-3-(prop-2-ynyloxy)propanoic acid (0.25 g, 1.37 mmol), NMM (0.20 mL, 1.78 mmol), IBCF (0.19 mL, 1.44 mmol), and 4-aminobenzylamine (0.16 mL, 1.44 mmol) gave crude desired product. The product was purified by column chromatography (SiO₂; 1/9 MeOH/CHCl₃) and then recrystallized (EtOAc) to yield 0.30 g (77%) of desired product as a light yellow solid: mp 97-98°C; [α]²⁷_{D} -4.1° (*c* 1, MeOH); *R_{f}* = 0.40 (1/9 MeOH/CHCl₃); IR (nujol mull) 3282, 2924, 2112, 1630, 15372, 1458 cm⁻¹; ¹H NMR (CDCl₃) *δ* 2.03 (s, C**H**₃C(O)), 2.45 (t, *J* = 2.4 Hz, CH₂C**CH**), 3.62 (dd, *J* = 7.1, 9.1 Hz, C**H**H'OCH₂), 3.62-3.74 (br m, N**H**₂), 3.92 (dd, *J* = 4.2, 9.1 Hz, CH**H**'OCH₂), 4.14 (1/2**H**H'_{q}, *J* = 2.4, 15.9 Hz, OC**H**H'C), 4.22 (1/2H**H**'_{q}, *J* = 2.4, 15.9 Hz, OCH**H**'C), 4.35 (d, *J* = 6.0, C**H**₂Ar), 4.53-4.59 (m, C**H**), 6.42-6.44 (br d, *J* = 6.3 Hz, N**H**CH), 6.48-6.56 (br m, N**H**CH₂), 6.62-6.67 (m, 2 Ar**H**), 7.03-7.08 (m, 2 Ar**H**); ¹³C NMR (CDCl₃) δ 23.6 (**C**H₃C(O)), 43.8 (**C**H₂Ph), 52.9 (**C**H), 59.0 (**C**H₂CCH), 69.7 (**C**H₂OCH₂), 75.7 (CH₂C**C**H), 79.4 (CH₂**C**CH), 115.6 (2**C**_{3'}), 128.0 (**C**_{1'}), 129.4, (2**C**_{2'}), 146.3 (**C**_{4'}), 169.9, 170.7 (2 **C**(O)); HRMS (ESI) 290.1499 [M + H⁺] (calcd. for C₁₅H₂₀N₃O₃ 290.1505); Anal. (C₁₅H₁₉N₃O₃) Calcd.: C, 62.27%; H, 6.62%; N, 14.52%. Found: C, 62.06%; H, 6.70%; N, 14.30%.

**(*R*)-*N*-(4-Azidobenzyl)-2-acetamido-3-(prop-2-ynyloxy)propanamide ((*R*)-8).** To a cooled (0 °C) CH₃CN solution (20 mL) of (*R*)-*N*-(4-aminobenzyl)-2-acetamido-3-(prop-2-ynyloxy)propanamide (1.04 g, 3.60 mmol) maintained under Ar, *t*-BuONO (1.28 mL, 10.80 mmol) followed by TMSN₃ (1.14 mL, 8.64 mmol) were slowly added. The resulting solution was allowed to stir at room temperature (16 h). The solvent was removed in vacuo, and the product was purified by column chromatography (SiO₂; 1/9 acetone/EtOAc) to give 0.87 g (76%) of (*R*)-8 as a yellowish white solid: mp 135-136 °C; [α]²⁴_{D} -15.7° (c 1.0, CHCl₃); *R_{f} =* 0.40 (1/9 acetone/EtOAc); IR (nujol mull) 3277, 2927, 2110, 1635, 1553, 1459 cm⁻¹; ¹H NMR (CDCl₃) *δ* 2.04 (s, C**H**₃C(O)), 2.46 (t, J = 2.5 Hz, CH₂CC**H**), 3.64 (dd, *J* = 7.1, 9.3 Hz, C**H**H'OCH₂), 3.93 (dd, *J* = 4.2, 9.3 Hz, CH**H**'OCH₂), 4.15 (1/2**H**H'_{q}, *J* = 2.5, 16.0 Hz, OCHH'C), 4.23 (1/2HH'q, *J* = 2.5, 16.0 Hz, OCHH'C), 4.37-4.51 (m, C**H**₂Ar), 4.57-4.63 (m, CH), 6.52 (br d, *J =* 6.3 Hz, NHCH), 6.75-6.80 (br m, N**H**CH₂), 6.96-7.01 (m, 2 Ar**H**), 7.25-7.27 (m, 2 Ar**H**), addition of excess (*R*)-(-)-mandelic acid to a CDCl₃ solution of (*R*)-8 gave only a single signal for the acetyl methyl protons and the alkyne proton, addition of excess (*R*)-(-)-mandelic acid to a CDCl₃ solution of (*R*)-**8** and (*S*)-**8** (1:2 ratio) gave two signals for the acetyl methyl protons (*δ* 2.006 (*R*) and 2.021 (*S*) (Δppm = 0.015)), and two signals for the alkyne proton (*δ* 2.402 (S) and 2.448 *(R)* (Δppm = 0.046)); ¹³C NMR (CDCl₃) *δ* 23.4 (**C**H₃C(O)), 43.2 (**C**H₂Ph), 52.7 (CH), 58.9 (**C**H₂CCH), 69.2 (**C**H₂OCH₂), 75.6 (CH₂C**C**H), 79.0 (CH₂**C**CH), 119.5, 129.2, 134.8, 139.5 (**C**₆H₅), 169.9, 170.6 (2 **C**(O)); HRMS (ESI) 316.1407 [M + H⁺] (calcd. for C₁₅H₁₈N₅O₃ 316.1410); Anal. (C₁₅H₁₇N₅O₃) Calcd.: C, 57.13%; , 5.43%; N, 22.21%. Found: C, 57.30%; H, 5.46%; N, 22.10%.

**(*S*)-*N*-(4-Azidobenzyl)-2-acetamido-3-(prop-2-ynyloxy)propanamide ((*S*)-8).** Using (*S*)-*N*-(4-aminobenzyl)-2-acetamido-3-(prop-2-ynyloxy)propanamide (1.10 g, 3.81 mmol), *t*-BuONO (1.36 mL, 11.42 mmol), TMSN₃ (1.20 mL, 9.14 mmol), and the preceding procedure gave 0.89 g (74%) of (S)-8 as a yellowish white solid: mp 135-136.5 °C; [α]²⁴_{D} +15.4° (c 1.0, CHCl₃); *R_{f}* = 0.40 (1/9 acetone/EtOAc); IR (nujol mull) 3279, 2924, 2111, 1638, 1551, 1458 cm⁻¹; ¹H NMR (CDCl₃) *δ* 2.03 (s, C**H**₃C(O)), 2.46 (t, *J* = 2.4 Hz, CH₂C**CH**), 3.65 (dd, *J* = 7.2, 9.1 Hz, C**H**H'OCH₂), 3.92 (dd, *J* = 4.4, 9.1 Hz, CH**H**'OCH₂), 4.15 (1/2**H**H'_{q}, *J =* 2.4, 15.9 Hz, OC**H**H'C), 4.23 (1/2H**H**'_{q}, *J* = 2.4, 15.9 Hz, OCH**H**'C), 4.36-4.50 (m, C**H**₂Ar), 4.58-4.64 (m, C**H**), 6.48 (br d, *J* = 6.9 Hz, N**H**CH), 6.80-6.84 (br m, N**H**CH₂), 6.96-7.00 (m, 2 Ar**H**), 7.24-7.28 (m, 2 Ar**H**), addition of excess (*R*)-(-)-mandelic acid to a CDCl₃ solution of (*S*)-8 gave only a single signal for the acetyl methyl protons and the alkyne proton, addition of excess (*R*)-(-)-mandelic acid to a CDCl₃ solution of (*R*)-**8** and (*S*)-**8** (1:2 ratio) gave two signals for the acetyl methyl protons (*δ* 2.006 (*R*) and 2.021 (*S*) (Δppm = 0.015)), and two signals for the alkyne proton (*δ* 2.402 (*S*) and 2.448 (R) (Δppm = 0.046)); ¹³C NMR (CDCl₃) *δ* 23.4 (**C**H₃C(O)), 43.2 (**C**H₂Ph), 52.7 (**C**H), 58.9 (**C**H₂CCH), 69.3 (**C**H₂OCH₂), 75.6 (CH₂C**C**H), 79.1 (CH₂**C**CH), 119.5, 129.2, 134.8, 139.5 (**C**₆H₅), 169.9, 170.6 (2 **C**(O)); HRMS (ESI) 316.1406 [M + H⁺] (calcd. for C₁₅H₁₈N₅O₃ 316.1410); Anal. (C₁₅H₁₇N₅O₃) Calcd.: C, 57.13%; H, 5.43%; N, 22.21%. Found: C, 56.99%; H, 5.45%; N, 22.10%.

**(*R*)-*N*-(4-Isothiocyanatobenzyl)-2-acetamido-3-(prop-2-ynyloxy)propanamide ((*R*)-9).** To a dry THF solution (20 mL) of (*R*)-*N*-(4-aminobenzyl)-2-acetamido-3-(prop-2-ynyloxy)propanamide (0.17 g, 0.60 mmol) was added dropwise a solution of DPT (0.21 mL, 0.89 mmol) in THF (8 mL). The reaction solution was stirred under Ar at room temperature (2 h). The solvent was removed in vacuo, and the product was purified by column chromatography (SiO₂; 1/9 acetone/EtOAc) to give 0.15 g (77%) of (*R*)-**9** as a white solid: mp 157-159 °C; [α]²³_{D} -10.9° (c 1.5, CHCl₃); *R_{f} =* 0.40 (1/9 acetone/EtOAc); IR (nujol mull) 3270, 2924, 2084, 1634, 1553, 1459 cm⁻¹; ¹H NMR (CDCl₃) δ 2.03 (s, C**H**₃C(O)), 2.48 (t, *J* = 2.5 Hz, CH₂C**CH**), 3.65 (dd, *J =* 7.1, 9.3 Hz, C**H**H'OCH₂), 3.92 (dd, *J* = 4.2, 9.3 Hz, CH**H**'OCH₂), 4.15 (1/2**H**H'q, *J* = 2.5, 15.9 Hz, OCHH'C), 4.22 (1/2H**H**'q, *J* = 2.5, 15.9 Hz, OCH**H**'C), 4.40 (1/2**H**H'q, *J* = 5.9, 15.5 Hz, C**H**H'Ar), 4.49 (1/2H**H**'_{q}, *J* = 5.9, 15.5 Hz, CH**H**'Ar), 4.60-4.66 (m, C**H**), 6.52 (br d, *J* = 6.9 Hz, N**H**CH), 6.96 (br t, *J* = 5.9 Hz, N**H**CH₂), 7.16-7.19 (m, 2 ArH), 7.24-7.27 (m, 2 Ar**H**), addition of excess (*R*)-(-)-mandelic acid to a CDCl₃ solution of (*R*)-**9** gave only a single signal for the acetyl methyl protons and the alkyne proton, addition of excess (*R*)-(-)-mandelic acid to a CDCl₃ solution of (*R*)-**9** and (*S*)-**9** (1:2 ratio) gave two signals for the acetyl methyl protons (*δ* 2.019 (*R*) and 2.033 (*S*) (Δppm = 0.014)), and two signals for the alkyne proton (*δ* 2.424 (*S*) and 2.462 (*R*) (Δppm = 0.038)); ¹³C NMR (CDCl₃) δ 23.4 (**C**H₃C(O)), 43.1 (**C**H₂Ph), 52.7 (CH), 58.9 (**C**H₂CCH), 69.3 (**C**H₂OCH₂), 75.6 (CH₂C**C**H), 79.0 (CH₂**C**CH), 126.1 (**2C**_{3'}), 128.8 **(2C_{2'}),** 130.6 (**C**_{4'}), 135.7 (NCS), 137.5 (**C**_{1'}), 170.0, 170.6 (2 **C**(O)); HRMS (ESI) 332.1065 [M + H⁺] (calcd. for C₁₆H₁₈N₃O₃S 332.1069); Anal. (C₁₆H₁₇N₃O₃S) Calcd.: C, 57.99%; H, 5.17%; N, 12.68%; S, 9.68%. Found: C, 58.04%; H, 5.18%; N, 12.47%; S, 9.61%.

**(*S*)-*N*-(4-Isothiocyanatobenzyl)-2-acetamido-3-(prop-2-ynyloxy)propanamide ((*S*)-9).** Using the preceding procedure, (*S*)-*N-*(4-aminobenzyl)-2-acetamido-3-(prop-2-ynyloxy)propanamide (0.30 g, 1.02 mmol), and DPT (0.32 g, 1.38 mmol) gave 0.25 g (75%) of pure (*S*)-**9** as a white solid: mp 157-158 °C; [α]²³_{D} +10.6° (c 1.5, CHCl₃); *R_{f}* = 0.40 (1/9 MeOH/CHCl₃); IR (nujol mull) 3272, 2924, 2084, 1633, 1553, 1459 cm⁻¹; ¹H NMR (CDCl₃) *δ* 2.04 (s, C**H**₃C(O)), 2.48 (t, *J* = 2.5 Hz, CH₂C**CH**), 3.65 (dd, *J* = 7.1, 9.2 Hz, C**H**H'OCH₂), 3.94 (dd, *J* = 4.1, 9.2 Hz, CH**H**'OCH₂), 4.16 (1/2**H**H'_{q}, *J* = 2.5, 16.0 Hz, OC**H**H'C), 4.24 (1/2H**H**'_{q}, *J* = 2.5, 16.0 Hz, OCH**H**'C), 4.42 (1/2**H**H'q, *J* = 6.3, 15.5 Hz, C**H**H'Ar), 4.50 (1/2H**H**'q, *J* = 6.3, 15.5 Hz, CH**H**'Ar), 4.57-4.63 (m, CH), 6.41-6.43 (br d, *J* = 6.6 Hz, N**H**CH), 6.81 (br t, *J* = 6.3 Hz, N**H**CH₂), 7.17-7.20 (m, 2 Ar**H**), 7.25-7.27 (m, 2 ArH), addition of excess (*R*)-(-)-mandelic acid to a CDCl₃ solution of (*S*)-**9** gave only a single signal for the acetyl methyl protons and the alkyne proton, addition of excess (R)-(-)-mandelic acid to a CDCl₃ solution of (*R*)-**9** and (*S*)-**9** (1:2 ratio) gave two signals for the acetyl methyl protons (*δ* 2.019 (*R*) and 2.033 (*S*) (Δppm = 0.014)), and two signals for the alkyne proton (*δ* 2.424 (*S*) and 2.462 (R) (Δppm = 0.038)); ¹³C NMR (CDCl₃) *δ* 23.3 (**C**H₃C(O)), 43.1 (**C**H₂Ph), 52.7 (**C**H), 58.8 (**C**H₂CCH), 69.3 (**C**H₂OCH₂), 75.6 (CH₂C**C**H), 79.0 (CH₂**C**CH), 126.1 (2**C**_{3'}), 128.7 (**2C_{2'}**), 130.5 (**C**_{4'}), 135.7 (NCS), 137.5 (**C**_{1'}), 170.0, 170.7 (2 **C**(O)); HRMS (ESI) 332.1063 [M + H⁺] (calcd. for C₁₆H₁₈N₃O₃S 332.1069); Anal. (C₁₆H₁₇N₃O₃S) Calcd.: C, 57.99%; H, 5.17%; N, 12.68%; S, 9.68%. Found: C, 57.77%; H, 5.14%; N, 12.44%; S, 9.55%.

### Reference Examples for Active Compounds of Formula Ia

Our laboratory has reported on the synthesis, characterization, and pharmacological activity of a class of compounds termed "functionalized amino acids" (FAAs, **A).** The protypical FAA is lacosamide (Vimpat®) **(B),** a compound that has recently been approved by the EMEA and FDA in Europe and the US, respectively, for the treatment of partial-onset seizures in adults with epilepsy. Lacosamide has also shown efficacy in phase III human trials for diabetic neuropathy and is under investigation for the treatment of migraines and fibromyalgia.

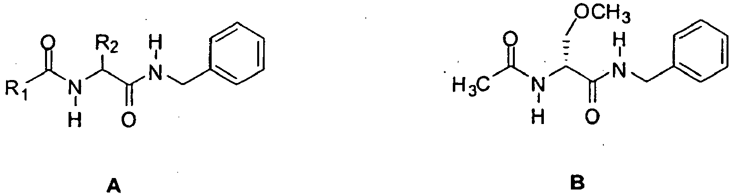

Recently, we have explored the structure-activity relationship (SAR) surrounding B and have discovered that select ***substituted** N*-benzylamide analogs exhibited unexpected and potent neurological activities in animal models for seizures and neuropathic pain. The potent activities of these *N*-benzyl substituted derivatives in animal models for seizures was surprising since similar studies with other FAAs showed that aryl substitution can lead to diminished activity. For example, we observed a sharp loss in seizure protection in mice (ip) in going from C (MES ED₅₀ = 32 mg/kg) to D (MES ED₅₀ -300 mg/kg), where MES is the maximal electroshock seizure assay. A similar decrease in activity was observed upon N-benzylamide substitution of E (MES ED₅₀ = 4.5 mg/kg) to give F (MES ED₅₀ >100, <300 mg/kg).

Our recently discovered compounds contain *atypical* substituents in the *N-*benzylamide unit. In general, an atypical substituent is a substituent that contains multiple groups or a substituent that is not easily characterized by its ability to either donate to or to withdraw electrons from the adjacent group *(e.g.,* alkyl or aryl group). Correspondingly, *typical* substituents such as "electron withdrawing" and "electron donating" groups are well understood by one skilled in the art and are discussed in Advanced Organic Chemistry by J. March, John Wiley and Sons, New York, N.Y., pp 16-18 (1985) and the discussion therein is incorporated herein by reference. Typical electron withdrawing groups usually include halo, including bromo, fluoro, chloro, iodo and the like; nitro, carboxy, lower alkenyl, lower alkynyl, formyl, carboxyamido, aryl, quaternary ammonium, aryl lower alkanoyl, carbalkoxy and the like. Typical electron donating groups usually include such groups as hydroxyl, lower alkoxy, including methoxy, ethoxy and the like; lower alkyl, such as methyl, ethyl, and the like; amino, lower alkylamino, di(loweralkyl) amino, aryloxy such as phenoxy, mercapto, lower alkylthio, lower alkylmercapto, disulfide (lower alkyldithio) and the like.

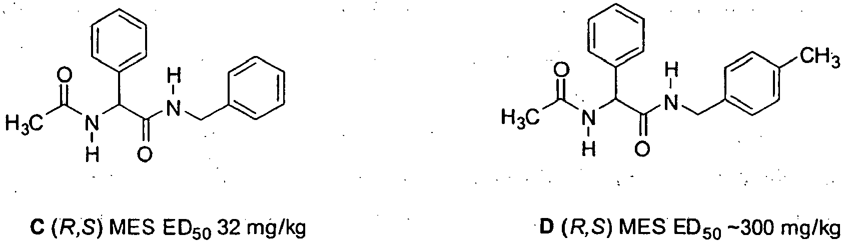

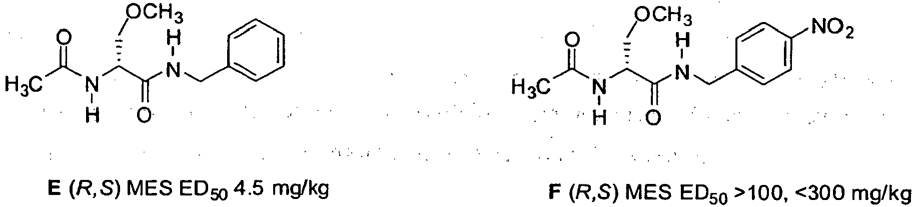

In this disclosure, we report the synthesis and characterization of compounds with atypical substituents attached to the *N*-benzylamido moiety of 2-(acylamido)acetic acid derivatives **(1)** and 2-(acylamido)propionic acid derivatives **(2),** and further disclose that the attachment of these moieties to the *N*-benzylamido substituent of 2-(acylamido)acetic acid derivatives **(1)** and the *N*-benzylamido substituent of 2-(acylamido)propionic acid derivatives **(2)** provides compounds with potent and efficacious activities against neurological disorders in mammals.

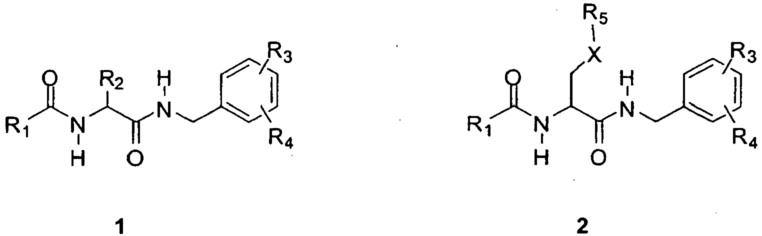

### Results

We have prepared compounds **3-10** and evaluated their activities at the National Institutes of Health (NIH) NINDS Anticonvulsant Screening Program (ASP). All compounds were prepared as the (*R*)-stereoisomer, while for **3** and **5** the corresponding (*S*)-stereoisomer was also prepared. Previous studies have shown that maximal anticonvulsant activity resided in the D-amino acid configuration, which for these compounds corresponds to the (*R*)-configuration. The compounds were prepared by the general synthetic routes outlined in **Schemes 1-7,** and all compounds were fully characterized by established methods. The optical purity of each compound was assessed by a ¹H NMR method and the optical activity recorded.

The pharmacological activities for **3-10** are summarized in **Table 1.** Unlike the dramatic reduction of anticonvulsant activity observed for **D** upon incorporation of a *p-*methyl unit in the *N*-benzylamide group of **C** or the attachment of a *p*-nitro group in E to give **F,** we found that *N*-benzylamide substitution led to compounds with excellent seizure protection in either mice (ip) and/or rats (po) (MES ED₅₀), and that these compounds exhibited minimal neurological toxicity (TD₅₀) in animal behavioral tests (*e.g*., the rotorod test in mice). The high TD₅₀ values compared with the low MES ED₅₀ value provided high Protective Index (PI) values (PI = TD₅₀/ED₅₀) that were comparable with proven antiepileptic agents (*i.e.,* phenytoin, phenobarbital, valproic acid). Similar to previous findings for FAAs **(A),** we observed maximal activity for the (*R*)-stereoisomer.

**Table 1. The Effect of N-Benzylamide Substitution.**

| | | | | | | | | |
|---|---|---|---|---|---|---|---|---|
| 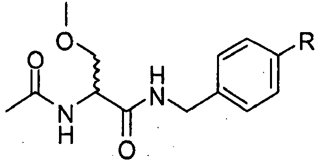 | | | | | | | | |

| | | **Mice(ip)*^{a}*** | | | | **Rat (po)*^{b}*** | | |
|---|---|---|---|---|---|---|---|---|
| | **R** | **MES,*^{d}* ED₅₀** | **6Hz, ED₅₀** | **Tox,*^{e}* TD₅₀** | **PI*^{f}*** | **MES,*^{d}* ED₅₀** | **Tox,*^{e}* TD₅₀** | **PI*^{f}*** |
| *(R)-***B** | (*R*)-**H** | 8.3 [0.5] | | 43 [0.25] | 5.2 | 3.8 [2] | 390 [1] | 100 |
| | | 7.9-9.8 | | (38-47) | | (2.9-5.5) | (320-500) | |
| (*S*)-**B** | (*S*)-**H** | >100, < 300 [0.5] | | > 300 [0.5] | > 1.5 | | | |
| | | | | [30; 100] | | 14 [0.5] | | |
| *(R)*-**3** | *(R)-***OCH₂C₆H₄(3'-F)** | [3; 1.0] [0.5] | 2/4 [0.25] | [0.5] | ∼9.3 | (6.1-27) [0.5] | > 500 [0.5] | > 36 |
| | | [10; 30] [4.0] | | [100; 300] | | < 30 [0.25 to | > 30 [0.25 to 4.0] | |
| | | | | [4.0] | | 4.0] | | |
| (*S*)-**3** | (*S*)-**OCH₂C₆H₄(3'-F)** | > 300 [0.5] | | > 300 [0.5] | | | | |
| (*R*)-**4** | (*R*)-**OCF**₃ | [3; 10] [0.5] | | [10; 30] [0.5] | ∼9.3 | < 30 [0.25 to 4.0] | > 30 [0.25 to 4.0] | |
| (*R*)-**5** | (*R*)-**N**₃ | 8.4 [0.25] | | 46.2 | 5.5 | 3.9 [0.5] | > 250 | > 64 |
| | | (5.7 - 12.2) | | | | (2.5-6.2) | | |
| (*S*)**-5** | (*S*)-**N**₃ | > 100, <300 | Around 100 | > 300 | | > 30 | > 30 | |
| (*R*)-**6** | (*R*)-**C≡CCH₂OMe** | [3; 10] [0.5] | | [10; 30] [0.5] | ∼2.8 | | < 30 [0.5] | > 30 |
| | | | | Death at 100 | | | | |
| (*R*)-**7** | (*R*)-**(CH₂)₃OMe** | [10; 30] [0.5] | | [30; 100] | ∼3.2 | 16 [0.5] | > 500 [0.5 to 24] | > 31.6 |
| | | | | | | (8.8-25) | | |
| (*R*)-**8** | (*R*)-**CH₂OMe** | [30; 100] [0.5] | | [100; 300] [0.5] | ∼3.1 | [>2h] 45 [0.5] (29-76) | > 500 [0.25 to 24]] | >11 |
| (*R*)-**9** | 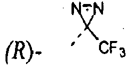 | 15.05 [0.25] (13.93-16.39) | | 68 [0.25] (58-78) | 4.4 | > 30[ 0.25 to 4.0] | > 30 [0.25 to 4.0] | |
| (*R*)-**10** | (R)-**CF**₃ | [30; 100] | | [100; 300] | ∼3.1 | < 30 [0.25 to 4.0] | > 30 [0.25 to 4.0] | |
| (*R*)-**11** | *(R)-***CH₂OC₆H₄(3'-F)** | [3; 10] [0.5] | | [10; 30] [0.5] | ∼2.8 | < 30 [0.25 to 4.0] | > 30 [0.25 to 4.0] | |
| | phenytoin*^{h}* | 9.5 [2] | | 66 [2] | 6.9 | 30 [4] | i | > 100 |
| | | (8.1-10) | | (53 - 72) | | (22-39) | | |
| | Phenobarbital*^{h}* | 22 [1] | | 69 [0.5] | 3.2 | 9.1 [5] | 61 [0.5] | 6.7 |
| | | (15 - 23) | | (63-73) | | (7.6-12) | (44-96) | |
| | valproate*^{h}* | 270 [0.25] | | 430 [0.25] | 1.6 | 490 [0.5] | 280 [0.5] | 0.6 |
| | | (250 - 340) | | (370-450) | | (350-730) | (190 - 350) | |

### Experimental Section

### Scheme 1. Preparation pf (R)-3 and (S)-3.

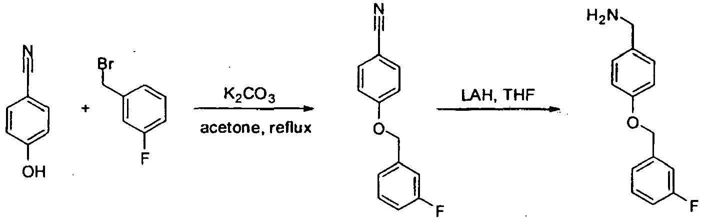

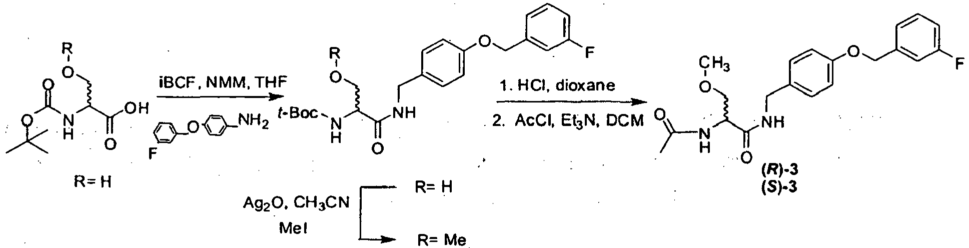

**Preparation of 4-(3-Fluorobenzyloxy)benzonitrile.** (Nakamoto, Kazutaka; Tsukada, Itaru; Tanaka, Keigo; Matsukura, Masayuki; Haneda, Toru; Inoue, Satoshi; Ueda, Norihiro; Abe, Shinya; Hata, Katsura; Watanabe, Naoaki, WO 2005033079 (2005)). A mixture of 4-cyanophenol (11.91 g, 100.0 mmol), K₂CO₃ (55.20 g, 400.0 mmol), and 3-fluorobenzylbromide (22.68 g, 120.0 mmol) were heated in acetone (400 mL) at reflux (5 h). The volatiles were evaporated and the residue was diluted in CH₂Cl₂ (300 mL), and then washed with H₂O (500 mL), dried (MgSO₄), and concentrated in vacuo to give white needles (19.81 g, 87%): *R_{f} =* 0.45 (hexanes/EtOAc 9/1); mp 104-105 °C; ¹H NMR (CDCl₃) *δ* 5.11 (s, C**H**₂O), 6.98-7.20 (m, 5 Ar**H**), 7.33-7.41 (m, 1 Ar**H**), 7.59 (d, *J* = 8.7 Hz, 2 ArH); HRMS (M+Na⁺)(ESI⁺) 250.0641 [M + Na⁺] (calcd for C₁₄H₁₀NONa⁺ 250.0641).

**Preparation of 4-(3-Fluorobenzyloxy)benzylamine.** (Aldrich data.) To a LiAlH₄ (5.02 g, 132.0 mmol) suspension in THF (400 mL) was added dropwise a THF (30 mL) solution of 4-(3-fluorobenzyloxy)benzonitrile (10.00 g, 44.0 mmol) at 0 °C. The mixture was stirred at room temperature (16 h) and H₂O (4 mL) was added dropwise at 0 °C followed by an aqueous NaOH solution (2 mL, 15% w/w), and then H₂O (4 mL). The mixture was stirred at room temperature (2 h), and the precipitate filtered through a Celite^{®} pad and the pad washed with CH₂Cl₂. The filtrate was concentrated in vacuo to obtain a white solid (8.82 g, 86%): *R_{f} =* 0.00 (hexanes/EtOAc 9/1); ¹H NMR (CDCl₃) *δ* 1.61 (br s, N**H**₂), 3.79 (s, C**H**₂NH₂), 5.04 (s, C**H**₂O), 6.90-7.04 (m, 3 Ar**H**) 7.13-7.37 (m, 5 Ar**H**); HRMS (M-NH₂⁺)(ESI⁺) 215.088 [M - NH₂⁺] (calcd for C₁₄H₁₂O⁺ 215.087).

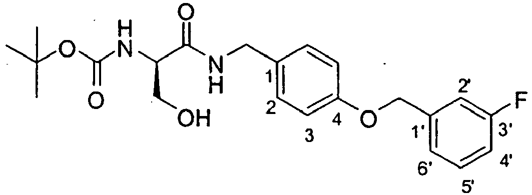

**Preparation of (*R*)-2-*N*-(*tert*.-Butoxycarbonyl)amino-3-hydroxy-*N*-(4-(3-fluorobenzyloxy)benzyl)-propanamide.** A THF solution (75 mL) of (*R*)-*t-*Boc-serine (4.00 g, 19.5 mmol) was stirred and cooled at -78 °C under Ar and then 4-methylmorpholine (NMM) (2.6 mL, 23.4 mmol) was added dropwise. After 2 min of stirring at this temperature, the isobutylchloroformate (IBCF) (3.0 mL, 23.4 mmol) was added dropwise leading to the precipitation of a white solid, and the reaction was allowed to proceed for additional 2 min. 4-(3-Fluorobenzyloxy)benzylamine (5.40 g, 23.4 mmol), was added portionwise at -78 °C. The mixture was allowed to stir at room temperature (2 h), and then the white solid filtered and the organic layer concentrated in vacuo. The residue was purified by flash column chromatography on silica gel with EtOAc/hexanes (50/50 to 80/20) as the eluant to obtain (*R*)-2-*N*-(*tert*.-butoxycarbonyl)amino-3-hydroxy-*N*-(4-(3-fluorobenzyloxy)benzyl)-propanamide as a white solid (5.40 g, 66%): *R_{f}* = 0.33 (hexanes/EtOAc 5/5); mp 88-89 °C; [α]^{25.8}_{D} +25.8° (*c* 1, CHCl₃); IR (nujol) 3319, 2966, 1659, 1527, 1456 1376, 1304, 1242, 1166, 1007, 864, 775, 673 cm⁻¹; ¹H NMR (CDCl₃) *δ*1.41 (s, (C**H**₃)₃), 3.21-3.33 (br m, C**H**H'), 3.59-3.72 (br m, CH**H**'), 4.04-4.20 (br m, C**H**, O**H**), 4.24-4.48 (br m, C**H**₂N), 5.03 (s, C**H**₂O), 5.56-5.66 (br d, N**H**), 6.86-6.92 (m, 2 ArH), 6.94-6.99 (m, 1 Ar**H**, N**H**), 7.10-7.21 (m, 4 Ar**H**), 7.30-7.38 (m, 1 Ar**H**); ¹³C NMR (CDCl₃) *δ* 28.2 ((**C**H₃)₃), 42.8 (**NC**H₂), 55.2 (OCH₂**C**H), 62.8 (O**C**H₂CH), 69.1 (**C**H₂O) 80.5 (**C**(CH₃)₃), 114.1 (d, *J* = 22.0 Hz, **C_{4'}** or **C_{2'}),** 114.6 (d, *J* = 21.0 Hz, **C_{2'}** or **C_{4'}),** 114.9 (**C₁**), 122.6 (d, *J* = 2.9 Hz, **C_{6'}),** 128.8 (ArC), 130.1 (d, *J* = 8.2 Hz, **C_{5'}),** 130.3 (ArC), 139.5 (d, *J* = 7.2 Hz, **C_{1'}),** 156.1 (N**C**(O)O), 157.8 (**C₄**), 162.9 (d, *J* = 244.7 Hz, **C_{3'}),** 171.1 (C(O)); HRMS (M+H⁺)(ESI⁺) 419.1983 [M + H⁺] (calcd for C₂₂H₂₇FN₂O₅H⁺ 419.1982); Anal. Calcd. for C₂₂H₂₇FN₂O₅: C, 63.14; H, 6.50; N, 6.69; F, 4.54. Found: C, 63.12; H, 6.55; N, 6.65; F, 4.38.

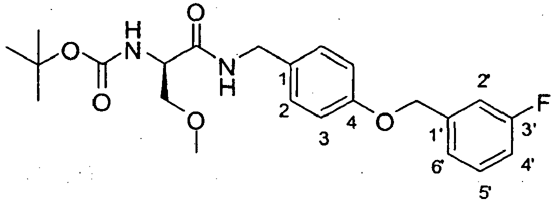

**Preparation of (*R*)-2-*N*-(*tert*.-Butoxycarbonyl)amino-3-methoxy-*N*-(4-(3-fluorobenzyloxy)benzyl)-propanamide.** Ag₂O (13.90 g, 60.0 mmol) was added to a CH₃CN solution (300 mL) of (*R*)-2-*N*-(*tert*.-butoxycarbonyl)amino-3-hydroxy-*N*-(4-(3-fluorobenzyloxy)benzyl)-propanamide (5.00 g, 12.0 mmol) and CH₃I (7.45 mL, 119.6 mmol) at room temperature under Ar. The reaction mixture was stirred at room temperature (3 d), filtered, and the filtrate concentrated in vacuo to obtain a white solid (5.10 g, 98%): *R_{f} =* 0.29 (1/1 EtOAc/hexanes); mp 68-70 °C; [α]^{24.3}_{D} -16.6° (c 1, CHCl₃); IR (nujol) 3308, 2951, 2857, 1648, 1523, 1457, 1376, 1317, 1168, 1088, 1046, 916, 862, 778, 677 cm⁻¹; ¹H NMR (CDCl₃) *δ* 1.42 (s, (C**H**₃)₃), 3.36 (s, OC**H**₃), 3.49 (dd, *J* = 6.3, 9.3 Hz, CHH'), 3.83 (dd, *J* = 3.9, 9.3 Hz, CHH'), 4.18-4.31 (br m, C**H**CH₂), 4.36-4.44 (br m, C**H**₂N), 5.05 (s, OC**H**₂), 5.36-5.48 (br m, OC(O)NH), 6.64-6.72 (br t, CH₂N**H**), 6.91 (d, *J* = 8.1 Hz, 2 Ar**H**), 6.97-7.04 (m, 1 Ar**H**), 7.11-7.24 (m, 4 Ar**H**), 7.30-7.38 (m, 1 ArH); ¹³C NMR (CDCl₃) *δ* 28.2 ((**C**H₃)₃). 42.8 (N**C**H₂), 53.6 (OCH₂**C**H), 59.0 (O**CH**₃), 69.1 (**C**H₂O) 72.0 (O**C**H₂CH), 80.2 (**C**(CH₃)₃), 114.1 (d, *J =* 22.0 Hz, **C_{4'}** or **C_{2'}),** 114.6 (d, *J =* 21.0 Hz, **C_{2'}** or **C_{4'}),** 114.9 (**C₁**), 122.6 (d, *J* = 2.9 Hz, **C_{6'}),** 128.8 (ArC), 130.0 (d, *J* = 8.2 Hz, **C_{5'}),** 130.7 (Ar**C**), 139.5 (d, *J* = 7.3 Hz, **C**_{1'}), 155.0 (N**C**(O)O). 157.8 (**C₄**), 162.9 (d, *J* = 244.8 Hz, **C_{3'}),** 170.1 (C(O)); HRMS (M+H⁺)(ESI⁺) 433.2139 [M + H⁺] (calcd for C₂₃H₂₉FN₂O₅H⁺ 433.2139); Anal. Calcd. for C₂₃H₂₉FN₂O₅: C, 63.87; H, 6.76; N, 6.48; F, 4.39. Found: C, 63.57; H, 6.75; N, 6.39; F, 4.13.

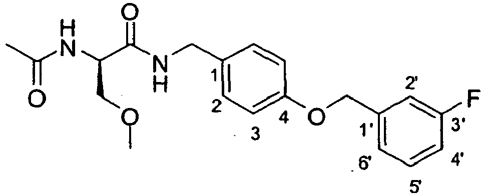

**Preparation of (*R*)-2-Acetamido-*N*-(4-(3-fluorobenzyloxy)benzyl)-3-methoxypropanamide ((*R*)-3).** A saturated HCl solution in dioxane (1 mmol/2 mL, 21.75 mL) was added to (*R*)-2-*N*-(*tert.*-butoxycarbonyl)amino-3-methoxy-*N*-(4-(3-fluorobenzyloxy) benzyl)-propanamide (4.70 g, 10.9 mmol) at 0 °C and the solution was stirred at room temperature (4 h). The reaction solution was concentrated in vacuo and dried (30 min): ¹H NMR (CDCl₃) *δ* 3.23 (s, OC**H**₃), 3.80-4.00 (br m, C**H**₂), 4.14 (d, *J* = 11.0 Hz, C**H**H'), 4.40 (d, *J* = 11.0 Hz, CH**H**'), 4.58-4.35 (m, NC(H)CO), 4.87 (s, OC**H**₂), 6.79 (d, *J* = 8.1 Hz, 2 Ar**H**), 6.95 (t, *J* = 8.4 Hz, 1 ArH), 7.01-7.20 (m, 4 Ar**H**), 7.22-7.30 (m, 1 Ar**H**), 8.11-8.28 (br s, N**H**₃), 8.55-8.61 (br s, NHC(O)); HRMS (M+H⁺)(ESI⁺) 333.1615 [M + H⁺] (calcd for C₁₈H₂₁FN₂O₃H⁺ 333.1614).

The residue was dissolved in CH₂Cl₂ (40 mL) and Et₃N (4.47 mL, 32.6 mmol) and AcCl (1.16 mL, 16.30 mmol) were successively added at 0 °C. The mixture was stirred at room temperature (2 h), aqueous 10% citric acid (60 mL) was added and then the organic layer was separated. The aqueous layer was extracted with CH₂Cl₂ (2 x 30 mL). All of the organic layers were combined, washed with aqueous saturated NaHCO₃ (30 mL) and H₂O (30 mL), dried (MgSO₄), and concentrated in vacuo. The solid was recrystallized with EtOAc to obtain (*R*)-**3** (2.60 g, 65%) as a white solid: *R_{f}* = 0.29 (7/3 hexanes/EtOAc); mp 152 °C; [α]^{24.5}_{D} -18.9° (c 1, CHCl₃); IR (nujol) 3284, 1633, 1552, 1457, 1376, 1305, 1247, 1137, 1050, 978, 722 cm⁻¹; ¹H NMR (CDCl₃) *δ* 2.03 (s, C**H**₃CO), 3.37 (s, OC**H**₃), 3.43 (dd, *J* = 7.2, 9.0 Hz, CHH'), 3.79 (dd, *J* = 3.9, 9.0 Hz, CH**H**'), 4.40 (d, *J* = 5.7 Hz, C**H**₂N), 4.49-4.55 (m, NC(H)CO), 5.05 (s, C**H**₂O), 6.43 (br d, *J* = 7.2 Hz, N**H**C(O)CH₃), 6.64-6.83 (br m, CH₂N**H**), 6.89-7.05 (m, 3 Ar**H**), 7.10-7.22 (m, 4 Ar**H**), 7.31-7.38 (m, 1 Ar**H**); addition of excess (*R*)-(-)-mandelic acid to a CDCl₃ solution of (*R*)-**3** gave only one signal for the acetyl methyl and one signal for the ether methyl protons; ¹³C NMR (CDCl₃) *δ* 23.1 (**C**H₃C(O)), 43.0 (N**C**H₂), 52.4 (OCH₂**C**H), 59.0 (OCH₃), 69.1 (**C**H₂O) 71.7 (O**C**H₂CH), 114.1 (d, *J* = 21.9 Hz, **C_{4'}** or **C_{2'}),** 114.8 (d, *J* = 21.1 Hz, **C_{2'}** or **C_{4'}),** 115.0 (**C₁**), 122.6 (d, J = 2.9 Hz, **C_{6'}**), 128.8 (ArC), 130.1 (d, *J* = 8.2 Hz, **C_{5'}**), 130.5 (ArC), 139.5 (d, *J* = 7.3 Hz, **C**_{1'}), 157.8 (**C₄**), 162.9 (d, *J* = 244.8 Hz, **C_{3'}),** 169.8, 170.3 (2 C(O)); HRMS (M+H⁺)(ESI⁺) 375.1720 [M + H⁺] (calcd for C₂₀H₂₃FN₂O₄H⁺ 375.1720); Anal. Calcd. for C₂₀H₂₃FN₂O₄: C, 64.16; H, 6.19; N, 7.48, F, 5.07. Found: C, 64.14; H, 6.15; N, 7.37; F, 5.05.

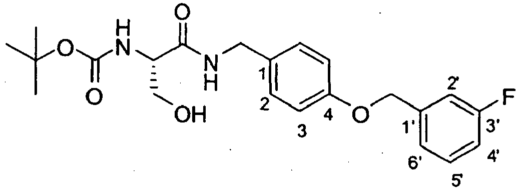

**Preparation of (*S*)-2-*N-*(*tert*.-Butoxycarbonyl)amino-3-hydroxy-*N*-(4-(3-fluorobenzyloxy)benzyl)-propanamide.** A THF solution (75 mL) of (*S*)-*t*-Boc-serine (4.00 g, 19.5 mmol) was stirred and cooled at -78 °C under Ar and then 4-methylmorpholine (NMM) (2.6 mL, 23.4 mmol) was added dropwise. After 2 min of stirring at this temperature, isobutylchloroformate (IBCF) (3.0 mL, 23.4 mmol) was added dropwise leading to the precipitation of a white solid. The reaction was allowed to proceed for additional 2 min and 4-(3-fluorobenzyloxy)benzylamine (5.40 g, 23.4 mmol) was added portionwise at -78 °C. The mixture was allowed to stir at room temperature (2 h), and then the white solid filtered and the organic layer concentrated in vacuo. The residue was purified by flash column chromatography on silica gel with EtOAc/hexanes (50/50 to 80/20) as the eluant to obtain (*S*)-2-*N*-(*tert*.-butoxycarbonyl)amino-3-hydroxy-*N*-(4-(3-fluorobenzyloxy)benzyl)-propanamide as a white solid (5.10 g, 60%): *R_{f}* = 0.33 (hexanes/EtOAc 5/5); mp 88-89 °C; [α]^{25.8}_{D} -24.0° (c 1, CHCl₃); IR (nujol) 3322, 2944, 2858, 1659, 1525, 1457 1375, 1304, 1243, 1166, 1008, 868, 775, 725 cm⁻¹; ¹H NMR (CDCl₃) *δ* 1.41 (s, (C**H**₃)₃), 3.21-3.39 (br m, CHH'), 3.59-3.74 (br m, CH**H'**), 4.04-4.20 (br m, CH, OH), 4.24-4.47 (br m, CH₂N), 5.03 (s, CH₂O), 5.63 (d, *J* = 6.6 Hz, NH), 6.87-6.93 (d, *J* = 9.0 Hz, 2 ArH), 6.94-7.09 (m, 1 ArH, NH), 7.10-7.21 (m, 4 ArH), 7.30-7.38 (m, 1 ArH); ¹³C NMR (CDCl₃) *δ* 28.2 ((**C**H₃)₃), 42.8 (N**C**H₂), 54.9 (OCH₂**C**H), 62.8 (O**C**H₂CH), 69.1 (d, *J* = 1.6 Hz, PhCH₂O), 80.6 (**C**(CH₃)₃), 114.1 (d, *J* = 22.2 Hz, **C**_{4'} or **C_{2'}**), 114.8 (d, *J* = 21.0 Hz, **C_{2'}** or **C_{4'}**), 115.0 (**C₁**), 122.6 (d, *J* = 2.9 Hz, **C_{6'}**), 128.9 (Ar**C**), 130.1 (d, *J* = 8.0 Hz, **C_{5'}**), 130.3 (ArC), 139.5 (d, *J* = 7.4 Hz, **C_{1'}**), 156.2 (N**C**(O)O), 157.9 (**C₄**), 162.9 (d, *J* = 244.7 Hz, **C_{3'}**), 171.2 (C(O)); HRMS (M+H⁺)(ESI⁺) 419.1983 [M + H⁺] (calcd for C₂₂H₂₇FN₂O₅H⁺ 419.1982); Anal. Calcd. for C₂₂H₂₇FN₂O₅: C, 63.14; H, 6.50; N, 6.69; F, 4.54. Found: C, 63.31; H, 6.53; N, 6.77; F, 4.45.

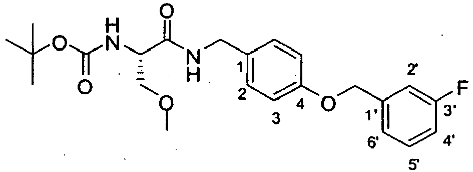

**Preparation of (*S*)-2-*N*-(*tert*.-Butoxycarbonyl)amino-3-methoxy-*N*-(4-(3-fluorobenzyloxy)benzyl)-propanamide.** Ag₂O (13.65 g, 58.6 mmol) was added to a CH₃CN solution (300 mL) of (*S*)-2-*N*-(*tert*.-butoxycarbonyl)amino-3-hydroxy-*N*-(4-(3-fluorobenzyloxy)benzyl)-propanamide (4.90 g, 11.7 mmol) and CH₃I (7.3 mL, 117.0 mmol) at room temperature under Ar. The reaction mixture was stirred at room temperature in the dark (3 d), filtered, and the filtrate concentrated in vacuo to obtain a white solid (4.90 g, 98%): *R_{f}* = 0.29 (1/1 EtOAc/hexanes); mp 70-71 °C; [α]^{24.3}_{D} +16.8° (c 1, CHCl₃); IR (nujol) 3412, 3170, 3120, 1648, 1522, 1457, 1375, 1246, 1167, 1088, 1048, 918, 861, 778, 677 cm⁻¹; ¹H NMR (CDCl₃) *δ* 1.42 (s, (C**H**₃)₃), 3.35 (s, OC**H**₃), 3.49 (dd, *J* = 6.3, 9.3 Hz, CHH'), 3.83 (dd, *J* = 3.9, 9.3 Hz, CHH'), 4.18-4.31 (br m, C**H**CH₂), 4.36-4.44 (br m, CH₂N), 5.05 (s, OC**H**₂), 5.36-5.48 (br m, OC(O)NH), 6.63-6.74 (br t, CH₂N**H**), 6.91 (d, *J* = 8.7 Hz, 2 ArH), 6.97-7.04 (m, 1 ArH), 7.11-7.23 (m, 4 ArH), 7.30-7.38 (m, 1 ArH); ¹³C NMR (CDCl₃) *δ* 28.2 ((**C**H₃)₃), 42.8 (N**C**H₂), 54.0 (OCH₂**C**H), 59.0 (O**C**H₃), 69.1 (d, *J =* 1.7 Hz, Ph**C**H₂O), 72.0 (O**C**H₂CH), 80.2 (**C**(CH₃)₃), 114.1 (d, *J =* 21.7 Hz, **C_{4'}** or **C₂'**), 114.7 (d, *J* = 21.1 Hz, **C_{2'}** or **C_{4'}**), 114.9 (**C₁**), 122.6 (d, *J* = 2.9 Hz, **C_{6'}**), 128.8 (ArC), 130.1 (d, *J* = 8.5 Hz, **C_{5'}**), 130.6 (ArC), 139.5 (d, *J=* 6.8 Hz, **C_{1'}**), 155.5 (N**C**(O)O), 157.8 (**C₄**), 162.9 (d, *J* = 244.7 Hz, **C_{3'}**), 170.1 (C(O)); HRMS (M+H⁺)(ESI⁺) 433.2139 [M + H⁺] (calcd for C₂₃H₂₉FN₂O₅H⁺ 433.2139); Anal. Calcd. for C₂₃H₂₉FN₂O₅: C, 63.87; H, 6.76; N, 6.48; F, 4.39. Found: C, 63.75; H, 6.82; N, 6.51; F, 4.22.

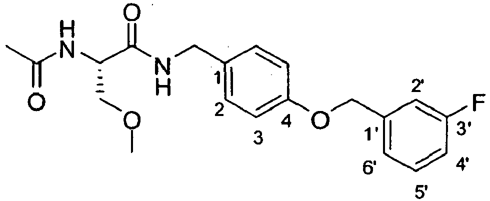

**Preparation of (S)-2-Acetamido-N-(4-(3-fluorobenzyloxy)benzyl)-3-methoxypropanamide ((*S*)-3)**. A saturated HCl solution in dioxane (1 mmol/2 mL, 20.8 mL) was added to (*S*)-2-*N*-(*tert.*-butoxycarbonyl)amino-3-methoxy-*N*-(4-(3-fluorobenzyloxy) benzyl)-propanamide (4.50 g, 10.4 mmol) at 0 °C and the solution was stirred at room temperature (4 h). The reaction solution was concentrated in vacuo and dried (30 min): ¹H NMR (DMSO-*d*₆) *δ* 3.29 (s, OC**H**₃), 3.71 (d, *J* = 4.8 Hz, C**H₂**), 3.94-4.06 (br m, CH), 4.27 (d, *J* = 5.4 Hz, NC**H**₂), 5.13 (s, OC**H**₂), 6.97 (d, *J* = 8.1 Hz, 2 ArH), 7.10-7.32 (m, 5 ArH), 7.40-7.47 (m, 1 ArH), 8.31-8.42 (br s, N**H₃**), 9.05-9.13 (br t, NHC(O)).

The residue was dissolved in CH₂Cl₂ (40 mL) and Et₃N (4.3 mL, 31.2 mmol) and AcCl (1.1 mL, 15.6 mmol) were successively added at 0 °C. The mixture was stirred at room temperature (2 h), aqueous 10% citric acid (60 mL) was added and then the organic layer was separated. The aqueous layer was extracted with CH₂Cl₂ (2 x 30 mL). All of the organic layers were combined, washed with aqueous saturated NaHCO₃ (30 mL) and H₂O (30 mL), dried (MgSO₄), and concentrated in vacuo. The solid was recrystallized with EtOAc to obtain (S)-3 (3.10 g, 80%) as a white solid: *R_{f}* = 0.29 (7/3 hexanes/EtOAc); mp 149-150 °C; [α]^{24.5}_{D} +18.8° (c 1, CHCl₃); IR (nujol) 3281, 2946, 2890, 1634, 1553, 1457, 1376, 1304, 1246, 1135, 1050, 953, 720 cm⁻¹; ¹H NMR (CDCl₃) *δ* 2.02 (s, C**H₃**CO), 3.36 (s, OC**H**₃), 3.43 (dd, *J* = 7.5, 9.1 Hz, CHH'), 3.79 (dd, *J* = 4.2, 9.1 Hz, CH**H'**), 4.40 (d, *J* = 5.7 Hz, CH₂N), 4.50-4.55 (m, NC(H)CO), 5.05 (s, C**H₂**O), 6.47 (br d, *J* = 6.0 Hz, N**H**C(O)CH₃), 6.70-6.79 (br m, CH₂N**H**), 6.90-7.05 (m, 3 ArH), 7.10-7.22 (m, 4 ArH), 7.31-7.38 (m, 1 ArH); addition of excess (*R*)-(-)-mandelic acid to a CDCl₃ solution of (S)-3 gave only one signal for the acetyl methyl and one signal for the ether methyl protons; ¹³C NMR (CDCl₃) *δ* 23.1 (**C**H₃C(O)), 42.9 (N**C**H₂), 52.4 (OCH₂**C**H), 59.0 (O**C**H₃), 69.1 (d, *J* = 2.3 Hz, CH₂O), 71.8 (O**C**H₂CH), 114.1 (d, *J* = 22.2 Hz, **C_{4'}** or **C_{2'}**), 114.8 (d, *J =* 21.1 Hz, **C_{2'}** or **C_{4'}**), 114.9 (**C₁**), 122.6 (d, J = 2.9 Hz, **C_{6'}**), 128.8 (ArC), 130.1 (d, *J =* 8.0 Hz, **C_{5'}**), 130.5 (ArC), 139.5 (d, *J* = 6.9 Hz, **C_{1'}**), 157.8 (**C₄**), 162.9 (d, *J* = 244.7 Hz, **C_{3'}**), 169.8, 170.3 (2 **C**(O)); HRMS (M+H⁺)(ESI⁺) 375.1720 [M + H⁺] (calcd for C₂₀H₂₃FN₂O₄H⁺ 375.1720); Anal. Calcd. for C₂₀H₂₃FN₂O₄: C, 64.16; H, 6.19; N, 7.48, F, 5.07. Found: C, 64.33; H, 6.23; N, 7.50; F, 5.01.

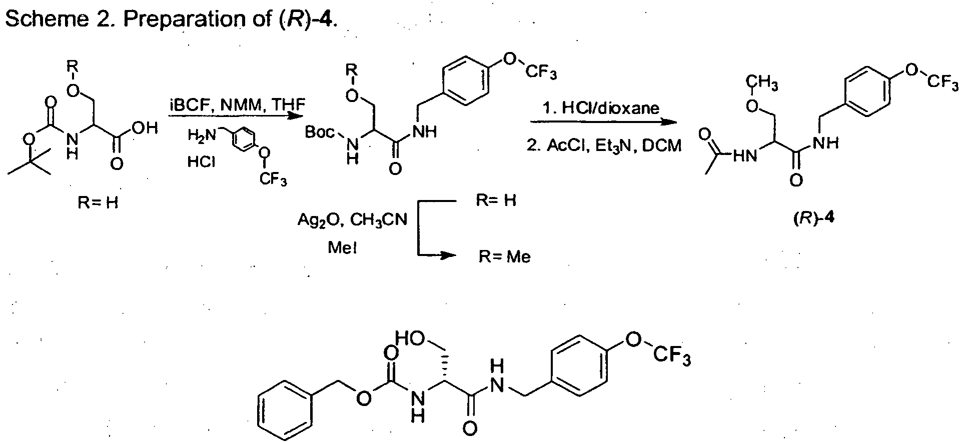

**Preparation of (R)-2-N-(Benzyloxycarbonyl)amino-3-hydroxy-*N*-(4-trifluoromethoxybenzyl)-propanamide.** A THF solution (200 mL) of (R)-Cbz-serine (6.00 g, 25.1 mmol) was stirred and cooled at -78 °C under Ar and then 4-methylmorpholine (NMM) (3.3 mL, 30.1 mmol) was added dropwise. After 2 min of stirring at this temperature, isobutylchloroformate (IBCF) (3.9 mL, 30.1 mmol) was added dropwise leading to the precipitation of a white solid, and the reaction was allowed to proceed for additional 2 min and then 4-trifluoromethoxybenzylamine (4.6 mL, 30.1 mmol), was added portionwise at -78 °C. The mixture was allowed to stir at room temperature (2 h), and then the white solid filtered and the organic layer concentrated in vacuo. The residue was recrystallized in EtOAc to obtain *(R)-2-N-*(benzyloxycarbonyl)amino-3-hydroxy-*N*-(4-trifluoromethoxybenzyl)-propanamide as a white solid (6.10 g, 59%): *R_{f}* = 0.30 (hexanes/EtOAc 5/5); mp 189-190 °C; [α]^{27.4}_{D} -6.5° (c 1, DMSO); IR (nujol) 3278, 3143, 2926, 2866, 1691, 1645, 1539, 1457, 1374, 1278, 1224, 1157, 1025, 963 739, 690 cm⁻¹; ¹H NMR (DMSO-*d*₆) *δ* 3.56 (m, CHH', CH**H'**), 4.05-4.12 (br dd, CH), 4.31 (d, *J* = 5.7 Hz, CH₂N), 4.91 (t, *J* = 5.5 Hz, OH), 5.04 (s, C**H₂**O), 7.25-7.38 (m, NH, 9 ArH), 8.50 (t, *J* = 5.7 Hz, CH₂N**H**); ¹³C NMR (DMSO-*d₆*) *δ* 41.3 (N**C**H₂), 57.3 (OCH₂**C**H), 61.7 (O**C**H₂CH), 65.5 (Ph**C**H₂O), 120.0 (q, *J* = 254.4 Hz, O**C**F₃), 120.7, 127.6, 127.7, 128.2, 128.7, 136.9, 138.9, (7 ArC), 147.0 (q, *J* = 1.7 Hz, **C**OCF₃), 155.9 (N**C**(O)O), 170.3 (C(O)); HRMS (M+H⁺)(ESI⁺) 413.1325 [M + H⁺] (calcd for C₁₉H₁₉F₃N₂O₅H⁺ 413.1324); Anal. Calcd. for C₁₉H₁₉F₃N₂O₅: C, 55.34; H, 4.64; F, 13.82; N, 6.79. Found: C, 55.06; H, 4.61; F, 13.70; N, 6.74.

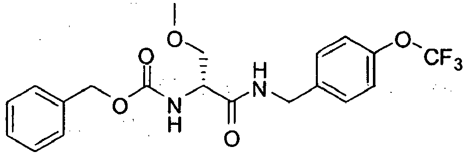

*Preparation of* **(*R*)-2-*N*-(Benzyloxycarbonyl)amino-3-methoxy-*N*-(4-trifluoromethylbenzyl)-propanamide.** Ag₂O (15.56 g, 66.7 mmol) was added to a CH₃CN solution (250 mL) of (*R*)-2-*N*-(benzyloxycarbonyl)amino-3-hydroxy-*N*-(4-trifluoromethoxybenzyl)-propanamide (5.50 g, 13.4 mmol) and CH₃I (8.3 mL, 134.0 mmol) at room temperature under Ar. The reaction mixture was stirred at room temperature in the dark (3 d), filtered, and the filtrate concentrated in vacuo. The solid was purified by flash column chromatography on silica gel with EtOAc/hexanes (50/50) as the eluant to obtain (*R*)-2-*N*-(benzyloxycarbonyl)amino-3-methoxy-*N*-(4-trifluoromethylbenzyl)-propanamide as a white solid (4.40 g, 97%): *R_{f}* = 0.77 (EtOAc/hexanes 5/5); mp 114-115 °C; [α]^{24.5}_{D} -21.4° (c 1, CHCl₃); IR (nujol) 3279, 3089, 2958, 2858, 1638, 1553, 1456, 1377, 1285, 1221, 1148, 988, 918, 841, 725, 610 cm⁻¹; ¹H NMR (CDCl₃) *δ* 3.36 (s, OC**H₃**), 3.49 (dd, *J* = 6.3, 9.0 Hz, CHH'), 3.86 (dd, *J* = 3.9, 9.0 Hz, CH**H'**), 4.29-4.40 (br m, C**H**CH₂), 4.46 (d, *J* = 6.3 Hz, CH₂N), 5.12 (s, OC**H**₂), 5.53-5.64 (br s, NH), 6.74-6.84 (br m, NH), 7.15 (d, *J* = 8.4 Hz, 2 ArH), 7.24-7.39 (m, 7 ArH); ¹³C NMR (CDCl₃) *δ* 42.7 (N**C**H₂), 54.4 (OCH₂**C**H), 59.1 (O**C**H₃), 67.3 (O**C**H₂), 71.9 (O**C**H₂CH), 120.4 (q, *J* = 255.5 Hz, O**C**F₃), 121.2, 128.2, 128.3, 128.6, 128.7, 135.9, 136.7 (7 ArC), 148.5 (**C**OCF₃), 156.1 (N**C**(O)O), 170.0 (**C**(O)); HRMS (M+H⁺)(ESI⁺) 427.1481 [M + H⁺] (calcd for C₂₀H₂₁F₃N₂O₅H⁺ 427.1481); Anal. Calcd. for C₂₀H₂₁F₃N₂O₅: C, 56.34; H, 4.96; F, 13.28; N, 6.57. Found: C, 56.34; H, 4.97; F, 13.28; N, 6.63.

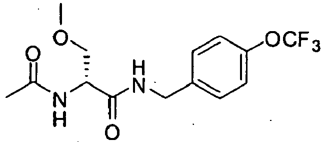

**Preparation of (*R*)-2-Acetamido-3-methoxy-*N*-(4-trifluoromethylbenzyl)-propanamide ((*R*)-4).** An EtOH solution (400 mL) of (*R*)-2-*N*-(benzyloxycarbonyl)amino-3-methoxy-*N*-(4-trifluoromethylbenzyl)-propanamide ((R)-25) (3.90 g, 9.2 mmol) was treated with H₂ (1 atm) in presence of 10% Pd/C (390 mg) at room temperature (16 h). The mixture was carefully filtered through a bed of Celite^{®} and the filtrate was evaporated in vacuo to obtain a brown oil: ¹H NMR (CDCl₃) *δ* 1.44-1.95 (br s, N**H₂**), 3.38 (s, OC**H**₃), 3.50-3.67 (br m, C**H₂**, CH), 4.46 (d, J = 5.7 Hz, NC**H₂**), 7.17 (d, *J* = 8.0 Hz, 2 ArH), 7.31 (d, *J* = 8.0 Hz, 2 Ar**H**), 7.80-8.00 (br s, N**H**C(O)).

The oil was dissolved in CH₂Cl₂ (100 mL) and then triethylamine (1.5 mL, 11.0 mmol) and acetyl chloride (0.78 mL, 11.0 mmol) were carefully added at 0 °C and the resulting solution was stirred at room temperature (2 h). An aqueous 10% citric acid solution (100 mL) was added and the organic layer was extracted with CH₂Cl₂ (3 x 100 mL). The organic layers were combined, washed with a saturated NaHCO₃ solution (100 mL), dried (MgSO₄), and concentrated in vacuo. The residue was recrystallized in EtOAc to obtain (*R*)-4 as a white solid (2.50 g, 83%): *R_{f}* = 0.49 (EtOAc); mp 134-135 °C; [α]^{24.9}_{D} -17.6° (c 0.5 , CHCl₃); IR (nujol) 3279, 3088, 2958, 2858, 1638, 1553, 1456, 1377, 1285, 1221, 1148, 988, 918, 841, 725 cm⁻¹; ¹H NMR (CDCl₃) *δ* 2.04 (s, C**H₃**CO), 3.39 (s, OC**H**₃), 3.44 (dd, *J* = 7.5, 9.0 Hz, CHH'), 3.82 (dd, *J* = 4.2, 9.0 Hz, CH**H'**), 4.44-4.52 (m, CH₂N), 4.52-4.59 (m, CH), 6.41 (br d, *J* = 6.6 Hz, N**H**C(O)CH₃), 6.78-6.89 (br t, CH₂N**H**), 7.18 (d, *J* = 8.1 Hz, 2 ArH), 7.29 (d, *J* = 8.1 Hz, 2 ArH), addition of excess (*R*)-(-)-mandelic acid to a CDCl₃ solution of (*R*)-**4** gave only one signal for the acetyl methyl and one signal for the ether methyl protons; ¹³C NMR (CDCl₃) 23.1 (**C**H₃CO), 42.7 (**C**H₂N), 52.5 (**C**HCH₂), 59.1 (O**C**H₃), 71.7 (**C**H₂OCH₃), 120.4 (q, *J* = 255.5 Hz, **C**F₃), 121.2, 128.7, 136.7, (3 ArC), 148.4 (d, *J=* 1.7 Hz, **C**OCF₃), 170.1, 170.4 (2 **C**(O)); HRMS (M+H⁺)(ESI⁺) 335.1219 [M + H⁺] (calcd for C₁₄H₁₇F₃N₂O₄H⁺ 335.1218); Anal. Calcd. for C₁₄H₁₇F₃N₂O₄: C, 50.30; H, 5.13; F, 17.05; N, 8.38. Found: C, 50.45; H, 5.13; F, 17.18; N, 8.39.

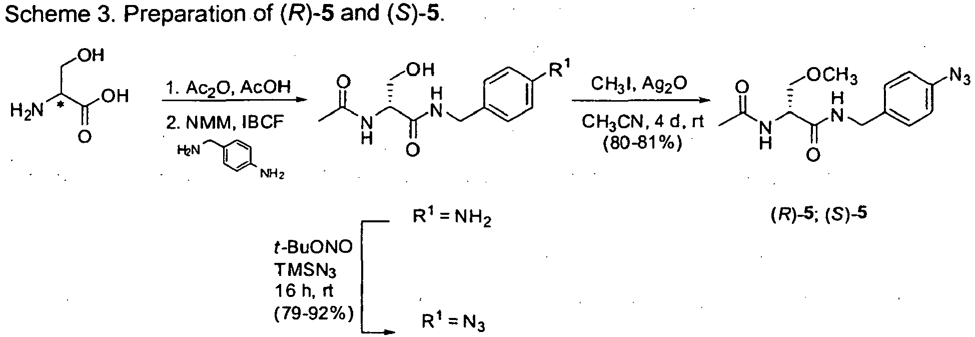

**General Procedure for the Preparation *N*-Benzylamide Amino Acids Derivatives Using Mixed-Anhydride Coupling (MAC) (Method A).** A dry THF solution of the carboxylic acid (-0.2-1.0 M) was cooled to -78 °C under Ar, and 4-methylmorpholine (NMM) (1.3-1.5 equiv) was added. After stirring (2 min), isobutyl chloroformate (IBCF) (1.05-1.25 equiv) was added leading to the precipitation of a white solid. The reaction was allowed to proceed for an additional 5 min, and then benzylamine (1.05-1.2 equiv) was added at -78 °C. The reaction mixture was allowed to stir at room temperature (1-2 h), and then the insoluble salts were filtered. The organic layer was concentrated in vacuo, and the product was purified by column chromatography on SiO₂ gel.

**General Procedure for the Preparation 3-Methoxy-2-amidopropionamide Derivatives (Method B).** To a CH₃CN solution of the alcohol (0.05-0.1 M) was successively added Ag₂O (5 equiv) and Mel (10 equiv) at room temperature. The reaction mixture was maintained at room temperature (2-4 d), filtered, and the solvent was evaporated in vacuo. The residue was purified by column chromatography on SiO₂.

**(*R*)-*N*-(4-Aminobenzyl)-2-acetamido-3-hydroxypropanamide.** To a stirred AcOH (80 mL) suspension of D-serine (10.00 g, 95.24 mmol) was added Ac₂O (9.44 mL, 100.00 mmol), and then the reaction suspension was stirred at room temperature (24 h). The AcOH was removed in vacuo to give an oily residue, and then THF (600 mL) was added to the residue. Utilizing Method A, NMM (15.71 mL, 142.86 mmol), IBCF (15.71 mL, 120.09 mmol), and 4-aminobenzylamine (12.93 mL, 114.29 mmol) gave 3.35 g (14%) of (*R*)-*N*-(4-aminobenzyl)-2-acetamido-3-hydroxypropanamide after purification by column chromatography (SiO₂; 1/7 MeOH/CHCl₃) as a white solid: mp 158-160 °C; [α]²⁶_{D} +18.3° (c 1.0, MeOH); *R_{f}* = 0.30 (1/7 MeOH/CHCl₃); IR (nujol mull) 3302, 2924, 2359, 1630, 1551, 1458 cm⁻¹; ¹H NMR (DMSO-*d*₆) *δ* 1.86 (s, C**H₃**C(O)), 3.55 (t, *J* = 5.6 Hz, C**H₂**OH), 4.06 (1/2**H**H'_{q}, *J* = 5.9, 14.7 Hz, C**H**H'Ar), 4.12 (1/2H**H'**_{q}, *J* = 5.9, 14.7 Hz, CH**H'**Ar), 4.24-4.30 (m, CH), 4.85 (t, *J* = 5.6 Hz, OH), 4.94 (s, N**H₂**), 6.46-6.50 (m, 2 ArH), 6.88-6.91 (m, 2 ArH), 7.88 (d, *J* = 7.8 Hz, N**H**CH), 8.14 (t, *J* = 5.9 Hz, N**H**CH₂); ¹³C NMR (DMSO-*d*₆) *δ* 22.7 (**C**H₃C(O)), 41.8 (**C**H₂Ph), 55.2 (CH), 61.8 (**C**H₂OH), 113.6, 126.1, 128.0, 147.4 (**C₆**H₅), 169.3, 169.8 (2 **C**(O)); HRMS (ESI) 252.1344 [M + H⁺] (calcd. for C₁₂H₁₈N₃O₃ 252.1348); Anal. (C₁₂H₁₇N₃O₃) Calcd.: C, 57.36%; H, 6.82%; N, 16.72%. Found: C, 57.13%; H, 6.87%; N, 16.55%.

**(*S*)-*N*-(4-Aminobenzyl)-2-acetamido-3-hydroxypropanamide.** Using L-serine (10.00 g, 95.24 mmol), Ac₂O (9.44 mL, 100.00 mmol), and the preceding procedure gave 3.30 g (14%) of (*S*)-*N*-(4-aminobenzyl)-2-acetamido-3-hydroxypropanamide as a white solid: mp 158.5-160 °C; [α]²⁶_{D} -18.7° (c 1.0, MeOH); *R_{f}* = 0.30 (1/7 MeOH/CHCl₃); IR (nujol mull) 3280, 2923, 1628, 1551, 1458 cm⁻¹; ¹H NMR (DMSO-*d*₆) *δ* 1.86 (s, C**H₃**C(O)), 3.55 (t, *J* = 5.6 Hz, C**H₂**OH), 4.06 (1/2**H**H'_{q}, *J* = 5.9, 14.7 Hz, C**H**H'Ar), 4.12 (1/2H**H'**_{q}, *J* = 5.9, 14.7 Hz, CH**H'**Ar), 4.24-4.30 (m, CH), 4.86 (t, *J* = 5.6 Hz, OH), 4.94 (s, N**H₂**), 6.46-6.51 (m, 2 ArH), 6.88-6.91 (m, 2 ArH), 7.88 (d, *J* = 7.8 Hz, NHCH), 8.14 (t, *J* = 5.9 Hz, N**H**CH₂); ¹³C NMR (DMSO-*d*₆) *δ* 22.7 (**C**H₃C(O)), 41.8 (**C**H₂Ph), 55.2 (CH), 61.8 (**C**H₂OH), 113.7, 126.1, 128.1, 147.4 (**C₆**H₅), 169.3, 169.9 (2 **C**(O)); HRMS (ESI) 252.1344 [M + H⁺] (calcd. for C₁₂H₁₈N₃O₃ 252.1348); Anal. (C₁₂H₁₇N₃O₃) Calcd.: C, 57.36%; H, 6.82%; N, 16.72%. Found: C, 57.12%; H, 6.84%; N, 16.43%.

**(*R*)-*N*-(4-Azidobenzyl)-2-acetamido-3-hydroxypropanamide.** To a cooled (0 °C) CH₃CN solution (70 mL) of (*R*)-*N*-(4-aminobenzyl)-2-acetamido-3-hydroxypropanamide (2.40 g, 9.56 mmol) maintained under Ar, *t*-BuONO (3.41 mL, 28.68 mmol) followed by TMSN₃ (3.02 mL, 22.94 mmol) were slowly added. The resulting solution was allowed to stir at room temperature (16 h) under Ar. The solvent was removed in vacuo, and the product was purified by column chromatography (SiO₂; 1/9 MeOH/CHCl₃) to give 2.10 g (79%) of (*R*)-*N*-(4-azidobenzyl)-2-acetamido-3-hydroxypropanamide as a white solid: mp 161-163 °C; [α]²⁶_{D} +13.2° (c 1.0, MeOH); *R_{f}* = 0.35 (1/9 MeOH/CHCl₃); IR (nujol mull) 3268, 2924, 2128, 1649, 1553, 1459 cm⁻¹; ¹H NMR (DMSO-*d*₆) *δ* 1.87 (s, C**H₃**C(O)), 3.58 (t, *J* = 5.7 Hz, C**H₂**OH), 4.26-4.32 (m, C**H₂**Ar and CH), 4.91 (t, *J* = 5.7 Hz, OH), 7.03-7.08 (m, 2 ArH), 7.28-7.31 (m, 2 ArH), 7.94 (d, *J* = 8.1 Hz, NHCH), 8.40 (t, *J* = 6.0 Hz, N**H**CH₂); ¹³C NMR (DMSO-*d*₆) δ 22.7 (**C**H₃C(O)), 41.5 (**C**H₂Ph), 55.3 (CH), 61.7 (**C**H₂OH), 118.9, 128.7, 136.5, 137.7 (**C₆**H₅), 169.4, 170.3 (2 **C**(O)); HRMS (ESI) 278.1249 [M + H⁺] (calcd. for C₁₂H₁₆N₅O₃ 278.1253); Anal. (C₁₂H₁₅N₅O₃) Calcd.: C, 51.98%; H, 5.45%; N, 25.26%. Found: C, 51.93%; H, 5.47%; N, 24.98%.

**(*S*)-*N*-(4-Azidobenzyl)-2-acetamido-3-hydroxypropanamide.** Using (S)-N-(4-aminobenzyl)-2-acetamido-3-hydroxypropanamide (2.80 g, 11.16 mmol), t-BuONO (3.98 mL, 33.48 mmol), TMSN₃ (3.52 mL, 26.78 mmol), and the preceding procedure gave 2.85 g (92%) of (*S*)-*N*-(4-azidobenzyl)-2-acetamido-3-hydroxypropanamide as a white solid: mp 161-162 °C; [α]²⁶_{D} -13.6° (c 1.0, MeOH); *R_{f}* = 0.35 (1/9 MeOH/CHCl₃); IR (nujol mull) 3267, 2924, 2129, 1648, 1552, 1459 cm⁻¹; ¹H NMR (DMSO-*d*₆) *δ* 1.87 (s, C**H₃**C(O)), 3.58 (t, *J* = 5.6 Hz, C**H₂**OH), 4.26-4.31 (m, C**H₂**Ar and CH), 4.91 (t, *J =* 5.6 Hz, OH), 7.03-7.08 (m, 2 ArH), 7.27-7.32 (m, 2 ArH), 7,94 (d, *J* = 7.8 Hz, NHCH), 8.40 (t, *J* = 6.0 Hz, N**H**CH₂); ¹³C NMR (DMSO-*d*₆) δ 22.6 (**C**H₃C(O)), 41.5 (**C**H₂Ph), 55.3 (CH), 61.7 (**C**H₂OH), 118.9, 128.7, 136.5, 137.7 (**C₆**H₅), 169.4, 170:3 (2 **C**(O)); HRMS (ESI) 278.1248 [M + H⁺] (calcd. for C₁₂H₁₆N₅O₃ 278.1253); Anal. (C₁₂H₁₅N₅O₃) Calcd.: C, 51.98%; H, 5.45%; N, 25.26%. Found: C, 52.08%; H, 5.51%; N, 25.00%.

**(*R*)-*N*-(4-Azidobenzyl)-2-acetamido-3-methoxypropanamide ((*R*)-5).** Utilizing Method B, (*R*)-*N*-(4-azidobenzyl)-2-acetamido-3-hydroxypropanamide (1.16 g, 4.19 mmol), Ag₂O (4.85 g, 20.94 mmol), and MeI (2.61 mL, 41.89 mmol) gave crude (*R*)-5 after 4 d. The product was purified by column chromatography (SiO₂; 1/9 MeOH/CHCl₃) to obtain 0.98 g (80%) of (R)-5 as a white solid: mp 149-150 °C; [α]²⁶_{D} -15.2° (c 1.0, MeOH); *R_{f}* = 0.5 (1/9 MeOH/CHCl₃); IR (nujol mull) 3285, 2931, 2113, 1635, 1560, 1456 cm⁻¹; ¹H NMR (CDCl₃) δ 2.03 (s, C**H₃**C(O)), 3.38 (s, OC**H**₃), 3.44 (dd, *J* = 7.5, 9.3 Hz, C**H**H'OCH₃), 3.80 (dd, *J* = 4.2, 9.3 Hz, CH**H'**OCH₃), 4.40 (1/2**H**H'_{q}, *J* = 6.2, 15.0 Hz, C**H**H'Ar), 4.46 (1/2H**H'**_{q}, *J* = 6.2, 15.0 Hz, CH**H'**Ar), 4.52-4.58 (m, CH), 6.48 (br d, *J* = 6.3 Hz, NHCH), 6.82-6.85 (br m, N**H**CH₂), 6.96-7.01 (m, 2 ArH), 7.23-7.28 (m, 2 ArH), addition of excess (R)-(-)-mandelic acid to a CDCl₃ solution of (R)-5 gave only a single signal for the acetyl methyl protons and the ether methyl protons, addition of excess (R)-(-)-mandelic acid to a CDCl₃ solution of (R)-**5** and (S)-**5** (1:2 ratio) gave two signals for the acetyl methyl protons (*δ* 1.995 (R) and 2.010 (S) (Δppm = 0.015)), and two signals for the ether methyl protons (*δ* 3.302 (S) and 3.342 (*R*) (Δppm = 0.040)); ¹³C NMR (CDCl₃) *δ* 23.4 (**C**H₃C(O)), 43.1 (**C**H₂Ph), 52.7 (CH), 59.3 (CH₂O**C**H₃), 71.8 (**C**H₂OCH₃), 119.5, 129.1, 134.9, 139.5 (**C₆**H₅), 170.2, 170.5 (2 **C**(O)); HRMS (ESI) 292.1406 [M + H⁺] (calcd. for C₁₃H₁₈N₅O₃ 292.1410); Anal. (C₁₃H₁₇N₅O₃) Calcd.: C, 53.60%; H, 5.88%; N, 24.04%. Found: C, 53.72%; H, 5.91%; N, 23.84%.

**(*S*)-*N*-(4-Azidobenzyl)-2-acetamido-3-methoxypropanamide ((*S*)-5).** Utilizing Method B, (*S*)-*N*-(4-Azidobenzyl)-2-acetamido-3-hydroxypropanamide (2.40 g, 8.66 mmol), Ag₂O (10.04 g, 43.30 mmol), and MeI (5.40 mL, 86.60 mmol) gave crude (S)-**5** after 4 d. The product was purified by column chromatography (SiO₂; 1/9 MeOH/CHCl₃) to obtain 2.05 g (81%) of (S)-5 as a white solid: mp 149-150 °C; [α]²⁶_{D} +15.4° (c 1.0, MeOH); *R_{f}* = 0.5 (1/9 MeOH/CHCl₃); IR (nujol mull) 3285, 2927, 2112, 1635, 1565, 1457 cm⁻¹; ¹H NMR (CDCl₃) *δ* 2.03 (s, C**H₃**C(O)), 3.38 (s, OC**H**₃), 3.43 (dd, *J* = 7.5, 9.0 Hz, C**H**H'OCH₃), 3.81 (dd, *J* = 4.2, 9.0 Hz, CH**H'**OCH₃), 4.40 (1/2**H**H'_{q}, *J* = 6.0, 15.0 Hz, C**H**H'Ar), 4.46 (1/2H**H'**_{q}, *J* = 6.0, 15.0 Hz, CH**H'**Ar), 4.51-4.57 (m, C**H**), 6.43 (br d, *J* = 6.3 Hz, NHCH), 6.78-6.83 (br m, N**H**CH₂), 6.96-7.01 (m, 2 ArH), 7.23-7.27 (m, 2 Ar**H**), addition of excess (*R*)-(-)-mandelic acid to a CDCl₃ solution of (*R*)-**5** gave only a single signal for the acetyl methyl protons and the ether methyl protons, addition of excess (*R*)-(-)-mandelic acid to a CDCl₃ solution of *(R)****-*5** and (*S*)-**5** (1:2 ratio) gave two signals for the acetyl methyl protons (*δ* 1.995 (*R*) and 2.010 (S) (Δppm = 0.015)), and the ether methyl protons (*δ* 3.302 (S) and 3.342 (R) (Δppm = 0.040)); ¹³C NMR (CDCl₃) *δ* 23.3 (**C**H₃C(O)), 43.1 (**C**H₂Ph), 52.7 (CH), 59.2 (CH₂O**C**H₃), 72.0 (**C**H₂OCH₃), 119.4, 129.1, 135.0, 139.4 (**C₆**H₅), 170.3, 170.6 (2 **C**(O)); HRMS (ESI) 292.1405 [M + H⁺] (calcd. for C₁₃H₁₈N₅O₃ 292.1410); Anal. (C₁₃H₁₇N₅O₃) Calcd.: C, 53.60%; H, 5.88%; N, 24.04%. Found: C, 53.76%; H, 5.97%; N, 24.22%.

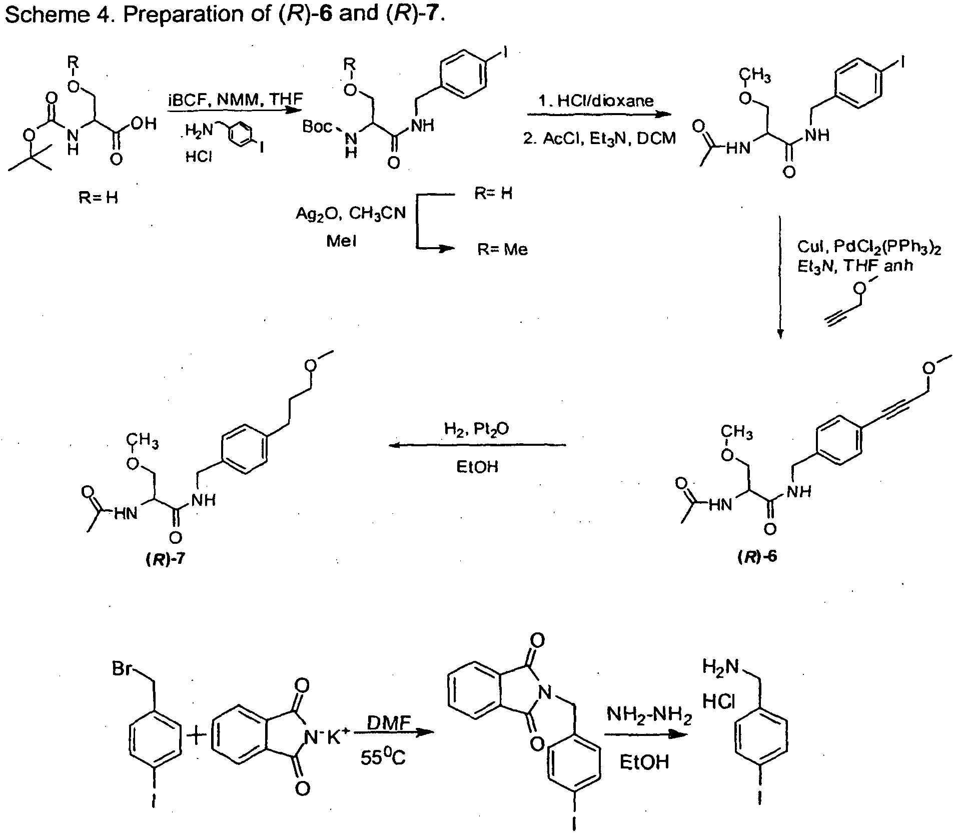

**Preparation of *N*-(4-Iodobenzyl)phthalimide.** (Brown, Stephen; Grigg, Ronald; Hinsley, Joanne; Korn, Stewart; Sridharan, Visuvanathar; Uttley, Michael; Tetrahedron (2001), 57(52), 10347-10355.) A mixture of 4-iodobenzylchloride (40.0 g, 134.7 mmol), potassium phthalimide (26.20 g, 141.5 mmol) and dry DMF (150 mL) was heated overnight at 55 °C under Ar, and then the solvent was removed at reduced pressure. The solid residue was triturated with CHCl₃ (200 mL), filtered, and washed with CHCl₃ (3 x 200 mL). The combined organic extracts were successively washed with aqueous 0.2 M NaOH (200 mL) and H₂O (400 mL), and then dried (MgSO₄). The solvent was removed at reduced pressure to afford a crude solid, which was triturated with Et₂O to obtain a white solid (37.00 g, 75%): *R_{f}* = 0.75 (1/1 EtOAc/hexanes); mp 138-139 °C; ¹H NMR (CDCl₃) □4.77 (s, C**H₂**), 7.17 (d, *J* = 8.6 Hz, 2 ArH), 7.63 (d, *J* = 8.6 Hz, 2 ArH), 7.78 (dd, *J* = 3.0, 5.7 Hz, 2 PhtH), 7.83 (dd, *J =* 3.0, 5.7 Hz, 2 PhtH); ¹³C NMR (CDCl₃) *δ* 41.0 (**C**H₂), 93.5 (**C**l), 123.4, 130.6, 131.9, 134.1, 135.9, 137.7 (6 **Ar)***,* 167.2 (2 **C**(O)); HRMS (ESI) 363.9831 [M+H⁺] (calcd for C₁₇H₁₃NO₂H⁺ 363.9835).

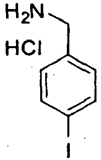

**Preparation of 4-Iodobenzylamine Hydrochloride.** An EtOH solution (50 mL) of hydrazine hydrate (7.28 mL, 152.9 mmol) was added to an EtOH solution (800 mL) of *N-*(4-iodobenzyl)phthalimide (37.00 g, 101.9 mmol) maintained at reflux under Ar. The solution was stirred at reflux (2.5 h), and then the solvent was removed at reduced pressure. The solid residue was dissolved in CHCl₃ (200 mL) and treated with aqueous 20% NaOH (200 mL). The aqueous phase was separated, extracted with CHCl₃ (3 x 300 mL), and the combined organic layers were dried (MgSO₄) and concentrated in vacuo to afford the free base as an oil.

The free base was converted to the corresponding hydrochloride salt by addition of a 4 M HCl solution in dioxane. The white precipitate was filtered and dried to obtain 25.00 g of 4-iodobenzylamine hydrochloride (91%): *R_{f}* = 0.1 (EtOAc); mp > 250 °C (lit. Aldrich data) mp 299-303 °C); ¹H NMR (DMSO-*d*₆) *δ* 3.97 (d, *J* = 5.4 Hz, C**H₂**), 7.34 (d, *J* = 8.1 Hz, 2 ArH), 7.76 (d, *J* = 8.1 Hz, 2 ArH), 8.50-8.85 (br s, NH₃); HRMS (ESI) 233.9780 [M+H⁺] (calcd for C₁₇H₁₃NO₂H⁺ 233.9775).

**Preparation of (*R*)-2-*N*-(*tert*.-Butoxycarbonyl)amino-3-hydroxy-*N*-(4-iodobenzyl)propanamide.** A THF solution (140 mL) of (*R*)-*tert*.-Boc-serine (1.72 g, 8.42 mmol) was stirred and cooled at -78 °C under Ar and then 4-methylmorpholine (NMM) (1.1 mL, 10.10 mmol) was added dropwise. The reaction was stirred at this temperature (2 min) and then isobutylchloroformate (IBCF) (1.3 mL, 10.10 mmol) was added dropwise leading to the precipitation of a white solid. The reaction was allowed to proceed for additional 2 min. A heterogeneous THF (10 mL) mixture of 4-iodobenzylamine hydrochloride (2.5 g, 9.26 mmol) and NMM (1.1 mL, 10.10 mmol) was added portionwise at -78 °C. The mixture was allowed to stir at room temperature (2 h) and the white solid was filtered and the organic layer was concentrated in vacuum. The solid was recrystallized in EtOAc to obtain (*R*)-2-*N*-(*tert.*-butoxycarbonyl)amino-3-hydroxy-*N*-(4-iodobenzyl)propanamide (2.51 g, 71%) as a white solid: *R_{f}* = 0.60 (EtOAc); mp 129-130 °C; [α]²⁴_{D} +0.97° (c 2.8, DMSO); IR (nujol) 3327, 1656, 1521, 1458, 1375, 1302, 1244, 1164, 1009 cm⁻¹; ¹H NMR (DMSO-*d*₆) *δ* 1.39 (s, (C**H₃**)₃C), 3.49-3.60 (br m, CHH'OH and CH**H'**OH), 3.95-4.01 (br m, C**H**CH₂), 4.18-4.31 (m, CH₂N), 4.86 (br s, OH), 6.68 (d, *J* = 7.8 Hz, *tert*.-BocNH), 7.08 (d, *J* = 8.1 Hz, 2 PhH), 7.64 (d, *J* = 8.1 Hz, 2 PhH), 8.37 (br s, CH₂N**H**); ¹³C NMR (CDCl₃) *δ* 28.2 ((**C**H₃)₃C), 42.8 (N**C**H₂), 54.7 (OCH₂**C**H), 62.7 (O**C**H₂CH), 80.8 ((CH₃)₃**C**), 92.8 (**C**l), 129.3, 137.5, 137.7, (**C**₆H₄), 156.4 (C(O)), 171.5 (C(O)); HRMS (M+Na⁺)(ESI⁺) 443.0435 [M + Na⁺] (calcd for C₁₅H₂₁IN₂O₄Na⁺ 443.0444); Anal. Calcd. for C₁₅H₂₁IN₂O₄: C, 42.87; H, 5.04; N, 6.67; I, 30.20. Found: C, 43.13; H, 5.14; N, 6.71; I, 29.96.

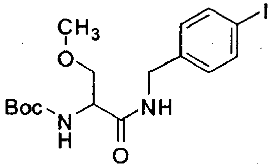

**Preparation of (*R*)-2-*N*-(*tert*.-Butoxycarbonyl)amino-3-methoxy-*N*-(4-iodobenzyl)propanamide.** Ag₂O (23.40 g, 101.20 mmol) was added to a CH₃CN solution (300 mL) of (*R*)-2-*N*-(*tert*.-butoxycarbonyl)amino-3-hydroxy-*N*-(4-iodobenzyl)propanamide (8.50 g, 20.24 mmol) and then CH₃I (12.60 mL, 202.4 mmol) was added at room temperature under Ar. The reaction mixture was stirred at room temperature (3 d), filtered, and the filtrate concentrated in vacuo. The residue was purified by column chromatography (SiO₂; 2/3 EtOAc/hexanes) to obtain 7.56 g (85%) of a white solid: *R_{f}* = 0.53 (1/1 EtOAc/hexanes); mp 86-87 °C; [α]²³_{D} -3.4° (c 1, DMSO); IR (nujol) 3334, 1659, 1528, 1461, 1376, 1303, 1245, 1165, 1110, 1049, 954, 870, 788, 619 cm⁻¹; ¹H NMR (CDCl₃) *δ* 1.43 (s, (C**H₃**)₃C), 3.37 (s, OC**H₃**) 3.48 (dd, *J* = 6.3, 9.3 Hz, C**H**H'OH), 3.84 (dd, *J* = 3.9, 9.3 Hz, CH**H'**OH), 4.20-4.28 (br m, C**H**CH₂), 4.41 (d, *J* = 5.4 Hz, CH₂N), 5.39 (br s, *tert*.-BocNH), 6.75-6.80 (br t, CH₂N**H**), 7.01 (d, *J* = 8.2 Hz, 2 PhH), 7.64 (d, *J* = 8.2 Hz, 2 PhH); ¹³C NMR (CDCl₃) □ 28.2 ((**C**H₃)₃C), 42.8 (N**C**H₂), 54.0 (OCH₂**C**H), 59.1 (O**C**H₃), 71.9 (O**C**H₂CH), 80.5 ((CH₃)₃**C**), 92.7 (**Cl**), 129.3, 137.7, 137.8, (**C**₆H₄), 155.5 (C(O)), 170.4 (C(O)); HRMS (M+H⁺)(ESI⁺) 435.0777 [M + H⁺] (calcd for C₁₆H₂₃IN₂O₄H⁺ 435.0781); Anal. Calcd. for C₁₆H₂₃IN₂O₄: C, 44.25; H, 5.34; N, 6.45; I, 29.22. Found: C, 44.51; H, 5.34; N, 6.41; I, 28.99.

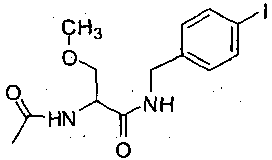

**Preparation of *(R)*-2-Acetamido-3-methoxy-*N*-(4-iodobenzyl)propanamide.** A saturated HCl solution in dioxane (1 mmol/2 mL, 17 mL) was added to *(S)*-2-*N*-(*tert.-*butoxycarbonyl)amino-3-methoxy-*N*-(4-iodobenzyl)propanamide (*(S)*-**4**) (3.70 g, 8.52 mmol) at 0 °C and the solution was stirred at room temperature (2 h). The reaction solution was concentrated in vacuo and dried (30 min). CH₂Cl₂ (30 mL) was added to the residue followed by the successive additions of Et₃N (3.6 mL, 25.60 mmol) and AcCl (906 µL, 12.30 mmol) at 0 °C. The mixture was stirred at room temperature (2 h), aqueous 10% citric acid was added and then the organic layer was separated. The aqueous layer was extracted with CH₂Cl₂ (2 x 30 mL). The organic layers were combined, washed with aqueous saturated NaHCO₃ (30 ml) and H₂O (30 ml), dried (MgSO₄), and concentrated in vacuo. The solid was recrystallized with EtOAc to obtain *(R)*-2-acetamido-3-methoxy-*N-*(4-iodobenzyl)propanamide (2.43 g, 76%) as a white solid: *R_{f}* = 0.76 (5/5 acetone/EtOAc); mp 159-160 °C; [α]²⁵_{D} = +3.3° (c 1, DMSO); IR (nujol) 3279, 1636, 1552, 1457, 1375, 1305, 1139, 725 cm⁻¹; ¹H NMR (CDCl₃) *δ* 2.02 (s, C**H₃**CO), 3.38 (s, OC**H**₃), 3.44 (dd, *J =* 7.2, 9.0 Hz, CHH'), 3.79 (dd, *J* = 4.2, 9.0 Hz, CHH'), 4.38-4.41 (m, CH₂N), 4.52-4.59 (m, NC(H)CO), 6.46 (br d, *J* = 6.6 Hz, N**H**C(O)CH₃), 6.85-6.93 (br t, CH₂N**H**), 7.00 (d, *J* = 8.4 Hz, 2 PhH), 7.64 (d, *J* = 8.4 Hz, 2 Ph**H**); ¹³ C NMR (CDCl₃) 23.1 (**C**H₃CO), 42.9 (**C**H₂N), 52.4 (**C**HCH₂), 59.1 (O**C**H₃), 71.6 (**C**H₂OCH₃), 92.7 (**C**l), 129.3, 137.7, 139.1 (**C**₆H₄), 170.1, 170.3 (2 **C**(O)); HRMS (M+Na⁺)(ESI⁺) 399.0177 [M + Na⁺] (calcd for C₁₃H₁₇IN₂O₃Na⁺ 399.0182); Anal. Calcd. for C₁₃H₁₇IN₂O₃: C, 41.51; H, 4.55; N, 7.45; I, 33.73. Found: C, 41.70; H, 4.49; N, 7.39; I, 33.69.

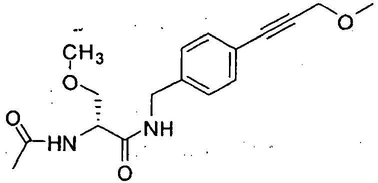

**Preparation of (*R*)-2-Acetamido-3-methoxy-*N*-(4-(3-methoxyprop-1-ynyl)benzyl)propanamide ((*R*)-6).** To an anhydrous THF (10 mL) solution of *(R)*-2-acetamido-3-methoxy-*N*-(4-iodobenzyl)propanamide (376 mg, 1.0 mmol), triethylamine (280 µL, 2.0 mmol), 3-methoxyprop-1-yne (125 µl, 1.5 mmol), dichlorobis(triphenylphosphine)-palladium (II) (70 mg, 0.1 mmol), and CuI (38 mg, 0.2 mmol) were sequentially added under Ar. The mixture was stirred at room temperature (4 h), and then Et₂O (10 mL) added and the precipitate filtered through a Celite pad. The filtrate was concentrated in vacuo and the residue was purified by flash chromatography on silica gel with EtOAc/MeOH (9/1) as the eluant to obtain (R)-6 (260 mg, 82%) as a beige solid: *R_{f}* = 0.27 (EtOAc); mp 141-142 °C; [α]²⁷_{D} = +4.4° (c 1, DMSO); IR (nujol) 3278, 3096, 1640, 1554, 1458, 1370, 1304, 1257, 1192, 1099, 966, 903, 810, 732 cm⁻¹; ¹H NMR (CDCl₃) *δ* 2.02 (s, C**H₃**CO), 3.37 (s, OC**H**₃), 3.45-3.47 (m, C**H**H' and OC**H**₃), 3.78 (dd, *J* = 4.2, 9.0 Hz, CHH'), 4.32 (s, C≡CC**H**₂OCH₃) 4.38-4.52 (m, CH₂N), 4.54-4.61 (m, NC(H)CO), 6.52 (d, *J* = 6.6 Hz, CHNH), 6.91-6.99 (br t, CH₂N**H**), 7.19 (d, *J* = 7.9 Hz, 2 ArH), 7.41 (d, *J* = 7.9 Hz, 2 ArH); ¹³C NMR (CDCl₃) *δ* 23.2 (**C**H₃CO), 43.2 (**C**H₂N), 52.5 (**C**HCH₂), 57.7 (C≡CCH₂O**C**H₃), 59.1 (O**C**H₃), 60.4 (C≡C**C**H₂OCH₃), 71.7 (**C**H₂OCH₃), 85.2 (C=C), 86.0 (C≡C), 121.8, 127.3, 132.1, 138.4 (**C**₆H₄), 170.4 (2 **C**(O)); HRMS (M+H⁺)(ESI⁺) 319.1652 [M + H⁺] (calcd for C₁₇H₂₂N₂O₄H⁺ 319.1658); Anal. Calcd. for C₁₇H₂₂N₂O₄ 0.33 H₂O: C, 62.95; H, 7.04; N, 8.64. Found: C, 62.98; H, 6.78; N, 8.47.

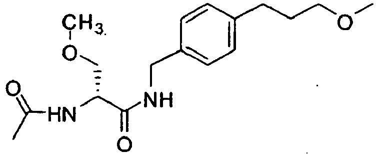

**Preparation of (*R*)-2-Acetamido-3-methoxy-*N*-(4-(3-methoxypropyl)benzyl)-propanamide ((*R*)-7).** An EtOH solution (30 mL) of (*R*)-2-acetamido-3-methoxy-*N*-(4-(3-methoxyprop-1-ynyl)benzyl)propanamide ((*R*)-6) (1.00 g, 3.1 mmol) was treated with H₂ (1 atm) in the presence of 10% PtO₂ (50 mg) at room temperature (16 h). The mixture was carefully filtered through a bed of Celite^{®}. The filtrate was concentrated in vacuo and the residue was purified by flash chromatography on silica gel with EtOAc as the eluant to obtain (R)-7 (510 mg, 51%) as a white solid: *R_{f}* = 0.27 (EtOAc); mp 105-107 °C; [α]²⁵_{D} +3.0° (c 0.5 , DMSO); IR (nujol) 3283, 3085, 1638, 1550, 1457, 1379, 1299, 1122, 979, 725, 605 cm⁻¹; ¹H NMR (CDCl₃) *δ* 1.81-1.92 (m, C**H₂**), 2.03 (s, C**H₃**CO), 2.67 (t, *J* = 7.8 Hz, C**H₂**Ph), 3.33-3.46 (m, CHH'O, C**H₂**O and 2 OC**H**₃), 3.80 (dd, *J* = 4.0, 9.1 Hz, CH**H'**O), 4.44 (d, *J* = 5.7 Hz, CH₂N), 4.50-4.57 (m, C**H**), 6.45 (br d, *J* = 6.6 Hz, N**H**C(O)CH₃), 6.70-6.75 (br t, CH₂N**H**), 7.15-7.20 (m, 4 Ar**H**); ¹³C NMR (CDCl₃) 23.2 (**C**H₃CO), 31.2, 31.9 (2 **C**H₂), 43.3 (**C**H₂N), 52.4 (**C**HCH₂), 58.6, 59.1 (2 OC**H**₃), 71.7, 71.9 (2 **C**H₂OMe), 127.5, 128.8, 135.3, 141.4 (**C**₆H₄), 169.9, 170.2 (2 **C**(O)); HRMS (M+Na⁺)(ESI⁺) 317.1784 [M + Na⁺] (calcd for C₁₇H₂₆N₂O₄Na⁺ 345.1790); Anal. Calcd. for C₁₇H₂₆N₂O₄: C , 63.33; H, 8.13; N, 8.69. Found: C, 63.12; H, 8.13; N, 8.64.

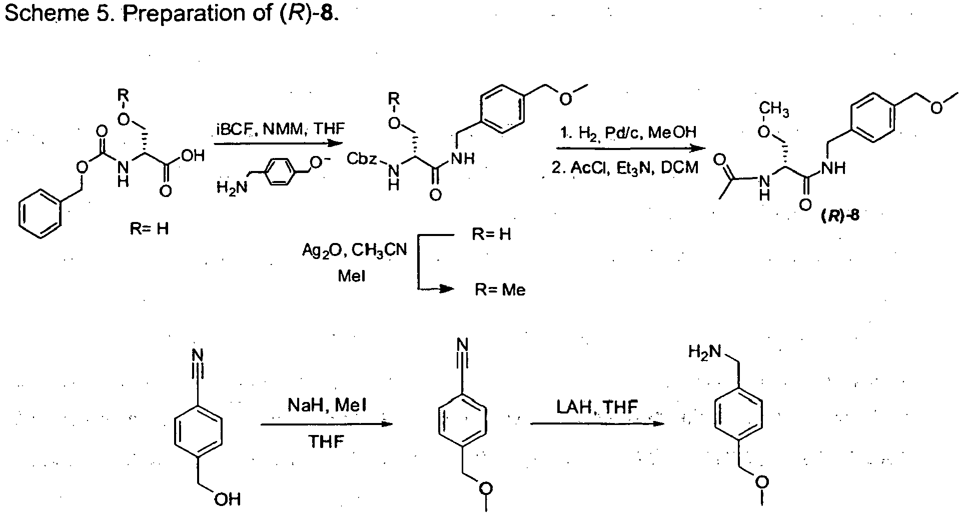

**Preparation of 4-(Methoxymethyl)benzonitrile.** (Sebastien Fortin, Emmanuel Moreau, Alexandre Patenaude, Michel Desjardins, Jacques Lacroix, Jean L. C. Rousseau and Rene' C-Gaudreault, Bioorganic & Medicinal Chemistry 15 (2007) 1430-1438.) A THF solution (250 mL) of 4-(hydroxymethyl)benzonitrile (10.00 g, 75.0 mmol) was added dropwise at 0 °C to a NaH (60% in mineral oil suspension, 11.50 g, 300.0 mmol) suspension in THF (600 mL). The mixture was stirred (10 min) and Mel (11.7 mL, 187.5 mmol) was added dropwise. The mixture was stirred at room temperature (3 h) and then a saturated aqueous NH₄Cl solution (100 mL) was added. The reaction mixture was extrated with CH₂Cl₂ (3 x 250 mL). The organic layers were combined, dried (Na₂SO₄). and concentrated in vacuo. The residue was distilled in vacuo (125 °C, 5 torr) to obtain a colorless oil (10.50 g, 95%): *R_{f}* = 0.59 (hexanes/EtOAc9/1); ¹H NMR (CDCl₃) □ 3.43 (s, OCH₃), 4.51 (s, CH₂O), 7.44 (d, *J =* 8.3 Hz, 2 ArH), 7.63 (d, J = 8.3 Hz, 2 ArH); ¹³C NMR (CDCl₃) 58.4 (CH₃O), 73.5 (**C**H₂OCH₃), 111.1 (CCN), 118.7 (CN), 127.6, 132.0, 143.8 (3 ArC); HRMS (M- CH₃⁺)(ESI⁺) 132.0443 [M - CH₃⁺] (calcd for C₈H₆NO⁺ 132.0443).

**Preparation of 4-(Methoxymethyl)benzylamine.** (Braun, Julius; Zobel, Friedrich. Berichte der Deutschen Chemischen Gesellschaft (1923), 56B: 690-6.) To a LiAlH₄ (7.36 g, 193.7 mmol) suspension in THF (400 mL) was added dropwise a THF (30 mL) solution of 4-(methoxymethyl)benzonitrile (9.50 g, 64.6 mmol) at 0 °C. The mixture was stirred at room temperature (16 h) and then H₂O (6 mL) was added dropwise at 0 °C followed by an aqueous NaOH solution (3 mL, 15% w/w) and then H₂O (6 mL). The mixture was stirred at room temperature (2 h) and the precipitate was filtered through Celite^{®}, and the pad was washed with CH₂Cl₂. The filtrate was concentrated in vacuo to give 8.20 g of a colorless oil (84%): *R_{f}* = 0.00 (hexanes/EtOAc 9/1); ¹H NMR (CDCl₃) *δ* 1.41 (br s, NH₂), 3.88 (s, OCH₃), 3.86 (s, CH₂NH₂), 4.47 (s, CH₂O), 7.20-7.40 (br m, 4 ArH); ¹³C NMR (CDCl₃) 46.2 (**C**H₂NH₂), 58.0 (CH₃O), 74.4 (**C**H₂OCH₃), 127.1, 128.0, 136.7, 142.8 (4 ArC); HRMS (M+H⁺)(ESI⁺) 152.1073 [M + H⁺] (calcd for C₉H₁₃NO⁺ 152.1075).

**Preparation of (*R*)-2-*N*-(Benzyloxycarbonyl)amino-3-hydroxy-*N*-(4-methoxymethylbenzyl)propanamide.** A THF solution (75 mL) of (*R*)-Cbz-serine (5.30 g, 22.0 mmol) was stirred and cooled at -78 °C under Ar and then 4-methylmorpholine (NMM) (2.9 mL, 26.4 mmol) was added dropwise. After 2 min of stirring at this temperature, isobutylchloroformate (IBCF) (3.5 mL, 26.4 mmol) was added dropwise leading to the precipitation of a white solid, and the reaction was allowed to proceed for additional 2 min. 4-Methoxymethylbenzylamine (4.00 g, 26.4 mmol) was added portionwise at -78 °C and the mixture was stirred at room temperature (2 h). The white solid was filtered and the organic layer was concentrated in vacuo. The residue was triturated with EtOAc resulting in a solid that was filtered and recrystallized with EtOAc to give (*R*)-2-*N*-(benzyloxycarbonyl)amino-3-hydroxy-*N-*(4-methoxymethylbenzyl)propanamide as a white solid (7.20 g, 88%): *R_{f}* = 0.63 (EtOAc); mp 138-140 °C; [α]^{25.8}_{D} -34.0° (c 1, DMSO); IR (nujol) 3385, 3294, 3106, 2923, 2859, 1690, 1646, 1544, 1458, 1373, 1307, 1243, 1098, 1028, 919, 738, 694 cm⁻¹; ¹H NMR (CDCl₃) *δ* 3.36 (s, OCH₃), 3.64 (dd, *J* = 5.1, 10.8 Hz CHH'OH), 3.88-4.06 (br d, CHH'OH), 4.19-4.29 (m, CH), 4.34-4.45 (m, CH₂OCH₃, NCH₂), 5.06 (s, CH₂O), 6.96 (d, *J* = 7.2 Hz, OC(O)NH), 7.02-7.14 (br s, NH), 7.19 (d, *J* = 7.8 Hz, 2 ArH), 7.27 (d, *J* = 7.8 Hz, 2 ArH), 7.30-7.38 (m, 5 ArH); ¹³C NMR (CDCl₃) *δ* 43.2 (NCH₂), 55.5 (OCH₂**C**H), 58.1 (CH₃O), 62.7 (O**C**H₂CH), 67.3 (**C**H₂O), 74.3 (**C**H₂OCH₃), 127.6, 128.0, 128.1, 128.3, 128.5, 135.9, 137.0, 137.5 (8 ArC), 156.7 (OC(O)N), 170.7 (C(O)); HRMS (M+H⁺)(ESI⁺) 373.1764 [M + H⁺] (calcd for C₂₀H₂₄N₂O₅H⁺ 373.1763); Anal. Calcd. for C₂₀H₂₄N₂O₅0.25H₂O: C, 63.73; H, 6.55; N, 7.43. Found: C, 63.35; H, 6.43; N, 7.29.

**Preparation of (*R*)-2-*N*-(Benzyloxycarbonyl)amino-3-methoxy-*N*-(4-methoxymethylbenzyl)propanamide.** Ag₂O (12.40 g, 53.7 mmol) was added to a CH₃CN solution (100 mL) of (*R*)-2-*N*-(benzyloxycarbonyl)amino-3-hydroxy-*N*-(4-methoxymethylbenzyl)-propanamide (4.00 g, 10.7 mmol) and CH₃I (6.71 mL, 107.5 mmol) at room temperature under Ar. The reaction mixture was stirred at room temperature (2 d), filtered, and the filtrate concentrated in vacuo to obtain 3.90 g of the desired compound as a white solid (94%): *R_{f}* = 0.79 (EtOAc); mp 107-108 °C; [α]^{24.6}_{D} -22.1° (c 1, CHCl₃); IR (nujol) 3285, 2958, 2732, 2681, 1688, 1645, 1545, 1458, 1376, 1305, 1240, 1112, 1048, 966, 815, 729, 696 cm⁻¹; ¹H NMR (COCl₃) *δ.* 3.36, 3.38 (2 s, 2 OCH₃), 3.49 (dd, *J* = 6.6, 9.3 Hz, CHH'), 3.85 (dd, *J* = 3.3, 9.3 Hz, CHH'), 4.28-4.38 (br m, CHCH₂), 4.43 (s, C**H**₂OCH₃), 4.46 (d, *J* = 6.3 Hz, C**H**₂N), 5.11 (s, OC**H**₂), 5.64-5.72 (br m, OC(O)NH), 6.66-6.74 (br m, CH₂N**H**), 7.23 (d, *J* = 8.4 Hz, 2 ArH), 7.29 (d, *J* = 8.4 Hz, 2 ArH), 7.31-7.38 (m, 5 ArH); ¹³C NMR (CDCl₃) *δ*. 43.2 (NCH₂), 54.3 (OCH₂**C**H), 58.0 (CH₂OCH₃) 59.0 (O**C**H₃), 67.2 (Ph**C**H₂O), 72.0 (O**C**H₂CH), 74.3 (**C**H₂OCH₃), 127.5, 128.0, 128.1, 128.2, 128.5, 136.0, 137.2, 137.6 (8 ArC), 156.1 (O**C**(O)), 169.8 (C(O)); HRMS (M+H⁺)(ESI⁺) 387.1920 [M + H⁺] (calcd for C₂₁H₂₆N₂O₅H⁺ 387.1920); Anal. Calcd. for C₂₁H₂₆N₂O₅: C, 65.27; H, 6.78; N, 7.25. Found: C, 65.27; H, 6.79; N, 7.38.

**Preparation of (*R*)-2-Acetamido-3-methoxy-*N*-(4-methoxymethylbenzyl)propanamide ((*R*)-8).** A MeOH solution (400 mL) of (*R*)-2-*N-*(benzyloxycarbonyl)amino-3-methoxy-*N*-(4-methoxymethylbenzyl)propanamide (3.50 g, 9.1 mmol) was treated with H₂ (1 atm) in presence of 10% Pd/C (350 mg) at room temperature (16 h). The mixture was carefully filtered through a bed of Celite^{®} and the filtrate was evaporated in vacuo to obtain a colorless oil. The oil was dissolved in CH₂Cl₂ (150 mL) and then triethylamine (1.52 mL, 10.9 mmol) and acetyl chloride (772 µL, 10.9 mmol) were carefully added at 0 °C and the resulting solution was stirred at room temperature (2 h). An aqueous 10% citric acid solution (150 mL) was added and the mixture was extracted with CH₂Cl₂ (3 x 100 mL). The organic layers were combined, washed with an aqueous saturated NaHCO₃ solution (100 mL), dried (MgSO₄), and concentrated in vacuo. The residue was triturated with EtOAc to give (*R*)-**8** as a white solid (1.50 g, 56%): *R_{f}* = 0.35 (EtOAc); mp 119-120 °C; [α]²⁵_{D} -25.4° (c 0.5 , CHCl₃); IR (nujol) 3266, 3069, 2935, 2863, 1635, 1550, 1458, 1457, 1382, 1282, 1226, 1194, 1125, 948, 836, 792, 726 cm⁻¹; ¹H NMR (CDCl₃) *δ.* 2.03 (s, C**H**₃CO), 3.37, 3.38 (2 s, 2 OCH₃), 3.43 (dd, *J* = 7.5, 9.1, CHH'), 3.80 (dd, *J* = 4.2, 9.1 Hz, CH**H**'), 4.41-4.49 (m, CH₂OCH₃, C**H**₂N), 4.51-4.58 (m, CH), 6.42-6.52 (br d, N**H**C(O)CH₃), 6.75-6.84 (br t, CH₂N**H**), 7.24 (d, *J* = 7.9 Hz, 2 ArH), 7.30 (d, *J* = 7.9 Hz, 2 ArH); addition of excess (*R*)-(-)-mandelic acid to a CDCl₃ solution of (*R*)-8 gave only one signal for the acetyl methyl and one signal for the ether methyl protons; ¹³C NMR (COCl₃) 23.2 (**C**H₃CO), 43.3 (**C**H₂N), 52.5 (**C**HCH₂), 58.1 (O**C**H₃), 59.1 (O**C**H₃), 71.8 (**C**H₂OCH₃), 74.3 (**C**H₂OMe), 127.5, 128.1, 137.3, 137.5 (4 Ar**C**), 170.0, 170.3 (2 **C**(O)); HRMS (M+Na⁺)(ESI⁺) 295.1658 [M + Na⁺] (calcd for C₁₅H₂₂N₂O₄H⁺ 295.1658); Anal. Calcd. for C₁₅H₂₂N₂O₄: C, 61.21; H, 7.45; N, 9.52. Found: C, 60.88; H, 7.45; N, 9.35.

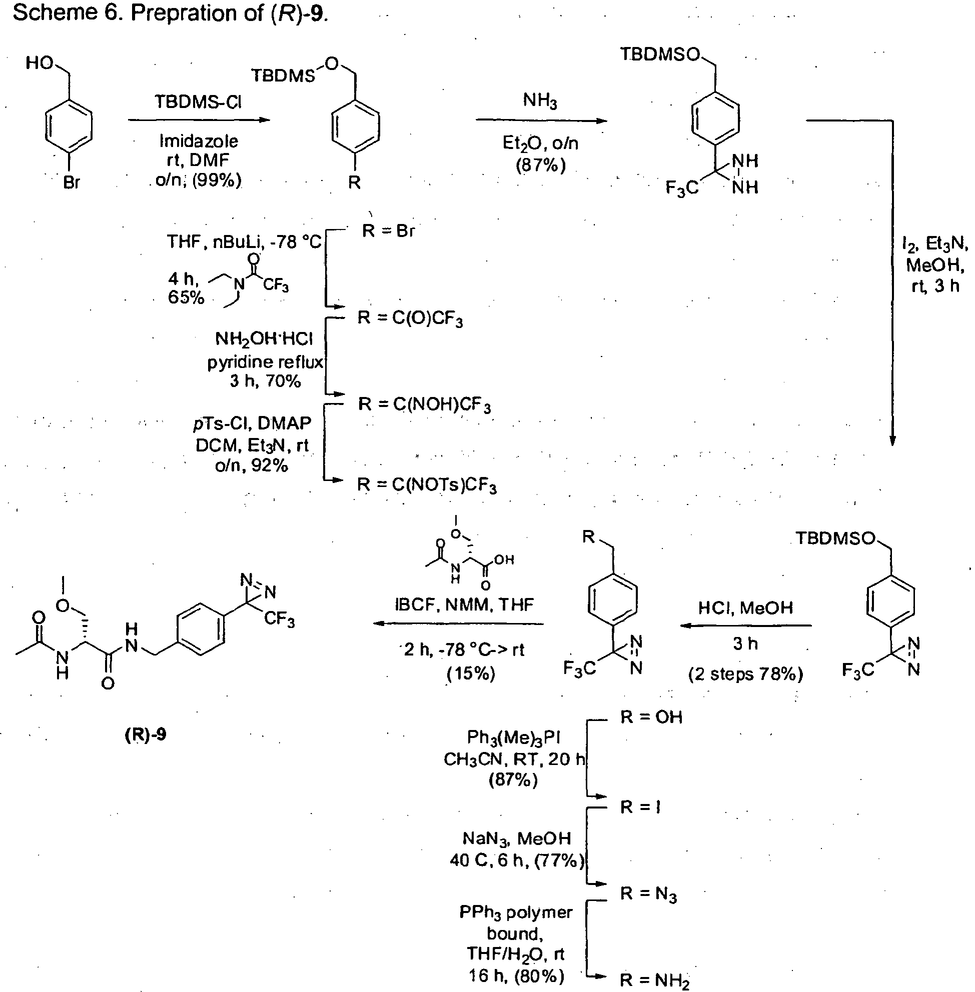

**Preparation of (4-Bromobenzyloxy)-(*tert*.-butyl)-dimethylsilane:** 4-Bromobenzyl alcohol (50.00 g, 267.3 mmol), tert-butyldimethylsilyl chloride (52.38 g, 347.5 mmol), and imidazole (23.66 g, 347.5 mmol) were dissolved in anhydrous DMF (300 mL). This solution was allowed to stir at room temperature overnight and then poured into ice-water (600 mL). The organic layer was separated off and washed with saturated aqueous sodium bicarbonate (2 x 200 mL) and water (2 x 200 mL). We obtained colorless oil (71.9 g, 88%): 'H NMR (CDCl₃): *δ* 0.10 (s, (C**H**₃)₂Si), 0.94 (s, (C**H**₃)₃C), 4.7 (s, C**H**₂O), 7.20 (d, J = 7.8 Hz, C₆**H**₄), 7.45 (d, J = 7.8 Hz, C₆**H**₄); ¹³C NMR (CDCl3): *δ*.

**Preparation of 1-(4-((*tert*.-Butyldimethylsilyloxy)methyl)phenyl)-2,2,2-Trifluoroethanone.** To a stirred solution of the (4-bromobenzyloxy)-(*ter.t*-butyl)-dimethylsilane (24.94 g, 82.8 mmol) in THF (420 mL) at -78°C was added dropwise a 1.6 M solution of nBuLi in hexane (62 mL, 99.2 mmol) over a 1 h period. After stirring the mixture at the same temperature for 75 min, a solution of N,N-diethyltrifluoroacetamide (19.19 g, 113 nmol) in THF (60 mL) was added dropwise over 1h. Following the addition the mixture at -78°C for a further 75 min before the reaction was quenched with an aqueous solution of NH₄Cl. Ether was added and the organic layer was extracted 3 times with ether (3 x 300 mL). The organic layers were combined, dried over Na₂SO₄ and concentrated in vacuum. The resulting pale yellow liquid was distillated in vacuo to give the 1-(4-((tert-butyldimethylsilyloxy)methyl)phenyl)-2,2,2-trifluoroethanone (65%) as a colorless liquid; b.p. 170-180°C (10 torr), 'H NMR (CDCl₃): *δ* 0.12 (s, (C**H**₃)₂Si), 0.96 (s, C**H**₃)₃C), 4.83 (s, C**H**₂), 7.50 (d, J = 8.2 Hz, C₆**H**₄), 8.05 (d, J = 8.2 Hz, C₆**H**₄); ¹³C NMR (CDCl₃): *δ* -5.4 (C**H**₃Si), 18.3 (**C**(CH₃)₃), 25.8 (C(**C**H₃)₃), 64.2 (**C**H₂O), 116.7 (q, J = 289.7 Hz, **C**F₃), 126.2 (**C**₆H₂), 128.6 (**C**₆H₂), 130.2 (**C**₆H₂), 150.1, (**C**₆H₂), 180.2 (q, J = 34.5 Hz, CO).

**Preparation of 1-(4-((*tert*.-Butyldimethylsilyloxy)methyl)phenyl)-2,2,2-Trifluoroethanone Oxime.** Hydroxylamine hydrochloride (6.55g, 94.3 mmol) was added to a pyridine (30 mL) solution of 1-(4-((*tert*.-butyldimethylsilyloxy)methyl)phenyl)-2,2,2-trifluoroethanone (10g, 31.4 mmol). The solution was stirred a reflux (4h). The pyridine was evaporated and 150 mL of an aqueous solution of citric acid (10%) and CH₂Cl₂ (100 mL) were added to the residue and the organic layer was extracted. The aqueous layer was washed 2 times with CH₂Cl₂ (2 x 100mL). Finally, the organic layers were combined and concentrated in vacuum. The residue was purified by silica gel flash chromatography (MPLC) to obtain 8.4 g of a white solid. *R_{f} =* 0.31 (1/9 EtOAc/hexanes); mp 63-65 °C; 'H NMR (CDCl₃): *δ* 0.12 (s, (C**H**₃)₂Si), 0.96 (s, C**H**₃)₃C), 4.73 (s, C**H**₂), 7.44 (d, J = 8.2 Hz, C₆**H**₄), 7.50 (d, J = 8.2 Hz, C₆**H**₄), 8.61 (s, OH); ¹³C NMR (CDCl₃): *δ* -5.3 (**C**H₃Si), 18.4 (**C**(CH₃)₃), 25.9 (C(**C**H₃)₃), 64.4 (**C**H₂O); 120,6 (q, J = 273.2 Hz, **C**F₃), 124.4 (**C**₆H₂), 125.9 (**C**₆H₂), 128.6 (**C**₆H₂), 144.2, (**C**₆H₂), 147.9 (q, J = 32.1 Hz, CN).

**Preparation of 1-(4-((*tert*.-Butyldimethylsilyloxy)methyl)phenyl)-2,2,2-trifluoroethanone O-Tosyl Oxime.** An anhydrous CH₂Cl₂ solution of pTs-Cl (10.10 g, 53.3 mmol) was added portionwise to an anhydrous CH₂Cl₂ solution of 1-(4-((*tert*.-butyldimethylsilyloxy)methyl)phenyl)-2,2,2-trifluoroethanone oxime, Et₃N and DMAP. The solution was stirred at rt (overnight). 200 mL of an aqueous solution of citric acid (10%) was added to the reaction solution and the organic layer was extracted. The aqueous layer was washed 2 times with CH₂Cl₂ (2 x 200mL). The organic layers were combined, dried (Na₂SO₄) and concentrated in vacuum. The residue was purified by silica gel flash chromatography to obtain 11.80 g of a colorless oil (92%). *R_{f}* = 0.58 (1/9 EtOAc/hexanes); 'H NMR (CDCl₃): ***δ*** 0.12 (s, (C**H**₃)₂Si), 0.96 (s, C**H**₃)₃C), 2.48 (s, C**H**₃Ph), 4.78 (s, C**H**₂), 7.37-7.45 (m, 6 C₆**H**₄), 7.89 (d, J = 8.4 Hz, 2 C₆**H**₄); ¹³C NMR (CDCl₃): *δ* -5.3 (**C**H₃Si), 18.4 (**C**(CH₃)₃), 21.7 (**C**H₃), 25.8 (C(**C**H₃)₃), 64.2 (**C**H₂O), 119,7 (q, J = 275.5 Hz, **C**F₃), 122.9 (**C**₆H₂), 126.0 (**C**₆H₂), 128.4 (**C**₆H₂), 129.3 (**C**₆H₂), 129.8 (**C**₆H₂), 131.3 (**C**₆H₂), 145.6 (C₆H₂), 146.0 (**C**₆H₂), 154.0 (q, J = 33.0 Hz, **C**N).

**Preparation of 3-(4-((*tert*.-Butyldimethylsilyloxy)methyl)phenyl)-3-(trifluoromethyl)diaziridine.** Liquid ammonia (2 mL) was added a -78°C to an ether (15 mL) solution of 1-(4-((*tert*.-butyldimethylsilyloxy)methyl)phenyl)-2,2,2-trifluoroethanone O-tosyl oxime (4.00 g, 8.2 mmol) in a sealed tube. The solution was stirred at rt (16 h). The mixture was carefully cooled at -78°C and the sealed tube was opened. The ammonia was evaporated and the white precipitate was filtered and washed with ether. The filtrate was concentrated in vaccum to obtain 2.4 g of a colorless oil (87%); 'H NMR (CDCl₃): *δ* 0.10 (s, (C**H**₃)₂Si), 0.95 (s, C**H**₃)₃C), 2.21 (d, J = 8.1 Hz, NH), 2.77 (d, J = 8.1 Hz, NH), 4.76 (s, C**H**₂), 7.38 (d, J = 8.4 Hz, 2 C₆**H**₄), 7.58 (d, J = 8.4 Hz, 2 C₆**H**₄); ¹³C NMR (CDCl₃): *δ* -5.3 (**C**H₃Si), 18.4 (**C**(CH₃)₃), 25.9 (C(**C**H₃)₃), 58.0 (q, J = 35.8 Hz, C diaziridine), 64.4 (**C**H₂O), 123,6 (q, J = 276.0 Hz, **C**F₃), 126.2 (**C**₆H₂), 128.0 (**C**₆H₂), 130.2 (**C**₆H₂), 143.8 (**C**₆H₂).

**Preparation of 4-[3-(Trifluoromethyl)-3H-Diazirin-3-yl]-Benzenemethanol.** I₂ (1.14 g, 4.5 mmol) was added to a MeOH (4 mL) solution of Et₃N (1.2 mL) and 3-(4-((*tert*.-butyldimethylsilyloxy)methyl)phenyl)-3-(trifluoromethyl)-diaziridine (1.65 g, 5.00 mmol). The solution was stirred at rt (3h). Aqueous citric acid 10 % (50 mL) and NaS₂O₃ were added. The organic layer was extracted 3 times with ether (3 x 30 mL). The organic layers were combined, dried amd concentrated in vacuum to obtain a yellow oil; 'H NMR (CDCl₃): *δ* 0.09 (s, (C**H**₃)₂Si), 0.94 (s, C**H**₃)₃C), 4.74 (s, C**H**₂), 7.15 (d, J = 8.0 Hz, 2 C₆**H**₄), 7.35 (d, J = 8.0 Hz, 2 C₆**H**₄). HCl in dioxane (4 M, 5 mL, 20 mmol) was added to a MeOH (10 mL) solution of the yellow residue. The solution was stirred at rt (3h) and the volatiles were evaporated. The residue was purified by silica gel flash chromatography to obtain 840 mg (78 %) of a yellow liquid: *R_{f}* = 0.19 (1/9 EtOAc/hexanes); 'H NMR (CDCl₃): *δ* 1.95-2.05 (br, OH), 4.70 (s, C**H**₂), 7.18 (d, J = 8.4 Hz, 2 C₆**H**₄), 7.38 (d, J = 8.4 Hz, 2 C₆**H**₄); ¹³C NMR (CDCl₃): *δ* 28.3 (q, J = 40.4 Hz, C diazirine), 64.4 (**C**H₂O), 122.1 (q, J = 273.0 Hz, **C**F₃), 126.7 (**C**₆H₂), 127.1 (**C**₆H₂), 128.3 (**C**₆H₂), 142.5 (**C**₆H₂).

**Preparation of 3-[4-(Iodomethyl)phenyl]-3-(Trifluoromethyl)-3H-Diazirine.** PPh₃MeI (3.35 g, 7.4 mmol) was added to a 4-[3-(trifluoromethyl)-3H-diazirin-3-yl]-benzenemethanol (8) (0.80 g, 3.7 mmol) in anhydrous acetonitrile (6 mL). The solution was stirred at rt and in the dark (20h). Ether (30 mL) and an aqueous NaOH 1 M (30 mL) were added successively. The organic layer was extracted and the aqueous one was washed with ether (2 x 30 mL). The organic layers were combined and concentrated in vacuum. Finally, the residue was purified by silica gel flash chromatography (0 → 20 % EtOAc in hexanes) to obtain 1.06 g (87 %) of a colorless liquid: *R_{f}* = 0.95 (1/9 EtOAc/hexanes); 'H NMR (CDCl₃): *δ* 4.42 (s, C**H**₂), 7.11 (d, J = 8.3 Hz, 2 C₆**H**₄), 7.39 (d, J = 8.3 Hz, 2 C₆**H**₄); ¹³C NMR (CDCl₃): *δ* 28.3 (q, J = 40.1 Hz, C diazirine), 33.8 (**C**H₂I), 122.0 (q, J = 273.2 Hz, **C**F₃), 126.9 (**C**₆H₂), 128.7 (**C**₆H₂), 129.1 (**C**₆H₂), 141.1 (**C**₆H₂).

**Preparation of 3-[4-(Azidomethyl)phenyl]-3-(Trifluoromethyl)- 3H-Diazirine.** NaN₃ (0.42 g, 6.2 mmol) was added to a MeOH (30 mL) solution of 3-[4-(iodomethyl)phenyl]-3-(trifluoromethyl)-3H-diazirine (1.01 g, 3.1 mmol). The solution was stirred at 45 °C (6h). The methanol was evaporated and CH₂Cl₂ (30 mL) and water (30 mL) were added. The organic layer was extracted and the aqueous layer was washed with CH₂Cl₂ (1 x 30 mL). The organic layers were combined and concentrated in vacuum. Finally, the residue was purified by silica gel flash chromatography (0 → 30 % EtOAc in hexanes) to obtain 570 mg (77 %) of a pale yellow liquid: *R_{f}* = 0.85 (1/9 EtOAc/hexanes); IR (nujol) 3298, 2927, 2859, 2100 (N₃), 1694, 1615, 1459, 1374, 1236, 1162, 1055, 939, 805, 728 cm⁻¹; ¹H NMR (CDCl₃): *δ* 4.37 (s, C**H**₂), 7.21 (d, J = 7.9 Hz, 2 C₆**H**₄), 7.35 (d, J = 7.9 Hz, 2 C₆**H**₄); ¹³C NMR (CDCl₃): *δ* 28.3 (q, J = 40.2 Hz, C diazirine), 54.1 (**C**H₂N₃), 122,1 (q, J = 273.0 Hz, **C**F₃), 127.0 (**C**₆H₂), 128.5 (**C**₆H₂), 129.2 (**C**₆H₂), 137.2 (**C**₆H₂).

**Preparation of 4-[3-(Trifluoromethyl)-3H-Diazirin-3-yl]-Benzenemethanamine.** Triphenylphosphine polymer bound (Fluka, cat # 93094) (1.6 g/mol, 6.3 mmol) was added to an aqueous (378 µL, 21.0 mmol)/THF (10 mL) solution of 3-[4-(azidomethyl)phenyl]-3-(trifluoromethyl)- 3H-diazirine (506 mg, 2.1 mmol). The mixture was shaken until the starting material was no longer evident by TLC analysis (18 h). The triphenylphosphine-based support was filtered, washed with CH₂Cl₂, and the filtrate was concentrated in vacuum to obtain 350 mg (80%) of a yellow oil corresponding to the free amine: ¹H NMR (CDCl₃) *δ* 1.35-1.50 (br s, NH₂), 3.89 (s, C**H**₂), 7.17 (d, J = 8.2 Hz, 2 C₆**H**₄), 7.35 (d, J = 8.2 Hz, 2 C₆**H**₄); ¹³C NMR (CDCl₃): *δ* 28.4 (q, J = 39.9 Hz, C diazirine), 45.9 (**C**H₂NH₂), 122.2 (q, J = 273.2 Hz, **C**F₃), 126.7 (**C**₆H₂), 127.5 (**C**₆H₂), 145.0 (**C**₆H₂) (one peak is overlapped with an other one).

**Preparation of (*R*)-2-Acetamido-3-methoxy-*N*-(4-(3-(trifluoromethyl)-3H-diazirin-3-yl)benzyl)propanamide ((*R*)-9).** 4-[3-(Trifluoromethyl)-3H-diazirin-3-yl]-benzenemethanamine hydrochloride (933 mg, 3.7 mmol) was added to a THF (31 mL) solution of the (*R*)-2-acetamido-3-methoxypropanoic acid (500 mg, 3.1 mmol), and the mixture was stirred at room temperature (5 min) and then NMM (0.41 mL, 3.7 mmol) was added. The mixture was stirred a room temperature (5 min) and DMTMM (1.03 g, 3.7 mmol) was added, and the mixture was stirred at room temperature (16 h). The white precipitate was filtered and the filtrate was concentrated in vacuo. The residue was purified by flash column chromatography on silica gel with EtOAc/acetone (10/0 -> 6/4) as the eluant to obtain a white solid (810 mg, 73%): *R_{f}* = 0.74 (EtOAc); mp 195 °C (decomp); [α]²⁵_{D} -11.0° (c 0.5, CHCl₃); IR (nujol) 3278, 1635, 1554, 1458, 1375, 1236, 1186, 1148, 1054, 940, 805, 731 cm⁻¹; ¹H NMR (CDCl₃) *δ* 2.02 (s, C**H**₃C(O)), 3.38 (s, OCH₃), 3.44 (dd, *J* = 7.2, 9.0 Hz, CHH'), 3.80 (dd, *J* = 4.2, 9.0 Hz, CH**H**'), 4.40-4.51 (m, C**H**₂), 4.52-4.59 (m, CH), 6.45 (d, *J* = 6.0 Hz, **NH**C(O)CH₃), 6.89-6.98 (br t, **NH**CH₂), 7.15 (d, J = 8.2 Hz, 2 ArH), 7.29 (d, *J* = 8.2 Hz, 2 ArH); addition of excess (R)-(-)-mandelic acid to a CDCl₃ solution of (*R*)-18 gave only one signal for the acetyl methyl and one signal for the ether methyl protons; ¹³C NMR (CDCl₃): □ 23.2 (**C**H₃C(O)), 28.3 (q, *J* = 40.1 Hz, C diazirine), 43.0 (**C**H₂N), 52.5 (CH), 59.1 (O**C**H₃), 71.5 (**C**H₂OCH₃), 122.1 (q, *J* = 273.1 Hz, **C**F₃), 126.9, 127.8, 128.3, 139.9 (4 ArC), 170.2, 170.4 (2 CO); HRMS (M+H⁺)(ESI⁺) 359.1334 [M + Na⁺] (calcd C₁₅H₁₇F₃N₄O₃H⁺ 359.1331); Anal. Calcd. for C₁₅H₁₇F₃N₄O₃0.05C₃H₆O: C, 50.34; H, 4.81; N, 15.56; F, 15.83. Found: C, 50.59; H, 4.76; N, 15.24; F, 15.46.

### EXAMPLE 2

### Formalin Test

Compounds of the present invention were active in treating pain in a formalin test carried out in accordance with standard techniques, as described in S. Hunskaar et al., J. Neurosci. Methods 14: 69-76 (1985). Briefly, 0.5% formalin is injected into the mouse hind paw to elicit a bi-phasic licking response, where the first acute phase is thought to correspond to the direct stimulation of peripheral fibers and the second (inflammatory) phase is caused by the release of inflammatory mediators. Results areas shown in **Table 2** below.

The foregoing is illustrative of the present invention, and is not to be construed as limiting thereof. The invention is defined by the following claims,

**Table 2. Formalin Test Results of Lacosamide AB & CR Probes**

| | | | | | | | | | |
|---|---|---|---|---|---|---|---|---|---|
| 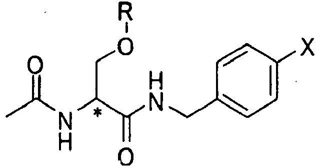 | | | | | | | | | |

| | | | | | Area Under the Curve | | | | |
|---|---|---|---|---|---|---|---|---|---|
| R | X | Stereo | Dose (mg/kg) | Test | Control | Drug Tested | % of Control | S.E.M | P value |
| CH₂C≡CH | H | *(R)* | 16.0 | Acute | 295.1 | 142.9 | 48.42 | 3.52 | < 0.01 |
| | | | 16.0 | Inflammatory | 797.3 | 468.3 | 54.99 | 14.75 | < 0.05 |
| | | | | | | | | | |
| CH₃ | N₃ | *(R)* | 8.0 | Acute | 224.0 | 131.4 | 58.67 | 12.49 | < 0.05 |
| | | | 8.0 | Inflammatory | 653.8 | 694.5 | 106.22 | 9.5 | > 0.05 |
| | | | | | | | | | |
| CH₂C≡CH | N₃ | *(R)* | 20.0 | Acute | 205.8 | 119.9 | 58.28 | 9.68 | < 0.05 |
| | | | 20.0 | Inflammatory | 530.0 | 414.3 | 78.17 | 14.84 | > 0.05 |

## Claims

1. A compound of Formula I: 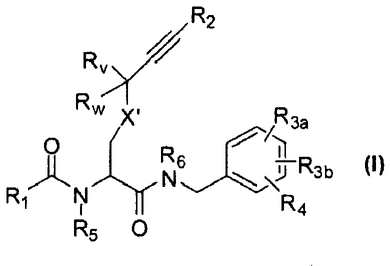 wherein:
R₁ is alkyl or cycloalkyl, each of which can be unsubstituted or substituted with from one to four independently selected electron-donating or electron-withdrawing groups;
R₂, Rᵥ and R_{w} are each independently hydrogen, alkyl, cycloalkyl, heterocyclo, aryl, or heteroaryl, which alkyl, cyloalkyl, heteroacylclo, aryl, or heteroaryl can be unsubstituted or substituted with from one to four independently selected electron-donating or electron-withdrawing groups, or R₂, Rᵥ and R_{w} are each independently an electron-donating or electron-withdrawing group;
R₃ₐ, R_{3b} and R₄ are each independently hydrogen, an electron donating or electron-withdrawing group, or alkyl, cycloalkyl, alkenyl, alkynyl, aryl, heterocyclo, heteroaryl, arylalkyl, heteroarylalkyl, heterocycloalkyl, each of which can be unsubstituted or substituted with from one to four independently selected electron-donating or electron-withdrawing groups;
R₅ and R₆ are each independently H or alkyl;
X' is S or O;
or a pharmaceutically acceptable salt thereof.

2. The compound of claim 1, wherein:
Rᵥ and R_{w} are both H;
R⁴ is H;
R⁵ is H;
R⁶ is H; and
X' is O;
or a pharmaceutically acceptable salt thereof.

3. The compound of claim 1, wherein:
Rᵥ and R_{w} are both H;
R₁ is C1-C4 alkyl;
R₂ is hydrogen or C1-C4 alkyl;
R₃ₐ is H;
R_{3b} is H, N₃, or NCS;
R₄ is H;
R₅ and R₆ are both H;
X' is O;
or a pharmaceutically acceptable salt thereof.

4. The compound of claim 1, wherein said compound is selected from the group consisting of:

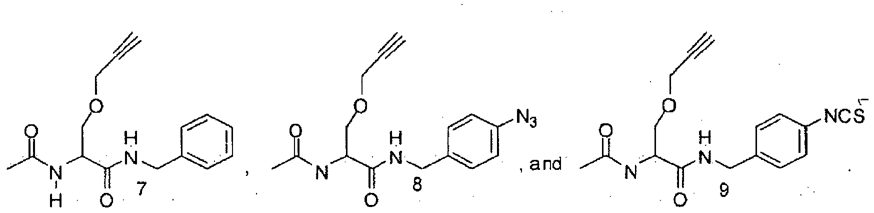

5. The compound of any preceding claim in the (*R*)-configuration.

6. The compound of any preceding claim in substantially pure form.

7. A pharmaceutical composition comprising a compound of any preceding claim in a pharmaceutically acceptable carrier.

8. The composition of claim 7, further comprising at least one additional neurological active agent.

9. The composition of any of claims 7 - 8 in oral administration form.

10. A compound of any of claims 1 - 5 for use in a treatment effective amount for treating a neurological disorder in a mammalian subject.

11. A method of making a compound of Formula I: 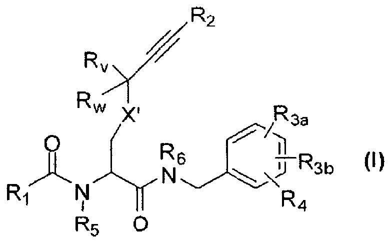 wherein:
R₁ is alkyl or cycloalkyl, each of which can be unsubstituted or substituted with from one to four independently selected electron-donating or electron-withdrawing groups;
R₂, Rᵥ and R_{w} are each independently hydrogen, alkyl, cycloalkyl, heterocyclo, aryl, or heteroaryl, which alkyl, cyloalkyl, heteroacylclo, aryl, or heteroaryl, can be unsubstituted or substituted with from one to four independently selected electron-donating or electron-withdrawing groups, or R₂, Rᵥ and R_{w} are each independently an electron-donating or electron-withdrawing group;
R₃ₐ, R_{3b} and R₄ are each independently hydrogen, an electron donating or electron-withdrawing group, or alkyl, cycloalkyl, alkenyl, alkynyl, aryl, heterocyclo, heteroaryl, arylalkyl, heteroarylalkyl, heterocycloalkyl, each of which can be unsubstituted or substituted with from one to four independently selected electron-donating or electron-withdrawing groups;
R₅ and R₆ are each independently H or alkyl; and
X' is S or O;
said method comprising:
reacting a compound of Formula **II:** 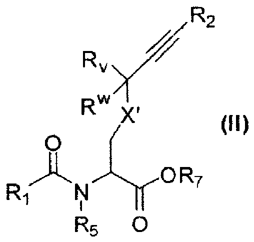
where R₇ is H with a peptide coupling reagent to form an activated intermediate and reacting said activated intermediate in a polar aprotic solvent with a compound of Formula **III:** 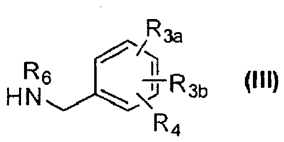 to produce said compound of **Formula I.**

## Patentansprüche

1. Verbindung der Formel I: 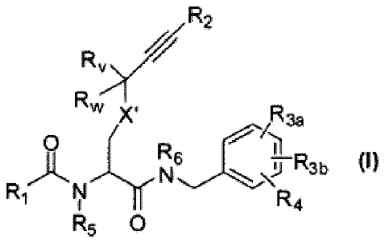 worin:
R₁ ist Alkyl oder Cycloalkyl, wobei jedes davon nicht substituiert oder mit einer bis vier unabhängig voneinander ausgewählten Elektronen abgebenden oder Elektronen ziehenden Gruppen substituiert sein kann;
R₂, Rᵥ und R_{w} sind jeweils unabhängig voneinander Wasserstoff, Alkyl, Cycloalkyl, Heterocyclo, Aryl oder Heteroaryl, wobei Alkyl, Cycloalkyl, Heterocyclo, Aryl oder Heteroaryl nicht substituiert oder mit einer bis vier unabhängig voneinander ausgewählten Elektronen abgebenden oder Elektronen ziehenden Gruppen substituiert sein können, oder R₂, Rᵥ und R_{w} sind jeweils unabhängig voneinander eine Elektronen abgebende oder eine Elektronen ziehende Gruppe;
R₃ₐ, R_{3b} und R₄ sind jeweils unabhängig voneinander Wasserstoff, eine Elektronen abgebende oder eine Elektronen ziehende Gruppe, oder Alkyl, Cycloalkyl, Alkenyl, Alkinyl, Aryl, Heterocyclo, Heteroaryl, Arylalkyl, Heteroarylalkyl, Heterocycloalkyl, wobei jedes davon nicht substituiert oder mit einer bis vier unabhängig voneinander ausgewählten Elektronen abgebenden oder Elektronen ziehenden Gruppen substituiert sein kann;
R₅ und R₆ sind jeweils unabhängig voneinander Wasserstoff oder Alkyl;
X' ist S oder O;
oder ein pharmazeutisch aufnehmbares Salz derselben.

2. Verbindung nach Anspruch 1, worin:
Rᵥ und R_{w} sind beide H;
R₄ ist H;
R₅ ist H;
R₆ ist H; und
X' ist O;
oder ein pharmazeutisch aufnehmbares Salz derselben.

3. Verbindung nach Anspruch 1, worin:
Rᵥ und R_{w} sind beide H;
R₁ ist C₁-C₄-Alkyl;
R₂ ist Wasserstoff oder C₁-C₄-Alkyl;
R₃ₐ 1st H;
R_{3b} ist H, N₃, oder NCS;
R₄ ist H;
R₅ und R₆ sind beide H;
X' ist O;
oder ein pharmazeutisch akzeptables Salz derselben.

4. Verbindung nach Anspruch 1, worin die Verbindung aus der Gruppe ausgewählt wird, die aus Folgendem besteht:

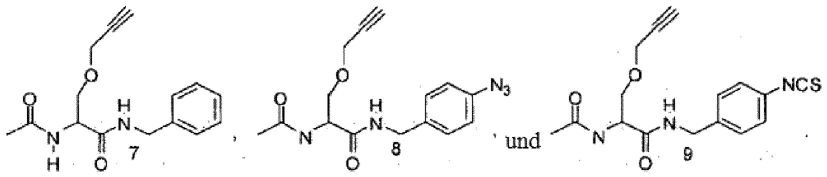

5. Verbindung nach einem der vorhergehenden Ansprüche in der (*R*)-Konfiguration.

6. Verbindung nach einem der vorhergehenden Ansprüche in im Wesentlichen reiner Form.

7. Pharmazeutische Zusammensetzung, umfassend eine Verbindung nach einem der vorhergehenden Ansprüche in einem pharmazeutisch akzeptablen Träger.

8. Zusammensetzung nach Anspruch 7, ferner mindestens einen zusätzlichen neurologisch aktiven Wirkstoff umfassend.

9. Zusammensetzung nach einem der Ansprüche 7 bis 8 in Oralverabreichungsform.

10. Verbindung nach einem der Ansprüche 1 bis 5 zur Verwendung in einer behandlungswirksamen Menge zur Behandlung einer neurologischen Störung in einem Säugetierpatienten.

11. Verfahren zur Herstellung einer Verbindung der Formel I: 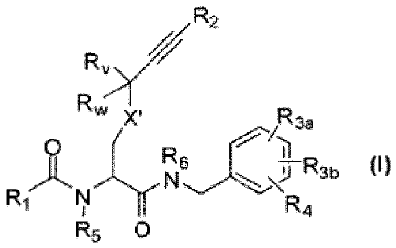 worin:
R₁ ist Alkyl oder Cycloalkyl, wobei jedes davon nicht substituiert oder mit einer bis vier unabhängig voneinander ausgewählten Elektronen abgebenden oder Elektronen ziehenden Gruppen substituiert sein kann;
R₂, Rᵥ und R_{w} sind jeweils unabhängig voneinander Wasserstoff, Alkyl, Cycloalkyl, Heterocyclo, Aryl oder Heteroaryl, wobei Alkyl, Cycloalkyl, Heterocyclo, Aryl oder Heteroaryl nicht substituiert oder mit einer bis vier unabhängig voneinander ausgewählten Elektronen abgebenden oder Elektronen ziehenden Gruppen substituiert sein können, oder R₂, Rᵥ und R_{w} sind jeweils unabhängig voneinander eine Elektronen abgebende oder eine Elektronen ziehende Gruppe;
R₃ₐ, R_{3b} und R₄ sind jeweils unabhängig voneinander Wasserstoff, eine Elektronen abgebende oder eine Elektronen ziehende Gruppe, oder Alkyl, Cycloalkyl, Alkenyl, Alkinyl, Aryl, Heterocyclo, Heteroaryl, Arylalkyl, Heteroarylalkyl, Heterocycloalkyl, wobei jedes davon nicht substituiert oder mit einer bis vier unabhängig voneinander ausgewählten Elektronen abgebenden oder Elektronen ziehenden Gruppen substituiert sein kann;
R₅ und R₆ sind jeweils unabhängig voneinander Wasserstoff oder Alkyl;
X' ist S oder O;
wobei das Verfahren umfasst:
zur Reaktion Bringen einer Verbindung der Formel **II:** 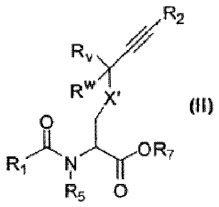
worin R₇ H ist, mit einem peptidbindenden Reagens, um ein aktiviertes Zwischenprodukt zu bilden, und zur Reaktion Bringen des aktivierten Zwischenprodukts in einem polaren aprotischen Lösungsmittel mit einer Verbindung der Formel **III:** 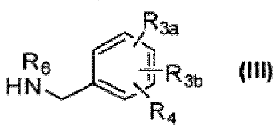 um die Verbindung der Formel I zu produzieren.

## Revendications

1. Composé de la Formule I: 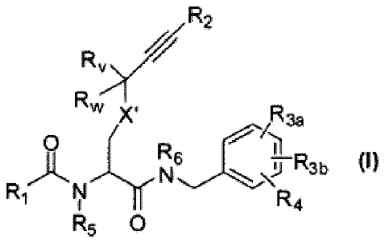 où:
R₁ est un alkyle ou un cycloalkyle, chacun desquels peut être non substitué ou substitué par de un à quatre groupes donneurs d'électrons ou attracteurs d'électrons sélectionnés indépendamment;
R₂, Rᵥ et R_{w} sont indépendamment un hydrogène, alkyle, cycloalkyle, hétérocyclo, aryle ou hétéroaryle, lequel alkyle, cycloalkyle, hétérocyclo, aryle ou hétéroaryle peut être non substitué ou substitué par de un à quatre groupes donneurs d'électrons ou attracteurs d'électrons sélectionnés indépendamment, ou bien R₂, Rᵥ et R_{w} sont chacun indépendamment un groupe donneur d'électrons ou attracteur d'électrons;
R₃ₐ, R_{3b} et R₄ sont chacun indépendamment un hydrogène, un groupe donneur d'électrons ou attracteur d'électrons, ou bien un alkyle, cycloalkyle, alcényle, alcynyle, aryle, hétérocyclo, hétéroaryle, arylalkyle, hétéroarylalkyke, hétérocycloalkyle, chacun desquels peut être non substitué ou substitué par de un à quatre groupes donneurs d'électrons ou attracteurs d'électrons sélectionnés indépendamment;
R₅ et R₆ sont chacun indépendamment H ou alkyle;
X' est S ou O;
ou un sel pharmaceutiquement acceptable de celui-ci.

2. Composé selon la revendication 1, dans lequel:
Rᵥ et R_{w} sont tous les deux H;
R⁴ est H;
R⁵ est H;
R⁶ est H; et
X' est O;
ou un sel pharmaceutiquement acceptable de celui-ci.

3. Composé selon la revendication 1, dans lequel:
Rᵥ et R_{w} sont tous les deux H;
R₁ est un alkyle C₁-C₄;
R₂ est un hydrogène ou un alkyle C₁-C₄;
R₃ₐ est H;
R_{3b} est H, N₃ ou NCS;
R₄ est H;
R₅ et R₆ sont tous les deux H;
X' est O;
ou un sel pharmaceutiquement acceptable de celui-ci.

4. Composé selon la revendication 1, où ledit composé est sélectionné parmi le groupe consistant en:

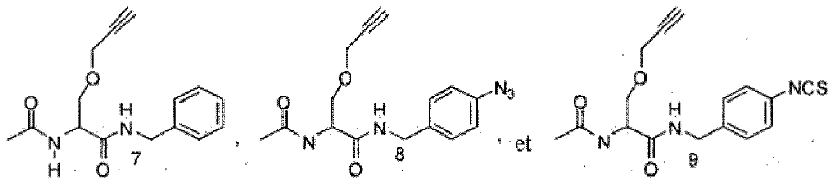

5. Composé selon l'une quelconque des revendications précédentes dans la configuration (R).

6. Composé selon l'une quelconque des revendications précédentes sous une forme essentiellement pure.

7. Composition pharmaceutique comprenant un composé selon l'une quelconque des revendications précédentes dans un véhicule pharmaceutiquement acceptable.

8. Composition selon la revendication 7, comprenant en outre au moins un agent neurologique actif supplémentaire.

9. Composition selon l'une quelconque des revendications 7 - 8, sous forme d'administration orale.

10. Composé selon l'une quelconque des revendications 1 - 5, à utiliser en une quantité de traitement efficace pour traiter un trouble neurologique chez un sujet mammalien.

11. Procédé de préparation d'un composé de la Formule I: 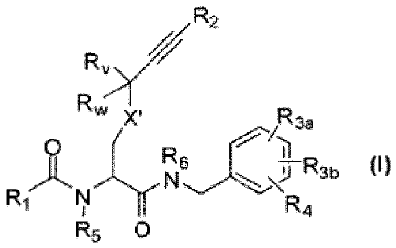 où:
R₁ est un alkyle ou un cycloalkyle, chacun desquels peut être non substitué ou substitué par de un à quatre groupes donneurs d'électrons ou attracteurs d'électrons sélectionnés indépendamment;
R₂, Rᵥ et R_{w} sont indépendamment un hydrogène, alkyle, cycloalkyle, hétérocyclo, aryle ou hétéroaryle, lequel alkyle, cycloalkyle, hétérocyclo, aryle ou hétéroaryle peut être non substitué ou substitué par de un à quatre groupes donneurs d'électrons ou attracteurs d'électrons sélectionnés indépendamment, ou bien R₂, Rᵥ et R_{w} sont chacun indépendamment un groupe donneur d'électrons ou attracteur d'électrons;
R₃ₐ, R_{3b} et R₄ sont chacun indépendamment un hydrogène, un groupe donneur d'électrons ou attracteur d'électrons, ou bien un alkyle, cycloalkyle, alcényle, alcynyle, aryle, hétérocyclo, hétéroaryle, arylalkyle, hétéroarylalkyke, hétérocycloalkyle, chacun desquels peut être non substitué ou substitué par de un à quatre groupes donneurs d'électrons ou attracteurs d'électrons sélectionnés indépendamment;
R₅ et R₆ sont chacun indépendamment H ou alkyle;
X' est S ou O;
ledit procédé comprenant:
faire réagir un composé de la Formule **II:** 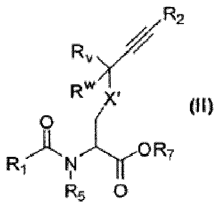 où R₇ est H, avec un réactif de couplage peptidique pour former un intermédiaire activé et faire réagir ledit intermédiaire activé dans un solvant polaire aprotique avec un composé de la Formule **III:** 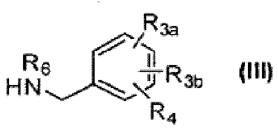 pour produire ledit composé de la Formule I.
